# EUROPÄISCHE PATENTSCHRIFT

(11) **EP 1 429 754 B1**
(45) Veröffentlichungstag und Bekanntmachung des Hinweises auf die Patenterteilung: **16.01.2008**
(21) Anmeldenummer: 02779368.6
(22) Anmeldetag: 18.09.2002
(51) Int. Cl.: A61K 31/216, A61P 13/10, A61K 31/137, A61K 31/485

(54) **KOMBINATION AUSGEWÄHLTER OPIOIDE MIT MUSCARIN-ANTAGONISTEN ZUR THERAPIE DER HARNINKONTINENZ**
COMBINATION OF SELECTED OPIOIDS WITH MUSCARINE ANTAGONISTS FOR TREATING URINARY INCONTINENCE
COMBINAISON D'OPIOIDES SPECIFIQUES ET D'ANTAGONISTES MUSCARINIQUES DESTINEE A TRAITER L'INCONTINENCE URINAIRE

(30) Priorität: 18.09.2001 DE 10146275
(43) Veröffentlichungstag der Anmeldung: 23.06.2004
(73) Patentinhaber: Grünenthal GmbH, 52078 Aachen (DE)
(72) Erfinder: CHRISTOPH, Thomas, 52080 Aachen (DE)
(74) Vertreter: Hellfeldt, Kurt
(86) Internationale Anmeldenummer: PCT/EP2002/010460
(87) Internationale Veröffentlichungsnummer: WO 2003/024444

(56) Entgegenhaltungen:
- WO-A-01/24783
- WO-A-01/62236
- WO-A-96/27375
- DE-A- 3 129 982
- GB-A- 857 194
- BUTERA J.A. ET AL: "Recent approaches to the treatment of urinary incontinence: A survey of patent activity from 1995 to 1998." EXPERT OPINION ON THERAPEUTIC PATENTS, (1998) 8/8 (1017-1035). , XP002223992
- SMITH C.P. ET AL: "Genitourinary tract patent update." EXPERT OPINION ON THERAPEUTIC PATENTS, (2001) 11/1 (17-31). , XP002223993
- DRAY A ET AL: "INHIBITION OF URINARY BLADDER CONTRACTIONS BY A SPINAL ACTION OF MORPHINE AND OTHER OPIOIDS" JOURNAL OF PHARMACOLOGY AND EXPERIMENTAL THERAPEUTICS, AMERICAN SOCIETY FOR PHARMACOLOGY AND, US, Bd. 231, Nr. 2, 1. November 1984 (1984-11-01), Seiten 254-260, XP000600400 ISSN: 0022-3565

## Beschreibung

Die Erfindung betrifft die Verwendung einer Kombination von Verbindungen der Gruppe A, insbesondere Opioiden, und Verbindungen der Gruppe B, insbesondere Antimuskarinika und anderen überwiegend peripher wirkenden Stoffen, zur Herstellung eines Arzneimittels zur Behandlung von vermehrtem Harndrang bzw. Haminkontinenz sowie entsprechende Arzneimittel und Verfahren zur Behandlung von vermehrtem Harndrang bzw. Harninkontinenz.

Harninkontinenz ist der unwillkürliche Harnabgang. Dieser tritt unkontrolliert auf, wenn der Druck innerhalb der Harnblase den Druck übersteigt, der zum Schließen des Harnleiters notwendig ist. Ursachen können zum einen ein erhöhter interner Blasendruck (z. B. durch Detrusorinstabilität) mit der Folge der Dranginkontinenz und zum anderen ein erniedrigter Sphinkterdruck (z. B. nach Geburt oder chirurgischen Eingriffen) mit der Folge der Streßinkontinenz sein. Der Detrusor ist die grob gebündelte mehrschichtige Blasenwandmuskulatur, deren Kontraktion zur Hamentleerung führt, der Sphinkter der Schließmuskel der Harnröhre. Es treten Mischformen dieser Inkontinenzarten sowie die sogenannte Überflußinkontinenz oder Reflexinkontinenz (z. B. nach Rückenmarksschädigungen) auf. So sind beispielsweise Harninkontinenz, Harndrang und erhöhte Miktionsfrequenz alles mögliche Symptome der benignen Prostatahyperplasie. Näheres zu diesem Komplex findet sich bei Chutka, D. S. und Takahashi, P. Y., 1998, Drugs 560: 587-595.

Harndrang ist der auf Harnentleerung (Miktion) abzielende Zustand vermehrter Blasenmuskelspannung bei Annäherung an die Blasenkapazität (bzw. bei deren Überschreitung). Dabei wirkt diese Anspannung als Miktionsreiz. Unter einem vermehrten Harndrang versteht man dabei insbesondere das Auftreten vorzeitigen oder gehäuften manchmal sogar schmerzhaften Harndrangs bis hin zum sog. Harnzwang. Das führt in der Folge zu einer deutlich häufigeren Miktion. Ursachen können u.a. Harnblasenentzündungen und neurogene Blasenstörungen sowie auch Blasentuberkulose sein. Es sind aber noch nicht alle Ursachen geklärt.

Vermehrter Harndrang wie auch Harninkontinenz werden als extrem unangenehm empfunden und es besteht ein deutlicher Bedarf bei von diesen Indikationen betroffenen Personen, eine möglichst langfristige Verbesserung zu erreichen.

Üblicherweise werden vermehrter Harndrang und insbesondere Harninkontinenz medikamentös mit Substanzen behandelt, die an den Reflexen des unteren Harntraktes beteiligt sind (Wein, A. J., 1998, Urology 51 (Suppl. 21): 43-47). Meistens sind dies Medikamente, die eine hemmende Wirkung auf den Detrusormuskel, der für den inneren Blasendruck verantwortlich ist, haben. Diese Medikamente sind z. B. Parasympatholytika wie Oxybutynin, Propiverin oder Tolterodin, trizyklische Antidepressiva wie Imipramin oder Muskelrelaxantien wie Flavoxat. Andere Medikamente, die insbesondere den Widerstand der Harnröhre oder des Blasenhalses erhöhen, zeigen Affinitäten zu α-Adrenorezeptoren wie Ephedrin, zu β-Adrenorezeptoren wie Clenbutarol oder sind Hormone wie Östradiol. Geeignet sind auch NMDA-Rezeptor-Antagonisten wie Dextrometorphan und Dextrorphan alleine oder in Kombination mit anderen oben genannten Wirkstoffen (WO 9627375).

Einen genauen Einblick in die verwendeten Therapeutika und Therapiemethoden, insbesondere bezüglich der Antimuskarinika und anderer peripher wirkender Stoffe, geben hier der Übersichtsartikel von K.E. Andersson et al. "The pharmacological treatment of urinary incontinence",. BJU International (1999), 84, 923 - 947, Butera J.A. et al, Expert Opinion on Therapeutic Patents (1998) 8/8 (1017-1035), Smith, C. P. and Chancellor, M. B., Expert Opinion on Therapeutic Patents (2001) 11(1). 17-31

Auch bestimmte Diarylmethylpiperazine und -piperidine sind für diese Indikation in der WO 93/15062 beschrieben. Ebenso wurde für Tramadol und einige seiner Metaboliten ein positiver Effekt auf die Blasenfunktion in einem Rattenmodell rhythmischer Blasenkontraktionen bzw. durch cystometrische Untersuchungen nachgewiesen (Nippon-Shinyaku, WO 98/46216; WO 0124783). Weiterhin gibt es in der Literatur Untersuchungen zur Charakterisierung der opioiden Nebenwirkung Harnretention, woraus sich einige Hinweise auf die Beeinflussung der Blasenfunktionen durch schwache Opioide wie Diphenoxylat (Fowler et al., 1987 J. Urol 138:735-738) und Meperidin (Doyle and Briscoe, 1976 Br J Urol 48:329-335), durch gemischte Opioidagonisten / -antagonisten wie Buprenorphin (Malinovsky et al., 1998 Anesth Analg 87:456-461; Drenger and Magora, 1989 Anesth Analg 69:348-353), Pentazocin (Shimizu et al. (2000) Br. J. Pharmacol. 131 (3): 610 - 616) und Nalbuphin (Malinovsky et al., 1998, a.a.O), sowie durch starke Opioide wie Morphin (Malinovsky et al., 1998 a.a.O; Kontani und Kawabata, (1988); Jpn J Pharmacol. Sep;48(1):31; Dray and Metsch J. Pharmacol Exp Ther1984, 231, 254-260) und Fentanyl (Malinovsky et al., 1998 a.a.O) ergeben. Allerdings erfolgten diese Untersuchungen zumeist in analgetisch wirksamen Konzentrationen.

Bei den hier in Frage kommenden Indikationen ist zu beachten, daß es sich im allgemeinen um sehr langfristige medikamentöse Anwendungen handelt und sich die Betroffenen im Gegensatz zu vielen Situationen, in denen Analgetika eingesetzt werden, einer sehr unangenehmen, aber nicht unaushaltbaren Situation gegenüber sehen. Daher ist hier - noch mehr als bei Analgetika - darauf zu achten, Nebenwirkungen zu vermeiden, will der Betroffene nicht ein Übel gegen das andere tauschen. Auch sind bei einer dauerhaften Harninkontinenzbehandlung auch analgetische Wirkungen weitgehend unerwünscht.

Aus dem Stand der Technik sind bereits Kombinationen von Opioiden mit Antimuskarinika bekannt, wobei jedoch die Behandlung der Harninkontinenz nicht offenbart ist. So offenbart DE 3129982 eine Kombination aus Atropinsulfat und Diphenoxylat-HCl als Antiallergikum. GB 857194 offenbart die Kombination von Morphin und Atropin gemeinsam mit lonenaustauscherharzen, wodurch die Freisetzung der Verbindungen beeinflusst wird. Auch hier wird kein Hinweis auf die Möglichkeit einer Verwendung gegen Harninkontinenz gegeben.

Aufgabe der vorliegenden Erfindung war es daher, Stoffe oder Stoffkombinationen aufzufinden, die zur Behandlung von vermehrtem Harndrang bzw. Harninkontinenz hilfreich sind und bei den wirksamen Dosen bevorzugt gleichzeitig geringere Nebenwirkungen und/oder analgetische Wirkungen zeigen als aus dem Stand der Technik bekannt, insbesondere einen synergistischen Effekt zur Behandlung der Harninkontinenz zeigen.

Überraschenderweise wurde nun gefunden, daß eine Kombination aus Verbindungen der Gruppe A, die Opioide und andere zentralwirkende Substanzen, die mit Opioid-Rezeptoren wechselwirken und deren Effekte durch Naloxon antagonisiert werden können, oder insbesondere Substanzen, die über einen Opiat-Rezeptor, insbesondere den µ-Rezeptor, wirken, umfaßt, und Verbindungen der Gruppe B, die Muskarinantagonisten, und andere überwiegend peripher wirkende, in der Harninkontinenz bekanntermaßen wirksame Substanzen umfaßt, eine hervorragende Wirkung auf die Blasenfunktion besitzen. Weiter erwiesen sich diese Kombinationen - deutlich über das Erwartete hinaus - bereits bei sehr geringen Dosen als so wirksam, daß die kombinierten Wirkstoffe niedrig dosiert eingesetzt werden konnten. Dadurch gehen bei therapeutischer Anwendung sonst bei höheren notwendigen Dosierungen auftretende Nebenwirkungen deutlich zurück, während die therapeutische Wirkung durch diese Kombination aus peripherem, überwiegend direkt auf die Blase oder Blasenmuskulatur wirkendem, antimuskarinem Effekt und zentralem Opioid-Effekt bzw. µ-Rezeptor-Effekt voll erhalten bleibt.

Dementsprechend ist Erfindungsgegenstand die Verwendung einer Wirkstoffkombination aus wenigstens einer der **Verbindungen A** und wenigstens einer der **Verbindungen B,** mit **Verbindung A** ausgewählt aus:
**Gruppe a)** enthaltend: Tramadol, O-Demethyltramadol, oder O-desmethyl-N-mono-desmethyl-tramadol gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in Form ihrer Säuren oder ihrer Basen oder in Form ihrer Salze, insbesondere der physiologisch verträglichen Salze, oder in Form ihrer Solvate, insbesondere der Hydrate;
**Gruppe b)** enthaltend:
   - Codein
   - Dextropropxyphen
   - Dihydrocodein
   - Diphenoxylat
   - Ethylmorphin
   - Meptazinol
   - Nalbuphin
   - Pethidin (Meperidine)
   - Tilidin
   - Tramadol
   - Viminol
   - Butorphanol
   - Dextromoramid
   - Dezocin
   - Diacetylmorphin (Heroin)
   - Hydrocodon
   - Hydromorphon
   - Ketobemidon
   - Levomethadon
   - Levomethadyl-Acetate (I-α-Acetylmethadol (LAAM))
   - Levorphanol
   - Morphin
   - Nalorphin
   - Oxycodon
   - Pentazocin
   - Piritramide
   - Alfentanil
   - Buprenorphin
   - Etorphin
   - Fentanyl
   - Remifentanil
   - Sufentanil
   gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in Form ihrer Säuren oder ihrer Basen oder in Form ihrer Salze, insbesondere der physiologisch verträglichen Salze, oder in Form ihrer Solvate, insbesondere der Hydrate;
**Gruppe c)** enthaltend:
   1-Phenyl-3-dimethylamino-propanverbindungen gemäß allgemeiner **Formel I** 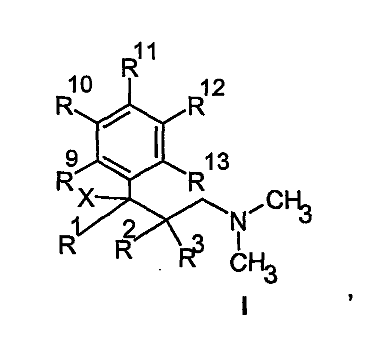
   worin
   X ausgewählt ist aus OH, F, Cl, H oder OC(O)R⁷ mit R⁷ ausgewählt aus C₁₋₃-Alkyl, verzweigt oder unverzweigt, gesättigt oder ungesättigt, unsubstituiert oder ein- oder mehrfach substituiert,
   R¹ ausgewählt ist aus C₁₋₄-Alkyl, verzweigt oder unverzweigt, gesättigt oder ungesättigt, unsubstituiert oder ein- oder mehrfach substituiert,
   R² und R³ jeweils unabhängig voneinander ausgewählt sind aus H oder C₁₋₄-Alkyl, verzweigt oder unverzweigt, gesättigt oder ungesättigt, unsubstituiert oder ein- oder mehrfach substituiert,
      oder
   R² und R³ zusammen einen gesättigten C₄₋₇-Cycloalkylrest bilden, unsubstituiert oder ein- oder mehrfach substituiert,
   R⁹ bis R¹³ jeweils unabhängig voneinander ausgewählt sind aus H, F, Cl, Br, I, CH₂F, CHF₂, CF₃, OH, SH, OR¹⁴, OCF₃, SR¹⁴, NR¹⁷R¹⁸, SOCH₃, SOCF₃; SO₂CH₃, SO₂CF₃, CN, COOR¹⁴, NO₂, CONR¹⁷R¹⁸; C₁₋₆-Alkyl, verzweigt oder unverzweigt, gesättigt oder ungesättigt, unsubstituiert oder ein-oder mehrfach substituiert; Phenyl, unsubstituiert oder ein- oder mehrfach substituiert;
      mit R¹⁴ ausgewählt aus C₁₋₆-Alkyl; Pyridyl, Thienyl, Thiazolyl, Phenyl, Benzyl oder Phenethyl, jeweils unsubstituiert oder ein- oder mehrfach substituiert; PO(O-C₁₋₄-Alkyl)₂, CO(OC₁₋₅-Alkyl), CONH-C₆H₄-(C₁₋₃-Alkyl), CO(C₁₋₅-Alkyl), CO-CHR¹⁷-NHR¹⁸, CO-C₆H₄-R¹⁵, mit R¹⁵ ortho-OCOC₁₋₃-Alkyl oder meta- oder para-CH₂N(R¹⁶)₂ mit R¹⁶ C₁₋₄-Alkyl oder 4-Morpholino, wobei in den Resten R¹⁴, R¹⁵ und R¹⁶ die Alkylgruppen verzweigt oder unverzweigt, gesättigt oder ungesättigt, unsubstituiert oder ein- oder mehrfach substituiert sein können;
      mit R¹⁷ und R18 jeweils unabhängig voneinander ausgewählt aus H; C₁₋₆-Alkyl, verzweigt oder unverzweigt, gesättigt oder ungesättigt, unsubstituiert oder ein- oder mehrfach substituiert; Phenyl, Benzyl oder Phenethyl, jeweils unsubstituiert oder ein- oder mehrfach substituiert,
      oder
   R⁹ und R¹⁰ oder R¹⁰ und R¹¹ zusammen einen OCH₂O-, OCH₂CH₂O-, OCH=CH-, CH=CHO-, CH=C(CH3)O-, OC(CH3)=CH-, (CH₂)₄- oder OCH=CHO-Ring bilden,
   gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form ihrer Säuren oder ihrer Basen oder in Form ihrer Salze, insbesondere der physiologisch verträglichen Salze, oder in Form ihrer Solvate, insbesondere der Hydrate;
**Gruppe d)** enthaltend:
   substituierte 6-Dimethylaminomethyl-1-phenylcyclohexanverbindungen gemäß allgemeiner **Formel II** 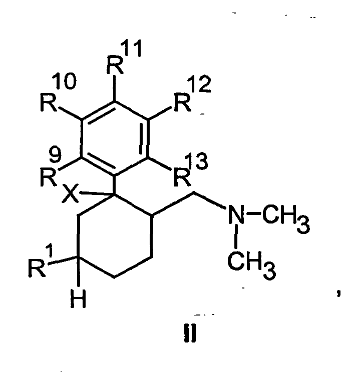
   worin
   X ausgewählt ist aus OH, F, Cl, H oder OC(O)R⁷ mit R⁷ ausgewählt aus C₁₋₃-Alkyl, verzweigt oder unverzweigt, gesättigt oder ungesättigt, unsubstituiert oder ein- oder mehrfach substituiert,
   R¹ ausgewählt ist aus C₁₋₄-Alkyl, Benzyl, CF₃, OH, OCH₂-C₆H₅, O-C₁₋₄-Alkyl, Cl oder F und
   R⁹ bis R¹³ jeweils unabhängig voneinander ausgewählt sind aus H, F, Cl, Br, I, CH₂F, CHF₂, CF₃, OH, SH, OR¹⁴, OCF₃, SR¹⁴, NR¹⁷R¹⁸, SOCH₃, SOCF_{3;} SO₂CH₃, SO₂CF₃, CN, COOR¹⁴, NO₂, CONR¹⁷R¹⁸_{;} C₁₋₆-Alkyl, verzweigt oder unverzweigt, gesättigt oder ungesättigt, unsubstituiert oder ein- oder mehrfach substituiert; Phenyl, unsubstituiert oder ein- oder mehrfach substituiert;
      mit R¹⁴ ausgewählt aus C₁₋₆-Alkyl; Pyridyl, Thienyl, Thiazolyl, Phenyl, Benzyl oder Phenethyl, jeweils unsubstituiert oder ein- oder mehrfach substituiert; PO(O-C₁₋₄-Alkyl)₂, CO(OC₁₋₅-Alkyl), CONH-C₆H₄-(C₁₋₃-Alkyl), CO(C₁₋₅-Alkyl), CO-CHR¹⁷-NHR¹⁸, CO-C₆H₄-R¹⁵, mit R¹⁵ ortho-OCOC₁₋₃-Alkyl oder meta- oder para-CH₂N(R¹⁶)₂ mit R¹⁶ C₁₋₄-Alkyl oder 4-Morpholino, wobei in den Resten R¹⁴, R¹⁵ und R¹⁶ die Alkylgruppen verzweigt oder unverzweigt, gesättigt oder ungesättigt, unsubstituiert oder ein- oder mehrfach substituiert sein können;
      mit R¹⁷ und R18 jeweils unabhängig voneinander ausgewählt aus H; C₁₋₆-Alkyl, verzweigt oder unverzweigt, gesättigt oder ungesättigt, unsubstituiert oder ein- oder mehrfach substituiert; Phenyl, Benzyl oder Phenethyl, jeweils unsubstituiert oder ein- oder mehrfach substituiert,
      oder
   R⁹ und R¹⁰ oder R¹⁰ und R¹¹ zusammen einen OCH₂O-, OCH₂CH₂O-, OCH=CH-, CH=CHO-, CH=C(CH3)O-, OC(CH3)=CH-, (CH₂)₄- oder OCH=CHO-Ring bilden,
   gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form ihrer Säuren oder ihrer Basen oder in Form ihrer Salze, insbesondere der physiologisch verträglichen Salze, oder in Form ihrer Solvate, insbesondere der Hydrate;
   **und/oder**
**Gruppe e)** enthaltend:
   6-Dimethylaminomethyl-1-phenyl-cyclohexanverbindungen gemäß allgemeiner **Formel III** 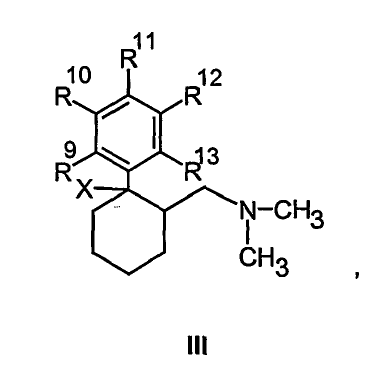
   worin
   X ausgewählt ist aus OH, F, Cl, H oder OC(O)R⁷ mit R⁷ ausgewählt aus C₁₋₃-Alkyl, verzweigt oder unverzweigt, gesättigt oder ungesättigt, unsubstituiert oder ein- oder mehrfach substituiert, und
   R⁹ bis R¹³ jeweils unabhängig voneinander ausgewählt sind aus H, F, Cl, Br, I, CH₂F, CHF₂, CF₃, OH, SH, OR¹⁴, OCF₃, SR¹⁴, NR¹⁷R¹⁸, SOCH₃, SOCF₃; SO₂CH₃, SO₂CF₃, CN, COOR¹⁴, NO₂, CONR¹⁷R¹⁸; C₁₋₆-Alkyl, verzweigt oder unverzweigt, gesättigt oder ungesättigt, unsubstituiert oder ein-oder mehrfach substituiert; Phenyl, unsubstituiert oder ein- oder mehrfach substituiert;
      mit R¹⁴ ausgewählt aus C₁₋₆-Alkyl; Pyridyl, Thienyl, Thiazolyl, Phenyl, Benzyl oder Phenethyl, jeweils unsubstituiert oder ein- oder mehrfach substituiert; PO(O-C₁₋₄-Alkyl)₂, CO(OC₁₋₅-Alkyl), CONH-C₆H₄-(C₁₋₃-Alkyl), CO(C₁₋₅-Alkyl), CO-CHR¹⁷-NHR¹⁸, CO-C₆H₄-R¹⁵, mit R¹⁵ ortho-OCOC₁₋₃-Alkyl oder meta- oder para-CH₂N(R¹⁶)₂ mit R¹⁶ C₁₋₄-Alkyl oder 4-Morpholino, wobei in den Resten R¹⁴, R¹⁵ und R¹⁶ die Alkylgruppen verzweigt oder unverzweigt, gesättigt oder ungesättigt, unsubstituiert oder ein- oder mehrfach substituiert sein können;
      mit R¹⁷ und R18 jeweils unabhängig voneinander ausgewählt aus H; C₁₋₆-Alkyl, verzweigt oder unverzweigt, gesättigt oder ungesättigt, unsubstituiert oder ein- oder mehrfach substituiert; Phenyl, Benzyl oder Phenethyl, jeweils unsubstituiert oder ein- oder mehrfach substituiert,
      oder
   R⁹ und R¹⁰ oder R¹⁰ und R¹¹ zusammen einen OCH₂O-, OCH₂CH₂O-, OCH=CH-, CH=CHO-, CH=C(CH3)O-, OC(CH3)=CH-, (CH₂)₄- oder OCH=CHO-Ring bilden,
   mit der Maßgabe, daß, wenn R⁹, R¹¹ und R¹³ H entsprechen, und einer von R¹⁰ oder R¹² H und der andere OCH₃ entspricht, X nicht OH sein darf,
   gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form ihrer Säuren oder ihrer Basen oder in Form ihrer Salze, insbesondere der physiologisch verträglichen Salze, oder in Form ihrer Solvate, insbesondere der Hydrate;
   **und** mit wenigstens einer der **Verbindungen B,** ausgewählt aus:
   den Antimuskarinika: Atropin, Oxybutinin, Propiverin, Propanthelin, Emepronium, Trospium, Tolterodin, Darifenacin und α,α-Diphenylessigsäure-4-(N-methylpiperidyl)-ester, sowie Duloxetin, Imipramin und Desmopressin,
   gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in Form ihrer Säuren oder ihrer Basen oder in Form ihrer Salze, insbesondere der physiologisch verträglichen Salze, oder in Form ihrer Solvate, insbesondere der Hydrate;
   zur Herstellung eines Arzneimittels zur Behandlung von vermehrtem Harndrang bzw. Harninkontinenz.

Überraschenderweise hatte sich herausgestellt, daß die Kombination der genannten Substanzen bestimmte physiologische Parameter, die bei vermehrtem Harndrang bzw. Harninkontinenz von Bedeutung sind, deutlich positiv beeinflußen. Jede einzelne dieser Veränderungen kann eine deutliche Erleichterung im symptomatischen Bild von betroffener Patienten bedeuten.

Im Sinne dieser Erfindung versteht man unter Alkyl- bzw. Cykloalkyl-Resten gesättigte und ungesättigte (aber nicht aromatische), verzweigte, unverzweigte und cyclische Kohlenwasserstoffe, die unsubstituiert oder ein- oder mehrfach substituiert sein können. Dabei steht C₁₋₂-Alkyl für C1- oder C2-Alkyl, C₁₋₃-Alkyl für C1-, C2- oder C3-Alkyl, C₁₋₄-Alkyl für C1-, C2-, C3- oder C4-Alkyl, C₁₋₅-Alkyl für C1-, C2-, C3-, C4- oder C5-Alkyl, C₁₋₆-Alkyl für C1-, C2-, C3-, C4-, C5- oder C6-Alkyl, C₁₋₇-Alkyl für C1-, C2-, C3-, C4-, C5-, C6- oder C7-Alkyl, C₁₋₈-Alkyl für C1-, C2-, C3-, C4-, C5-, C6-, C7- oder C8-Alkyl, C₁₋₁₀-Alkyl für C1-, C2-, C3-, C4-, C5-, C6-, C7-, C8,- C9- oder C10-Alkyl und C₁₋₁₈-Alkyl für C1-, C2-, C3-, C4-, C5-, C6-, C7-, C8,- C9-, C10-, C11-, C12-, C13-, C14-, C15-, C16-, C17- oder C18-Alkyl. Weiter steht C₃₋₄-Cycloalkyl für C3- oder C4-Cycloalkyl, C₃₋₅-Cycloalkyl für C3-, C4- oder C5-Cycloalkyl, C₃₋₆-Cycloalkyl für C3-, C4-, C5- oder C6-Cycloalkyl, C₃₋₇-Cycloalkyl für C3-, C4-, C5-, C6- oder C7-Cycloalkyl, C₃₋₈-Cycloalkyl für C3-, C4-, C5-, C6-, C7- oder C8-Cycloalkyl, C₄₋₅-Cycloalkyl für C4- oder C5-Cycloalkyl, C₄₋₆-Cycloalkyl für C4-, C5- oder C6-Cycloalkyl, C₄₋₇-Cycloalkyl für C4-, C5-, C6- oder C7-Cycloalkyl, C₅₋₆-Cycloalkyl für C5- oder C6-Cycloalkyl und C₅₋₇-Cycloalkyl für C5-, C6- oder C7-Cycloalkyl. In Bezug auf Cycloalkyl umfaßt der Begriff auch gesättigte Cycloalkyle, in denen ein oder 2 Kohlenstoffatome durch ein Heteroatom, S,-N oder O ersetzt sind. Unter den Begriff Cycloalkyl fallen aber insbesondere auch ein- oder mehrfach, vorzugsweise einfach, ungesättigte Cycloalkyle ohne Heteroatom im Ring, solange das Cycloalkyl kein aromatisches System darstellt. Vorzugsweise sind die Alkyl- bzw. Cykloalkyl-Reste Methyl, Ethyl, Vinyl (Ethenyl), Propyl, Allyl (2-Propenyl), 1-Propinyl, Methylethyl, Butyl, 1-Methylpropyl, 2-Methylpropyl, 1,1-Dimethylethyl, Pentyl, 1,1-Dimethylpropyl, 1,2-Dimethylpropyl, 2,2-Dimethylpropyl, Hexyl, 1-Methylpentyl, Cyclopropyl, 2-Methylcyclopropyl, Cyclopropylmethyl, Cyclobutyl, Cyclopentyl, Cyclopentylmethyl, Cyclohexyl, Cycloheptyl, Cyclooctyl, aber auch Adamantyl, CHF₂, CF₃ oder CH₂OH sowie Pyrazolinon, Oxopyrazolinon, [1,4]Dioxan oder Dioxolan.

Dabei versteht man im Zusammenhang mit Alkyl und Cycloalkyl - solange dies nicht ausdrücklich anders definiert ist - unter dem Begriff substituiert im Sinne dieser Erfindung die Substitution mindestens eines (gegebenenfalls auch mehrerer) Wasserstoffreste(s) durch F, Cl, Br, l, NH₂, SH oder OH, wobei unter "mehrfach substituiert" bzw. "substituiert" bei mehrfacher Substitution zu verstehen ist, daß die Substitution sowohl an verschiedenen als auch an gleichen Atomen mehrfach mit den gleichen oder verschiedenen Substituenten erfolgt, beispielsweise dreifach am gleichen C-Atom wie im Falle von CF₃ oder an verschiedenen Stellen wie im Falle von -CH(OH)-CH=CH-CHCl₂. Besonders bevorzugte Substituenten sind hier F, Cl und OH. In Bezug auf Cycloalkyl kann der Wasserstoffrest auch durch OC₁₋₃-Alkyl oder C₁₋₃-Alkyl (jeweils ein- oder mehrfach substituiert oder unsubstituiert), insbesondere Methyl, Ethyl, n-Propyl, i-Propyl, CF₃, Methoxy oder Ethoxy, ersetzt sein.

Unter dem Begriff (CH₂)₃₋₆ ist -CH₂-CH₂-CH₂-, -CH₂-CH₂-CH₂-CH₂-, -CH₂-CH₂-CH₂-CH₂-CH₂- und CH₂-CH₂-CH₂-CH₂-CH₂-CH₂- zu verstehen, unter (CH₂)₁₋₄ ist -CH₂-, -CH₂-CH₂-, -CH₂-CH₂-CH₂- und -CH₂-CH₂-CH₂-CH₂- zu verstehen, unter (CH₂)₄₋₅ ist -CH₂-CH₂-CH₂-CH₂- und -CH₂-CH₂-CH₂-CH₂-CH₂- zu verstehen, etc.

Unter einem Aryl-Rest werden Ringsysteme mit mindestens einem armomatischen Ring aber ohne Heteroatome in auch nur einem der Ringe verstanden. Beispiele sind Phenyl-, Naphthyl-, Fluoranthenyl-, Fluorenyl-, Tetralinyl- oder Indanyl, insbesondere 9H-Fluorenyl- oder Anthracenyl-Reste, die unsubstituiert oder einfach oder mehrfach substituiert sein können.

Unter einem Heteroaryl-Rest werden heterocyclische Ringsysteme mit mindestens einem ungesättigten Ring verstanden, die ein oder mehrere Heteroatome aus der Gruppe Stickstoff, Sauerstoff und/oder Schwefel enthalten und auch einfach oder mehrfach substituiert sein können. Beispielhaft seien aus der Gruppe der Heteroaryle Furan, Benzofuran, Thiophen, Benzothiophen, Pyrrol, Pyridin, Pyrimidin, Pyrazin, Chinolin, Isochinolin, Phthalazin, Benzo-1,2,5 thiadiazol, Benzothiazol, Indol, Benzotriazol, Benzodioxolan, Benzodioxan, Carbazol, Indol und Chinazolin aufgeführt.

Dabei versteht man im Zusammenhang mit Aryl und Heteroaryl unter substituiert die Substitution des Aryls oder Heteroaryls mit R²³, OR²³ einem Halogen, vorzugsweise F und/oder Cl, einem CF₃, einem CN, einem NO₂, einem NR²⁴R²⁵, einem C₁₋₆-Alkyl (gesättigt), einem C₁₋₆-Alkoxy, einem C₃₋₈-Cycloalkoxy, einem C₃₋₈-Cycloalkyl oder einem C₂₋₆-Alkylen.

Dabei steht der Rest R²³ für H, einen C₁₋₁₀-Alkyl-, vorzugsweise einen C₁₋₆-Alkyl-, einen Aryl- oder Heteroaryl- oder für einen über eine C₁₋₃-Alkylen-Gruppe gebundenen Aryl- oder Heteroaryl-Rest, wobei diese Aryl und Heteroarylreste nicht selbst mit Aryl- oder Heteroaryl-Resten substituiert sein dürfen,
die Reste R²⁴ und R²⁵, gleich oder verschieden, für H, einen C₁₋₁₀-Alkyl-, vorzugsweise einen C₁₋₆-Alkyl-, einen Aryl-, einen Heteroaryl- oder einen über eine C₁₋₃-Alkylen-Gruppe gebundenen Aryl- oder Heteroaryl-Rest bedeuten, wobei diese Aryl und Heteroarylreste nicht selbst mit Aryl- oder Heteroaryl-Resten substituiert sein dürfen,
oder die Reste R²⁴ und R²⁵ bedeuten zusammen CH₂CH₂OCH₂CH₂, CH₂CH₂NR²⁶CH₂CH₂ oder (CH₂)₃₋₆, und
der Rest R²⁶ für H, einen C₁₋₁₀-Alkyl-, vorzugsweise einen C₁₋₆-Alkyl-, einen Aryl-, oder Heteroaryl- Rest oder für einen über eine C₁₋₃-AlkylenGruppe gebundenen Aryl- oder Heteroaryl-Rest, wobei diese Aryl und Heteroarylreste nicht selbst mit Aryl- oder Heteroaryl-Resten substituiert sein dürfen.

Unter dem Begriff Salz ist im Sinne dieser Erfindung jegliche Form des erfindungsgemäßen Wirkstoffes zu verstehen, in dem dieser eine ionische Form annimmt bzw. geladen ist und mit einem Gegenion (einem Kation oder Anion) gekoppelt ist bzw. sich in Lösung befindet. Darunter sind auch Komplexe des Wirkstoffes mit anderen Molekülen und Ionen zu verstehen, insbesondere Komplexe, die über ionische Wechselwirkungen komplexiert sind.

Unter dem Begriff des physiologisch verträglichen Salzes (insbesondere mit Kationen oder Basen) versteht man im Sinne dieser Erfindung Salze mindestens einer der erfindungsgemäßen Verbindungen - meist einer (deprotonierten) Säure - als Anion mit mindestens einem, vorzugsweise anorganischen, Kation, die physiologisch - insbesondere bei Anwendung im Menschen und/oder Säugetier - verträglich sind. Besonders bevorzugt sind die Salze der Alkali- und Erdalkalimetalle aber auch mit NH₄⁺, insbesondere aber (Mono-) oder (Di-) Natrium-, (Mono-) oder (Di-) Kalium-, Magnesium- oder Calzium-Salze.

Unter dem Begriff des physiologisch verträglichen Salzes (insbesondere mit Anionen oder Säuren) versteht man im Sinne dieser Erfindung weiter Salze mindestens einer der erfindungsgemäßen Verbindungen - meist, beispielsweise am Stickstoff, protoniert - als Kation mit mindestens einem Anion, die physiologisch - insbesondere bei Anwendung im Menschen und/oder Säugetier - veträglich sind. Insbesondere versteht man darunter im Sinne dieser Erfindung das mit einer physiologisch verträglichen Säure gebildete Salz, nämlich Salze des jeweiligen Wirkstoffes mit anorganischen bzw. organischen Säuren, die physiologisch - insbesondere bei Anwendung im Menschen und/oder Säugetier - verträglich sind. Beispiele für physiologisch verträgliche Salze bestimmter Säuren sind Salze der: Salzsäure, Bromwasserstoffsäure, Schwefelsäure, Methansulfonsäure, Ameisensäure, Essigsäure, Oxalsäure, Bernsteinsäure, Apfelsäure, Weinsäure, Mandelsäure, Fumarsäure, Milchsäure, Zitronensäure, Glutaminsäure, 1,1-Dioxo-1,2-dihydro1b6-benzo[d]isothiazol-3-on (Saccharinsäure), Monomethylsebacinsäure, 5-Oxo-prolin, Hexan-1-sulfonsäure, Nicotinsäure, 2-, 3- oder 4-Aminobenzoesäure, 2,4,6-Trimethyl-benzoesäure, a-Liponsäure, Acetylglycin, Acetylsalicylsäure, Hippursäure und/oder Asparaginsäure. Besonders bevorzugt ist das Hydrochlorid-Salz.

Geeignete Salze im Sinne dieser Erfindung und in jeder beschriebenen Verwendung und jedem der beschriebenen Arzneimittel sind Salze des jeweiligen Wirkstoffes mit anorganischen bzw. organischen Säuren und/oder einem Zuckeraustauschstoff wie Saccharin, Cyclamat oder Acesulfam. Besonders bevorzugt ist jedoch das Hydrochlorid.

Verbindungen der **Gruppe c)** und deren Herstellung sind aus der DE 44 26 245 A1 bzw. der US 6,248,737 bekannt. Verbindungen der **Gruppe d)** und **e)** und deren Herstellung sind aus der DE 195 25 137 A1 bzw. US 5,733,936 bzw. US RE37355E bekannt.

In einer bevorzugten Ausführungsform gilt für die erfindungsgemäße Verwendung, daß die **Verbindung A** in **Gruppe a)** ausgewählt ist aus:
Tramadol, (+)-Tramadol, (+)-O-Demethyltramadol oder (+)-O-desmethyl-N-mono-desmethyl-tramadol,
vorzugsweise Tramadol oder (+)-Tramadol,
insbesondere (+)-Tramadol.

In einer bevorzugten Ausführungsform gilt für die erfindungsgemäße Verwendung, daß die **Verbindung A** in **Gruppe b)** ausgewählt ist aus:
- Codein
- Dextropropxyphen
- Dihydrocodein
- Diphenoxylat
- Ethylmorphin
- Meptazinol
- Nalbuphin
- Pethidin (Meperidine)
- Tilidin
- Viminol
- Butorphanol
- Dezocin
- Nalorphin
- Pentazocin
- Buprenorphin
vorzugsweise
- Codein
- Dextropropxyphen
- Dihydrocodein
- Meptazinol
- Nalbuphin
- Tilidin
- Buprenorphin

In einer bevorzugten Ausführungsform gilt für die erfindungsgemäße Verwendung, daß die **Verbindung A** in **Gruppe c)** ausgewählt ist aus Verbindungen gemäß **Formel I** für die gilt:
X ausgewählt ist aus
   OH, F, Cl, OC(O)CH₃ oder H, vorzugsweise OH, F, OC(O)CH₃ oder H,
   **und/oder**
R¹ ausgewählt ist aus
   C₁₋₄-Alkyl, gesättigt und unsubstituiert, verzweigt oder unverzweigt; vorzugsweise CH₃, C₂H₅, C₄H₉ oder t-Butyl, insbesondere CH₃ oder C₂H₅,
   **und/oder**
R² und R³ unabhängig voneinander ausgewählt sind aus
   H, C₁₋₄-Alkyl, gesättigt und unsubstituiert, verzweigt oder unverzweigt; vorzugsweise H, CH₃, C₂H₅, i-Propyl oder t-Butyl, insbesondere H oder CH₃, vorzugsweise R³ = H,
      oder
   R² und R³ zusammen einen C₅₋₆-Cycloalkylrest bilden, gesättigt oder ungesättigt, unsubstituiert oder ein- oder mehrfach substituiert, vorzugsweise gesättigt und unsubstituiert, insbesondere Cyclohexyl.
   **und/oder**
R⁹ bis R¹³, wobei 3 oder 4 der Reste R⁹ bis R¹³ H entsprechen müssen, unabhängig voneinander ausgewählt sind aus
   H, Cl, F, OH, CF₂H, CF₃ oder C₁₋₄-Alkyl, gesättigt und unsubstituiert, verzweigt oder unverzweigt; OR¹⁴ oder SR¹⁴, mit R¹⁴ ausgewählt aus C₁₋₃-Alkyl, gesättigt und unsubstituiert, verzweigt oder unverzweigt;
      vorzugsweise H, Cl, F, OH, CF₂H, CF₃, OCH₃ oder SCH₃
   oder R¹² und R¹¹ einen 3,4-OCH=CH-Ring bilden
   insbesondere
   wenn R⁹, R¹¹ und R¹³ H entsprechen, einer von R¹⁰ oder R¹² auch H entspricht, während der andere ausgewählt ist aus:
      Cl, F, OH, CF₂H, CF₃, OR¹⁴ oder SR¹⁴, vorzugsweise OH, CF₂H, OCH₃ oder SCH₃
      oder,
   wenn R⁹ und R¹³ H entsprechen und R¹¹ OH, OCH₃, Cl oder F, vorzugsweise Cl, entspricht, einer von R¹⁰ oder R¹² auch H entspricht, während der andere OH, OCH₃, Cl oder F, vorzugsweise Cl, entspricht,
      oder,
   wenn R⁹, R¹⁰, R¹² und R¹³ H entsprechen, R¹¹ ausgewählt ist aus CF₃, CF₂H, Cl oder F, vorzugsweise F,
      oder,
   wenn R¹⁰, R¹¹ und R¹² H entsprechen, einer von R⁹ oder R¹³ auch H entspricht, während der andere ausgewählt ist aus OH, OC₂H₅ oder OC₃H₇.

Dabei ist es für Verbindungen der **Gruppe c)** besonders bevorzugt, wenn gilt, daß Verbindungen der **Formel I** mit R³ = H in Form der Diastereomeren mit der relativen Konfiguration la 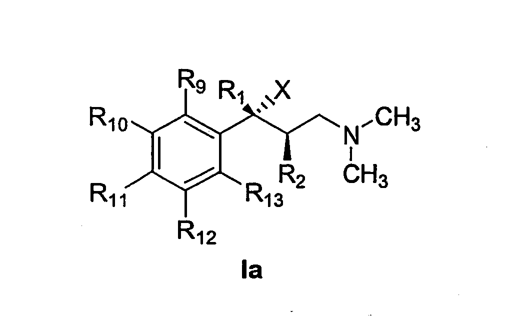 vorliegen, insbesondere in Mischungen mit höherem Anteil dieses Diastereomeren im Vergleich zum anderen Diastereomeren oder als reines Diastereomer verwendet werden
**und/oder**
daß die Verbindungen der **Formel I** in Form des (+)-Enantiomeren, insbesondere in Mischungen mit höherem Anteil des (+)-Enantiomeren im Vergleich zum (-)-Enantiomeren einer racemischen Verbindung oder als reines (+)-Enantiomer verwendet werden.

Dabei ist es besonders bevorzugt, wenn **Verbindung A** ausgewählt aus folgender Gruppe verwendet wird:
■ (2RS,3RS)-1-Dimethylamino-3-(3-methoxy-phenyl)-2-methyl-pentan-3-ol,
■ (2R,3R)-1-Dimethylamino-3-(3-methoxy-phenyl)-2-methyl-pentan-3-ol,
■ (+)-(2R,3R)-1-Dimethylamino-3-(3-methoxy-phenyl)-2-methyl-pentan-3-ol,
■ (2RS,3RS)-3-(3,4-Dichlorophenyl)-1-dimethylamino-2-methyl-pentan-3-ol,
■ (2RS,3RS)-3-(3-Difluoromethyl-phenyl)-1-dimethylamino-2-methyl-pentan-3-ol,
■ (2RS,3RS)-1-Dimethylamino-2-methyl-3-(3-methylsulfanylphenyl)-pentan-3-ol,
■ (3RS)-1-Dimethylamirio-3-(3-methoxy-phenyl)-4,4-dimethylpentan-3-ol,
■ (2RS,3RS)-3-(3-Dimethylamino-1-ethyl-1-hydroxy-2-methylpropyl)-phenol,
■ (1RS,2RS)-3-(3-Dimethylamino-1-hydroxy-1,2-dimethyl-propyl)-phenol,
■ (+)-(1R,2R)-3-(3-Dimethylamino-1-hydroxy-1,2-dimethylpropyl)-phenol,
■ (+)-(1R,2R)-3-(3-Dimethylamino-1-hydroxy-1,2-dimethyl-propyl)-phenol,
■ (1R,2R)-3-(3-Dimethylamino-1-ethyl-2-methyl-propyl)-phenol,
■ (-)-(1R,2R)-3-(3-Dimethylamino-1-ethyl-2-methyl-propyl)-phenol,
■ (1S,2S)-3-(3-Dimethylamino-1-ethyl-2-methyl-propyl)-phenol,
■ (+)-(1S,2S)-3-(3-Dimethylamino-1-ethyl-2-methyl-propyl)-phenol,
■ (+)-(1R,2R)-Essigsäure-3-dimethylamino-1-ethyl-1-(3-methoxy-phenyl)-2-methyl-propylester;
■ (1RS)-1-(1-Dimethylaminomethyl-cyclohexyl)-1-(3-methoxy-phenyl)-propan-1-ol,
■ (2RS, 3RS)-3-(4-Chlorophenyl)-1-dimethylamino-2-methyl-pentan-3-ol,
■ (+)-(2R,3R)-3-(3-Dimethylamino-1-ethyl-1-hydroxy-2-methylpropyl)-phenol,
■ (2RS,3RS)-4-Dimethylamino-2-(3-methoxy-phenyl)-3-methyl-butan-2-ol und
■ (+)-(2R,3R)-4-Dimethylamino-2-(3-methoxy-phenyl)-3-methyl-butan-2-ol,
vorzugsweise als Hydrochlorid.

In einer bevorzugten Ausführungsform gilt für die erfindungsgemäße Verwendung, daß die **Verbindung A** in **Gruppe d)** ausgewählt ist aus Verbindungen gemäß **Formel II** für die gilt, daß:
X ausgewählt ist aus
   OH, F, Cl, OC(O)CH₃ oder H, vorzugsweise OH, F oder H, insbesondere OH,
   **und/oder**
R¹ ausgewählt ist aus
   C₁₋₄-Alkyl, CF₃, OH, O-C₁₋₄-Alkyl, Cl oder F, vorzugsweise OH, CF₃ oder CH₃,
   **und/oder**
R⁹ bis R¹³, wobei 3 oder 4 der Reste R⁹ bis R¹³ H entsprechen müssen, unabhängig voneinander ausgewählt sind aus
   H, Cl, F, OH, CF₂H, CF₃ oder C₁₋₄-Alkyl, gesättigt und unsubstituiert, verzweigt oder unverzweigt; OR¹⁴ oder SR¹⁴, mit R¹⁴ ausgewählt aus C₁₋₃-Alkyl, gesättigt und unsubstituiert, verzweigt oder unverzweigt;
      vorzugsweise H, Cl, F, OH, CF₂H, CF₃, OCH₃ oder SCH₃
   oder R¹² und R¹¹ einen 3,4-OCH=CH-Ring bilden,
   insbesondere
   wenn R⁹, R¹¹ und R¹³ H entsprechen, einer von R¹⁰ oder R¹² auch H entspricht, während der andere ausgewählt ist aus:
      Cl, F, OH, CF₂H, CF₃, OR¹⁴ oder SR¹⁴, vorzugsweise OH, CF₂H, OR¹⁴ oder SCH₃, insbesondere OH oder OC₁₋₃-Alkyl, vorzugsweise OH oder OCH₃,
      oder,
   wenn R⁹ und R¹³ H entsprechen und R¹¹ OH, OCH₃, Cl oder F, vorzugsweise Cl, entspricht, einer von R¹⁰ oder R¹² auch H entspricht, während der andere OH, OCH₃, Cl oder F, vorzugsweise Cl, entspricht,
      oder,
   wenn R⁹, R¹⁰, R¹² und R¹³ H entsprechen, R¹¹ ausgewählt ist aus CF₃, CF₂H, Cl oder F, vorzugsweise F,
      oder,
   wenn R¹⁰, R¹¹ und R¹² H entsprechen, einer von R⁹ oder R¹³ auch H entspricht, während der andere ausgewählt ist aus OH, OC₂H₅ oder OC₃H₇,
      ganz insbesondere bevorzugt,
   wenn R⁹, R¹¹ und R¹³ H entsprechen, einer von R¹⁰ oder R¹² auch H entspricht, während der andere ausgewählt ist aus:
      Cl, F, OH, SH, CF₂H, CF₃, OR¹⁴ oder SR¹⁴, vorzugsweise OH oder OR¹⁴, insbesondere OH oder OC₁₋₃-Alkyl, vorzugsweise OH oder OCH₃.

Dabei ist es für Verbindungen der **Gruppe d)** besonders bevorzugt, wenn gilt, daß Verbindungen der **Formel II** in Form der Diastereomeren mit der relativen Konfiguration IIa 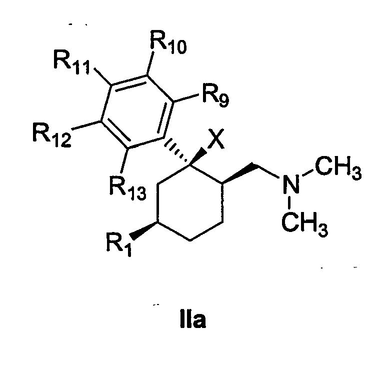 vorliegen, insbesondere in Mischungen mit höherem Anteil dieses Diastereomeren im Vergleich zum anderen Diastereomeren oder als reines Diastereomer verwendet werden,
**und/oder**
daß die Verbindungen der **Formel II** in Form des (+)-Enantiomeren, insbesondere in Mischungen mit höherem Anteil des (+)-Enantiomeren im Vergleich zum (-)-Enantiomeren einer racemischen Verbindung oder als reines (+)-Enantiomer verwendet werden.

Dabei ist es besonders bevorzugt, wenn **Verbindung A** ausgewählt aus folgender Gruppe verwendet wird:
■ (1RS,3RS,6RS)-6-Dimethylaminomethyl-1-(3-methoxy-phenyl)-cyclohexan-1,3-diol,
■ (+)-(1R,3R,6R)-6-Dimethylaminomethyl-1-(3-methoxy-phenyl)-cyclohexan-1,3-diol,
■ (1RS,3RS,6RS)-6-Dimethylaminomethyl-1-(3-hydroxyphenyl)-cyclohexan-1,3-diol,
■ (1RS,3SR,6RS)-6-Dimethylaminomethyl-1-(3-methoxy-phenyl)-cyclohexan-1,3-diol,
■ (+)-(1R,2R,5S)-3-(2-Dimethy)aminomethyl-1-hydroxy-5-methyl-cyclohexyl)-phenol oder
■ (1RS,2RS,5RS)-3-(2-Dimethylaminomethyl-1-hydroxy-5-trifluoromethyl-cyclohexyl)-phenol,
vorzugsweise als Hydrochlorid.

In einer bevorzugten Ausführungsform gilt für die erfindungsgemäße Verwendung, daß die **Verbindung A** in **Gruppe e)** ausgewählt ist aus Verbindungen gemäß **Formel III** für die gilt, daß:
X ausgewählt ist aus
   OH, F, Cl, OC(O)CH₃ oder H, vorzugsweise OH, F oder H, insbesondere F oder H.
   **und/oder**
R⁹ bis R¹³, wobei 3 oder 4 der Reste R⁹ bis R¹³ H entsprechen müssen, unabhängig voneinander ausgewählt sind aus
   H, Cl, F, OH, CF₂H, CF₃ oder C₁-₄-Alkyl, gesättigt und unsubstituiert, verzweigt oder unverzweigt; OR¹⁴ oder SR¹⁴, mit R¹⁴ ausgewählt aus C₁₋₃-Alkyl, gesättigt und unsubstituiert, verzweigt oder unverzweigt;
      vorzugsweise H, Cl, F, OH, CF₂H, CF₃, OCH₃ oder SCH₃
   oder R¹² und R¹¹ einen 3,4-OCH=CH-Ring bilden
insbesondere dadurch gekennzeichnet, daß,
   wenn R⁹, R¹¹ und R¹³ H entsprechen, einer von R¹⁰ oder R¹² auch H entspricht, während der andere ausgewählt ist aus:
      Cl, F, OH, CF₂H, CF₃, OR¹⁴ oder SR¹⁴, vorzugsweise OH, CF₂H, OR¹⁴ oder SCH₃, insbesondere OH oder OC₁₋₃-Alkyl, vorzugsweise OH oder OCH₃,
      oder,
   wenn R⁹ und R¹³ H entsprechen und R¹¹ OH, OCH₃, Cl oder F, vorzugsweise Cl, entspricht, einer von R¹⁰ oder R¹² auch H entspricht, während der andere OH, OCH₃, Cl oder F, vorzugsweise Cl, entspricht,
      oder,
   wenn R⁹, R¹⁰, R¹² und R¹³ H entsprechen, R¹¹ ausgewählt ist aus CF₃, CF₂H, Cl oder F, vorzugsweise F,
      oder,
   wenn R¹⁰, R¹¹ und R¹² H entsprechen, einer von R⁹ oder R¹³ auch H entspricht, während der andere ausgewählt ist aus OH, OC₂H₅ oder OC₃H₇,
      ganz insbesondere bevorzugt,
   wenn R⁹, R¹¹ und R¹³ H entsprechen, einer von R¹⁰ oder R¹² auch H entspricht, während der andere ausgewählt ist aus:
      Cl, F, OH, SH, CF₂H, CF₃, OR¹⁴ oder SR¹⁴, vorzugsweise OH oder OR¹⁴, insbesondere OH oder OC₁₋₃-Alkyl, vorzugsweise OH oder OCH₃.

Dabei ist es für Verbindungen der **Gruppe e)** besonders bevorzugt, wenn gilt, daß Verbindungen der **Formel III** in Form ihrer Diastereomeren mit der relativen Konfiguration IIIa 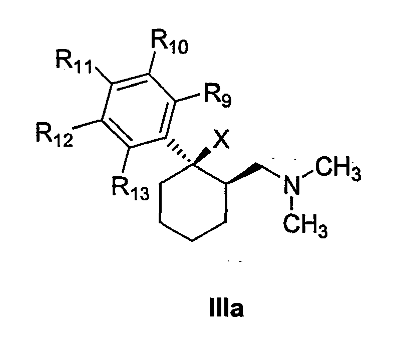 vorliegen, insbesondere in Mischungen mit höherem Anteil dieses Diastereomeren im Vergleich zum anderen Diastereomeren oder als reines Diastereomer verwendet werden
**und/oder**
, daß die Verbindungen der **Formel III** in Form des (+)-Enantiomeren, insbesondere in Mischungen mit höherem Anteil des (+)-Enantiomeren im Vergleich zum (-)-Enantiomeren einer racemischen Verbindung oder als reines (+)-Enantiomer verwendet werden.

Dabei ist es besonders bevorzugt, wenn **Verbindung A** ausgewählt aus folgender Gruppe verwendet wird:
■ (+)-(1R,2R)-3-(2-Dimethylaminomethyl-1-fluoro-cyclohexyl)-phenol,
■ (+)-(1S,2S)-3-(2-Dimethylaminomethyl-cyclohexyl)-phenol oder
■ (1S,2S)-3-(2-Dimethylaminomethyl-cyclohexyl)-phenol oder
■ (-)-(1R,2R)-3-(2-Dimethylaminomethyl-cyclohexyl)-phenol,
■ (1R,2R)-3-(2-Dimethylaminomethyl-cyclohexyl)-phenol,
■ (-)-(1R,2R)-[2-(3-Methoxy-phenyl)-cyclohexylmethyl]-dimethylamin,
■ (1R,2R)-[2-(3-Methoxy-phenyl)-cyclohexylmethyl]-dimethylamin,
vorzugsweise als Hydrochlorid.

Für eine besonders bevorzugte Verwendung gilt, daß die **Verbindung B** ausgewählt ist aus:
Darifenacin, Duloxetin, Oxybutinin oder Tolterodin,
vorzugsweise ausgewählt ist aus
Duloxetin, Oxybutinin oder Tolterodin,
vorzugsweise ausgewählt ist aus
Oxybutinin oder Tolterodin.

Auch wenn die erfindungsgemässen Verwendungen lediglich geringe Nebenwirkungen zeigen, kann es beispielsweise zur Vermeidung von bestimmten Formen der Abhängigkeit auch von Vorteil sein, neben der Kombination der **Verbindungen A** und **B** auch Morphinantagonisten, insbesondere Naloxon, Naltrexon und/oder Levallorphan, zu verwenden.

Ein weiterer Gegenstand der Erfindung ist eine Wirkstoffkombination aus wenigstens einer der **Verbindungen A** und wenigstens einer der **Verbindungen B,** mit **Verbindung A** ausgewählt aus:
**Gruppe a)** enthaltend:
   Tramadol, O-Demethyltramadol oder O-desmethyl-N-monodesmethyl-tramadol gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in Form ihrer Säuren oder ihrer Basen oder in Form ihrer Salze, insbesondere der physiologisch verträglichen Salze, oder in Form ihrer Solvate, insbesondere der Hydrate;
**Gruppe b)** enthaltend:
   - Codein
   - Dextropropxyphen
   - Dihydrocodein
   - Meptazinol
   - Nalbuphin
   - Tilidin
   - Buprenorphin
   gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in Form ihrer Säuren oder ihrer Basen oder in Form ihrer Salze, insbesondere der physiologisch verträglichen Salze, oder in Form ihrer Solvate, insbesondere der Hydrate;
**Gruppe c)** enthaltend:
   1-Phenyl-3-dimethyfamino-propanverbindungen gemäß allgemeiner **Formel I** 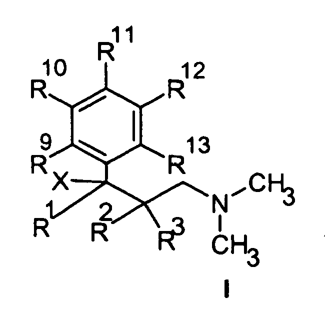
   worin
   X ausgewählt ist aus OH, F, Cl, H oder OC(O)R⁷ mit R⁷ ausgewählt aus C₁₋₃-Alkyl, verzweigt oder unverzweigt, gesättigt oder ungesättigt, unsubstituiert oder ein- oder mehrfach substituiert,
   R¹ ausgewählt ist aus C₁₋₄-Alkyl, verzweigt oder unverzweigt, gesättigt oder ungesättigt, unsubstituiert oder ein- oder mehrfach substituiert,
   R² und R³ jeweils unabhängig voneinander ausgewählt sind aus H oder C₁₋₄-Alkyl, verzweigt oder unverzweigt, gesättigt oder ungesättigt, unsubstituiert oder ein- oder mehrfach substituiert,
      oder
   R² und R³ zusammen einen gesättigten C₄₋₇-Cycloalkylrest bilden, unsubstituiert oder ein- oder mehrfach substituiert,
   R9 bis R¹³ jeweils unabhängig voneinander ausgewählt sind aus H, F, Cl, Br, I, CH₂F, CHF₂, CF₃, OH, SH, OR¹⁴, OCF₃, SR¹⁴, NR¹⁷R¹⁸, SOCH₃, SOCF₃; SO₂CH₃, SO₂CF₃, CN, COOR¹⁴, NO₂, CONR¹⁷R¹⁸; C₁₋₆-Alkyl, verzweigt oder unverzweigt, gesättigt oder ungesättigt, unsubstituiert oder ein-oder mehrfach substituiert; Phenyl, unsubstituiert oder ein- oder mehrfach substituiert;
      mit R¹⁴ ausgewählt aus C₁₋₆-Alkyl; Pyridyl, Thienyl, Thiazolyl, Phenyl, Benzyl oder Phenethyl, jeweils unsubstituiert oder ein- oder mehrfach substituiert; PO(O-C₁₋₄-Alkyl)₂, CO(OC₁₋₅-Alkyl), CONH-C₆H₄-(C₁₋₃-Alkyl), CO(C₁₋₅-Alkyl), CO-CHR¹⁷-NHR¹⁸, CO-C₆H₄-R¹⁵, mit R¹⁵ ortho-OCOC₁₋₃-Alkyl oder meta- oder para-CH₂N(R¹⁶)₂ mit R¹⁶ C₁₋₄-Alkyl oder 4-Morpholino, wobei in den Resten R¹⁴, R¹⁵ und R¹⁶ die Alkylgruppen verzweigt oder unverzweigt, gesättigt oder ungesättigt, unsubstituiert oder ein- oder mehrfach substituiert sein können;
      mit R¹⁷ und R18 jeweils unabhängig voneinander ausgewählt aus H; C₁₋₆-Alkyl, verzweigt oder unverzweigt, gesättigt oder ungesättigt, unsubstituiert oder ein- oder mehrfach substituiert; Phenyl, Benzyl oder Phenethyl, jeweils unsubstituiert oder ein- oder mehrfach substituiert,
      oder
   R⁹ und R¹⁰ oder R¹⁰ und R¹¹ zusammen einen OCH₂O-, OCH₂CH₂O-, OCH=CH-, CH=CHO-, CH=C(CH₃)O-, OC(CH₃)=CH-, (CH₂)₄- oder OCH=CHO-Ring bilden,
   gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form ihrer Säuren oder ihrer Basen oder in Form ihrer Salze, insbesondere der physiologisch verträglichen Salze, oder in Form ihrer Solvate, insbesondere der Hydrate;
**Gruppe d)** enthaltend:
   substituierte 6-Dimethylaminomethyl-1-phenyl-cyclohexanverbindungen gemäß allgemeiner **Formel II** 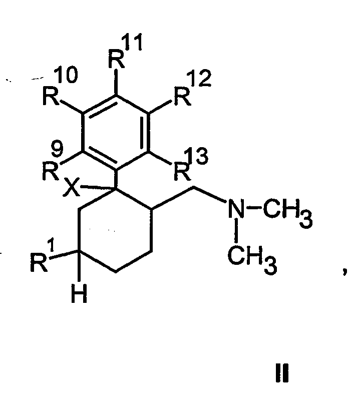
   worin
   X ausgewählt ist aus OH, F, Cl, H oder OC(O)R⁷ mit R⁷ ausgewählt aus C₁₋₃-Alkyl, verzweigt oder unverzweigt, gesättigt oder ungesättigt, unsubstituiert oder ein- oder mehrfach substituiert,
   R¹ ausgewählt ist aus C₁₋₄-Alkyl, Benzyl, CF₃, OH, OCH₂-C₆H₅, O-C₁₋₄-Alkyl, Cl oder F und
   R⁹ bis R¹³ jeweils unabhängig voneinander ausgewählt sind aus H, F, Cl, Br, I, CH₂F, CHF₂, CF₃, OH, SH, OR¹⁴, OCF₃, SR¹⁴, NR¹⁷R¹⁸, SOCH₃, SOCF₃; SO₂CH₃, SO₂CF₃, CN, COOR¹⁴, NO₂, CONR¹⁷R¹⁸; C₁₋₆-Alkyl, verzweigt oder unverzweigt, gesättigt oder ungesättigt, unsubstituiert oder ein- oder mehrfach substituiert; Phenyl, unsubstituiert oder ein- oder mehrfach substituiert;
      mit R¹⁴ ausgewählt aus C₁₋₆-Alkyl; Pyridyl, Thienyl, Thiazolyl, Phenyl, Benzyl oder Phenethyl, jeweils unsubstituiert oder ein- oder mehrfach substituiert; PO(O-C₁₋₄-Alkyl)₂, CO(OC₁₋₅-Alkyl), CONH-C₆H₄-(C₁₋₃-Alkyl), CO(C₁₋₅-Alkyl), CO-CHR¹⁷-NHR¹⁸, CO-C₆H₄-R¹⁵, mit R¹⁵ ortho-OCOC₁₋₃-Alkyl oder meta- oder para-CH₂N(R¹⁶)₂ mit R¹⁶ C₁₋₄-Alkyl oder 4-Morpholino, wobei in den Resten R¹⁴, R¹⁵ und R¹⁶ die Alkylgruppen verzweigt oder unverzweigt, gesättigt oder ungesättigt, unsubstituiert oder ein- oder mehrfach substituiert sein können;
      mit R¹⁷ und R18 jeweils unabhängig voneinander ausgewählt aus H; C₁₋₆-Alkyl, verzweigt oder unverzweigt, gesättigt oder ungesättigt, unsubstituiert oder ein- oder mehrfach substituiert; Phenyl, Benzyl oder Phenethyl, jeweils unsubstituiert oder ein- oder mehrfach substituiert,
      oder
   R⁹ und R¹⁰ oder R¹⁰ und R¹¹ zusammen einen OCH₂O-, OCH₂CH₂O-, OCH=CH-, CH=CHO-, CH=C(CH3)O-, OC(CH3)=CH-, (CH₂)₄- oder OCH=CHO-Ring bilden,
   gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form ihrer Säuren oder ihrer Basen oder in Form ihrer Salze, insbesondere der physiologisch verträglichen Salze, oder in Form ihrer Solvate, insbesondere der Hydrate;
   **und/oder**
**Gruppe e)** enthaltend:
   6-Dimethylaminomethyl-1-phenyl-cyclohexanverbindungen gemäß allgemeiner **Formel III** 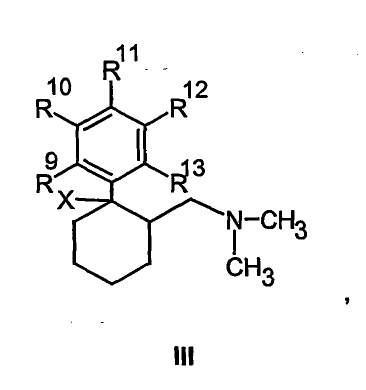
   worin
   X ausgewählt ist aus OH, F, Cl, H oder OC(O)R⁷ mit R⁷ ausgewählt aus C₁₋₃-Alkyl, verzweigt oder unverzweigt, gesättigt oder ungesättigt, unsubstituiert oder ein- oder mehrfach substituiert, und
   R⁹ bis R¹³ jeweils unabhängig voneinander ausgewählt sind aus H, F, Cl, Br, I, CH₂F, CHF₂, CF₃, OH, SH, OR¹⁴, OCF₃, SR¹⁴, NR¹⁷R¹⁸, SOCH₃, SOCF_{3;} SO₂CH₃, SO₂CF₃, CN, COOR¹⁴, NO₂, CONR¹⁷R¹⁸; C₁₋₆-Alkyl, verzweigt oder unverzweigt, gesättigt oder ungesättigt, unsubstituiert oder ein-oder mehrfach substituiert; Phenyl, unsubstituiert oder ein- oder mehrfach substituiert;
      mit R¹⁴ ausgewählt aus C₁₋₆-Alkyl; Pyridyl, Thienyl, Thiazolyl, Phenyl, Benzyl oder Phenethyl, jeweils unsubstituiert oder ein- oder mehrfach substituiert; PO(O-C₁₋₄-Alkyl)₂, CO(OC₁₋₅-Alkyl), CONH-C₆H₄-(C₁₋₃-Alkyl), CO(C₁₋₅-Alkyl), CO-CHR¹⁷-NHR¹⁸, CO-C₆H₄-R¹⁵, mit R¹⁵ ortho-OCOC₁₋₃-Alkyl oder meta- oder para-CH₂N(R¹⁶)₂ mit R¹⁶ C₁₋₄-Alkyl oder 4-Morpholino, wobei in den Resten R¹⁴, R¹⁵ und R¹⁶ die Alkylgruppen verzweigt oder unverzweigt, gesättigt oder ungesättigt, unsubstituiert oder ein- oder mehrfach substituiert sein können;
      mit R¹⁷ und R18 jeweils unabhängig voneinander ausgewählt aus H; C₁₋₆-Alkyl, verzweigt oder unverzweigt, gesättigt oder ungesättigt, unsubstituiert oder ein- oder mehrfach substituiert; Phenyl, Benzyl oder Phenethyl, jeweils unsubstituiert oder ein- oder mehrfach substituiert,
      oder
   R⁹ und R¹⁰ oder R¹⁰ und R¹¹ zusammen einen OCH₂O-, OCH₂CH₂O-, OCH=CH-, CH=CHO-, CH=C(CH3)O-, OC(CH3)=CH-, (CH₂)₄- oder OCH=CHO-Ring bilden,
   mit der Maßgabe, daß, wenn R⁹, R¹¹ und R¹³ H entsprechen, und einer von R¹⁰ oder R¹² H und der andere OCH₃ entspricht, X nicht OH sein darf,
   gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form ihrer Säuren oder ihrer Basen oder in Form ihrer Salze, insbesondere der physiologisch verträglichen Salze, oder in Form ihrer Solvate, insbesondere der Hydrate;
   **und** mit wenigstens einer der **Verbindungen B**, ausgewählt aus:
   den Antimuskarinika: Atropin, Oxybutinin, Propiverin, Propanthelin, Emepronium, Trospium, Tolterodin, Darifenacin und α,α-Diphenylessigsäure-4-(N-methylpiperidyl)-ester, sowie Duloxetin, lmipramin und Desmopressin,
   gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in Form ihrer Säuren oder ihrer Basen oder in Form ihrer Salze, insbesondere der physiologisch verträglichen Salze, oder in Form ihrer Solvate, insbesondere der Hydrate.

Für die Wirkstoffkombination ist es besonders bevorzugt, wenn gilt, daß die **Verbindung A** in **Gruppe a)** ausgewählt ist aus:
Tramadol, (+)-Tramadol, (+)-O-Demethyltramadol oder (+)-O-desmethyl-N-mono-desmethyl-tramadol, vorzugsweise Tramadol oder (+)-Tramadol, insbesondere (+)-Tramadol.

Für die Wirkstoffkombination ist es besonders bevorzugt, wenn gilt, daß die **Verbindung A** in **Gruppe c)** ausgewählt ist aus Verbindungen gemäß **Formel I** für die gilt, daß:
X ausgewählt ist aus
   OH, F, Cl, OC(O)CH₃ oder H, vorzugsweise OH, F, OC(O)CH₃ oder H,
   **und/oder**
R¹ ausgewählt ist aus
   C₁₋₄-Alkyl, gesättigt und unsubstituiert, verzweigt oder unverzweigt; vorzugsweise CH₃, C₂H₅, C₄Hg oder t-Butyl, insbesondere CH₃ oder C₂H₅,
   **und/oder**
   R² und R³ unabhängig voneinander ausgewählt sind aus
      H, C₁₋₄-Alkyl, gesättigt und unsubstituiert, verzweigt oder unverzweigt; vorzugsweise H, CH₃, C₂H₅, i-Propyl oder t-Butyl, insbesondere H oder CH₃, vorzugsweise R³ = H,
         oder
      R² und R³ zusammen einen C₅₋₆-Cycloalkylrest bilden, gesättigt oder ungesättigt, unsubstituiert oder ein- oder mehrfach substituiert, vorzugsweise gesättigt und unsubstituiert, insbesondere Cyclohexyl.
      **und/oder**
   R⁹ bis R¹³, wobei 3 oder 4 der Reste R⁹ bis R¹³ H entsprechen müssen, unabhängig voneinander ausgewählt sind aus
      H, Cl, F, OH, CF₂H, CF₃ oder C₁₋₄-Alkyl, gesättigt und unsubstituiert, verzweigt oder unverzweigt; OR¹⁴ oder SR¹⁴, mit R¹⁴ ausgewählt aus C₁₋₃-Alkyl, gesättigt und unsubstituiert, verzweigt oder unverzweigt;
         vorzugsweise H, Cl, F, OH, CF₂H, CF₃, OCH₃ oder SCH₃
      oder R¹² und R¹¹ einen 3,4-OCH=CH-Ring bilden
      insbesondere
      wenn R⁹, R¹¹ und R¹³ H entsprechen, einer von R¹⁰ oder R¹² auch H entspricht, während der andere ausgewählt ist aus:
         Cl, F, OH, CF₂H, CF₃, OR¹⁴ oder SR¹⁴, vorzugsweise OH, CF₂H, OCH₃ oder SCH₃
         oder,
      wenn R⁹ und R¹³ H entsprechen und R¹¹ OH, OCH₃, Cl oder F, vorzugsweise Cl, entspricht, einer von R¹⁰ oder R¹² auch H entspricht, während der andere OH, OCH₃, Cl oder F, vorzugsweise Cl, entspricht,
         oder,
      wenn R⁹, R¹⁰, R¹² und R¹³ H entsprechen, R¹¹ ausgewählt ist aus CF₃, CF₂H, Cl oder F, vorzugsweise F,
         oder,
      wenn R¹⁰, R¹¹ und R¹² H entsprechen, einer von R⁹ oder R¹³ auch H entspricht, während der andere ausgewählt ist aus OH, OC₂H₅ oder OC₃H₇.

Dabei ist es für Verbindungen der **Gruppe c)** besonders bevorzugt, wenn gilt, daß die Verbindungen der **Formel I** mit R³ = H in Form der Diastereomeren mit der relativen Konfiguration la 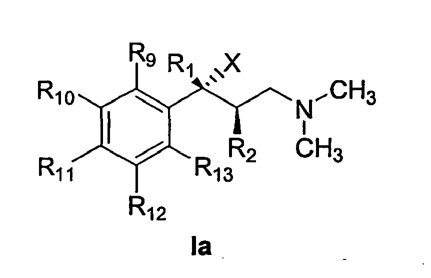 vorliegen, insbesondere in Mischungen mit höherem Anteil dieses Diastereomeren im Vergleich zum anderen Diastereomeren oder als reines Diastereomer
**und/oder**
daß die Verbindungen der **Formel I** in Form des (+)-Enantiomeren, insbesondere in Mischungen mit höherem Anteil des (+)-Enantiomeren im Vergleich zum (-)-Enantiomeren einer racemischen Verbindung oder als reines (+)-Enantiomer vorliegen.

Dabei ist es besonders bevorzugt, wenn **Verbindung A** ausgewählt ist aus folgender Gruppe:
■ (2RS,3RS)-1-Dimethylamino-3-(3-methoxy-phenyl)-2-methyl-pentan-3-ol,
■ (2R,3R)-1-Dimethylamino-3-(3-methoxy-phenyl)-2-methyl-pentan-3-ol,
■ (+)-(2R,3R)-1-Dimethylamino-3-(3-methoxy-phenyl)-2-methyl-pentan-3-ol,
■ (2RS,3RS)-3-(3,4-Dichlorophenyl)-1-dimethylamino-2-methyl-pentan-3-ol,
■ (2RS,3RS)-3-(3-Difluoromethyl-phenyl)-1-dimethylamino-2-methyl-pentan-3-ol,
■ (2RS,3RS)-1-Dimethylamino-2-methyl-3-(3-methylsulfanylphenyl)-pentan-3-ol,
■ (3RS)-1-Dimethylamino-3-(3-methoxy-phenyl)-4,4-dimethyl-pentan-3-ol,
*■* (2RS,3RS)-3-(3-Dimethylamino-1-ethyl-1-hydroxy-2-methyl-propyl)-phenol,
■ (1RS,2RS)-3-(3-Dimethylamino-1-hydroxy-1,2-dimethyl-propyl)-phenol,
■ (+)-(1R,2R)-3-(3-Dimethylamino-1-hydroxy-1,2-dimethylpropyl)-phenol,
■ (+)-(1R,2R)-3-(3-Dimethylamino-1-hydroxy-1,2-dimethyl-propyl)-phenol,
■ (1R,2R)-3-(3-Dimethylamino-1-ethyl-2-methyl-propyl)-phenol,
■ (-)-(1R,2R)-3-(3-Dimethylamino-1-ethyl-2-methyl-propyl)-phenol,
■ (1S,2S)-3-(3-Dimethylamino-1-ethyl-2-methyl-propyl)-phenol,
■ (+)-(1S,2S)-3-(3-Dimethylamino-1-ethyl-2-methyl-propyl)-phenol,
■ (+)-(1R,2R)-Essigsäure-3-dimethylamino-1-ethyl-1-(3-methoxy-phenyl)-2-methyl-propylester,
■ (1RS)-1-(1-Dimethylaminomethyl-cyclohexyl)-1-(3-methoxy-phenyl)-propan-1-ol,
■ (2RS,3RS)-3-(4-Chlorophenyl)-1-dimethylamino-2-methyl-pentan-3-ol,
■ (+)-(2R,3R)-3-(3-Dimethylamino-1-ethyl-1-hydroxy-2-methylpropyl)-phenol,
■ (2RS,3RS)-4-Dimethylamino-2-(3-methoxy-phenyl)-3-methyl-butan-2-ol und
■ (+)-(2R,3R)-4-Dimethylamino-2-(3-methoxy-phenyl)-3-methyl-butan-2-ol,
vorzugsweise als Hydrochlorid._

Für die Wirkstoffkombination ist es besonders bevorzugt, wenn gilt, daß die **Verbindung A i**n **Gruppe d)** ausgewählt ist aus Verbindungen gemäß **Formel II** für die gilt, daß:
X ausgewählt ist aus
   OH, F, Cl, OC(O)CH₃ oder H, vorzugsweise OH, F oder H, insbesondere OH,
   **und/oder**
R¹ ausgewählt ist aus
   C₁₋₄-Alkyl, CF₃, OH, O-C₁₋₄-Alkyl, Cl oder F, vorzugsweise OH, CF₃ oder CH₃,
   **und/oder**
R⁹ bis R¹³, wobei 3 oder 4 der Reste R⁹ bis R¹³ H entsprechen müssen, unabhängig voneinander ausgewählt sind aus
   H, Cl, F, OH, CF₂H, CF₃ oder C₁₋₄-Alkyl, gesättigt und unsubstituiert, verzweigt oder unverzweigt; OR¹⁴ oder SR¹⁴, mit R¹⁴ ausgewählt aus C₁₋₃-Alkyl, gesättigt und unsubstituiert, verzweigt oder unverzweigt;
      vorzugsweise H, Cl, F, OH, CF₂H, CF₃, OCH₃ oder SCH₃
   oder R¹² und R¹¹ einen 3,4-OCH=CH-Ring bilden,
   insbesondere
   wenn R⁹, R¹¹ und R¹³ H entsprechen, einer von R¹⁰ oder R¹² auch H entspricht, während der andere ausgewählt ist aus:
      Cl, F, OH, CF₂H, CF₃, OR¹⁴ oder SR¹⁴, vorzugsweise OH, CF₂H, OR¹⁴ oder SCH₃, insbesondere OH oder OC₁₋₃-Alkyl, vorzugsweise OH oder OCH₃,
      oder,
   wenn R⁹ und R¹³ H entsprechen und R¹¹ OH, OCH₃, Cl oder F, vorzugsweise Cl, entspricht, einer von R¹⁰ oder R¹² auch H entspricht, während der andere OH, OCH₃, Cl oder F, vorzugsweise Cl, entspricht,
      oder,
   wenn R⁹, R¹⁰, R¹² und R¹³ H entsprechen, R¹¹ ausgewählt ist aus CF₃, CF₂H, Cl oder F, vorzugsweise F,
      oder,
   wenn R¹⁰, R¹¹ und R¹² H entsprechen, einer von R⁹ oder R¹³ auch H entspricht, während der andere ausgewählt ist aus OH, OC₂H₅ oder OC₃H₇,
      ganz insbesondere bevorzugt,
   wenn R⁹, R¹¹ und R¹³ H entsprechen, einer von R¹⁰ oder R¹² auch H entspricht, während der andere ausgewählt ist aus:
      Cl, F, OH, SH, CF₂H, CF₃, OR¹⁴ oder SR¹⁴, vorzugsweise OH oder OR¹⁴, insbesondere OH oder OC₁₋₃-Alkyl, vorzugsweise OH oder OCH₃.

Dabei ist es für Verbindungen der **Gruppe d)** besonders bevorzugt, wenn gilt, daß die Verbindungen der **Formel II** in Form der Diastereomeren mit der relativen Konfiguration lla 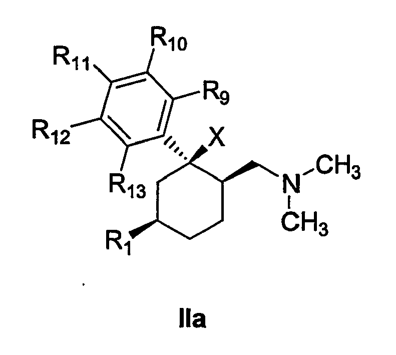 vorliegen, insbesondere in Mischungen mit höherem Anteil dieses Diastereomeren im Vergleich zum anderen Diastereomeren oder als reines Diastereomer,
**und/oder**
daß die Verbindungen der **Formel II** in Form des (+)-Enantiomeren, insbesondere in Mischungen mit höherem Anteil des (+)-Enantiomeren im Vergleich zum (-)-Enantiomeren einer racemischen Verbindung oder als reines (+)-Enantiomer vorliegen.

Dabei ist es besonders bevorzugt, wenn **Verbindung A** ausgewählt ist aus folgender Gruppe:
■ (1RS,3RS,6RS)-6-Dimethylaminomethyl-1-(3-methoxyphenyl)-cyclohexan-1,3-diol,
■ (+)-(1R,3R,6R)-6-Dimethylaminomethyl-1-(3-methoxyphenyl)-cyclohexan-1,3-diol,
■ (1RS,3RS,6RS)-6-Dimethylaminomethyl-1-(3-hydroxyphenyl)-cyclohexan-1,3-diol,
■ (1RS,3SR,6RS)-6-Dimethylaminomethyl-1-(3-methoxyphenyl)-cyclohexan-1,3-diol,
■ (+)-(1R,2R,5S)-3-(2-Dimethylaminomethyl-1-hydroxy-5-methyl-cyclohexyl)-phenol oder
■ (1RS,2RS,5RS)-3-(2-Dimethylaminomethyl-1-hydroxy-5-trifluoromethyl-cyclohexyl)-phenol,
vorzugsweise als Hydrochlorid.

Für die Wirkstoffkombination ist es besonders bevorzugt, wenn gilt, daß die **Verbindung A** in **Gruppe e)** ausgewählt ist aus Verbindungen gemäß **Formel III** für die gilt, daß:
X ausgewählt ist aus
   OH, F, Cl, OC(O)CH₃ oder H, vorzugsweise OH, F oder H, insbesondere F oder H.
   **und/oder**
R⁹ bis R¹³, wobei 3 oder 4 der Reste R⁹ bis R¹³ H entsprechen müssen, unabhängig voneinander ausgewählt sind aus H, Cl, F, OH, CF₂H, CF₃ oder C₁₋₄-Alkyl, gesättigt und unsubstituiert, verzweigt oder unverzweigt; OR¹⁴ oder SR¹⁴, mit R¹⁴ ausgewählt aus C₁₋₃-Alkyl, gesättigt und unsubstituiert, verzweigt oder unverzweigt;
   vorzugsweise H, Cl, F, OH, CF₂H, CF₃, OCH₃ oder SCH₃
oder R¹² und R¹¹ einen 3,4-OCH=CH-Ring bilden
insbesondere dadurch gekennzeichnet, daß,
wenn R⁹, R¹¹ und R¹³ H entsprechen, einer von R¹⁰ oder R¹² auch H entspricht, während der andere ausgewählt ist aus:
   Cl, F, OH, CF₂H, CF₃, OR¹⁴ oder SR¹⁴, vorzugsweise OH, CF₂H, OR¹⁴ oder SCH₃, insbesondere OH oder OC₁₋₃-Alkyl, vorzugsweise OH oder OCH₃,
   oder,
wenn R⁹ und R¹³ H entsprechen und R¹¹ OH, OCH₃, Cl oder F, vorzugsweise Cl, entspricht, einer von R¹⁰ oder R¹² auch H entspricht, während der andere OH, OCH₃, Cl oder F, vorzugsweise Cl, entspricht,
   oder,
wenn R⁹, R¹⁰, R¹² und R¹³ H entsprechen, R¹¹ ausgewählt ist aus CF₃, CF₂H, Cl oder F, vorzugsweise F,
   oder,
wenn R¹⁰, R¹¹ und R¹² H entsprechen, einer von R⁹ oder R¹³ auch H entspricht, während der andere ausgewählt ist aus OH, OC₂H₅ oder OC₃H₇,
   ganz insbesondere bevorzugt,
wenn R⁹, R¹¹ und R¹³ H entsprechen, einer von R¹⁰ oder R¹² auch H entspricht, während der andere ausgewählt ist aus:
   Cl, F, OH, SH, CF₂H, CF₃, OR¹⁴ oder SR¹⁴, vorzugsweise OH oder OR¹⁴, insbesondere OH oder OC₁₋₃-Alkyl, vorzugsweise OH oder OCH₃.

Dabei ist es für Verbindungen der **Gruppe e)** besonders bevorzugt, wenn gilt, daß die Verbindungen der **Formel III** in Form ihrer Diastereomeren mit der relativen Konfiguration IIIa 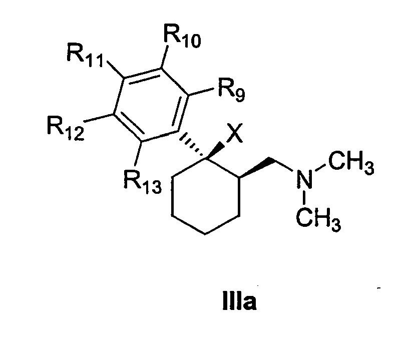 vorliegen, insbesondere in Mischungen mit höherem Anteil dieses Diastereomeren im Vergleich zum anderen Diastereomeren oder als reines Diastereomer
**und/oder**
, daß die Verbindungen der **Formel III** in Form des (+)-Enantiomeren, insbesondere in Mischungen mit höherem Anteil des (+)-Enantiomeren im Vergleich zum (-)-Enantiomeren einer racemischen Verbindung oder als reines (+)-Enantiomer vorliegen.

Dabei ist es besonders bevorzugt, wenn **Verbindung A** ausgewählt ist aus -folgender Gruppe:
■ (+)-(1R,2R)-3-(2-Dimethylaminomethyl-1-fluoro-cyclohexyl)-phenol,
■ (+)-(1S,2S)-3-(2-Dimethylaminomethyl-cyclohexyl)-phenol oder
■ (1S,2S)-3-(2-Dimethylaminomethyl-cyclohexyl)-phenol oder
■ (-)-(1R,2R)-3-(2-Dimethylaminomethyl-cyclohexyl)-phenol,
■ (1R,2R)-3-(2-Dimethylaminomethyl-cyclohexyl)-phenol,
■ (-)-(1R,2R)-[2-(3-Methoxy-phenyl)-cyclohexylmethyl]-dimethylamin,
■ (1R,2R)-[2-(3-Methoxy-phenyl)-cyclohexylmethyl]-dimethylamin,
vorzugsweise als Hydrochlorid.

In einer generell besonders bevorzugten Form der erfindungsgemäßen Wirkstoffkombination ist die **Verbindung B** ausgewählt aus:
Darifenacin, Duloxetin, Oxybutinin oder Tolterodin,
vorzugsweise ausgewählt ist aus
Duloxetin, Oxybutinin oder Tolterodin,
vorzugsweise ausgewählt ist aus
Oxybutinin oder Tolterodin.

Ein weiterer Gegenstand der Erfindung ist ein Arzneimittel, vorzugsweise zur Behandlung von vermehrtem Harndrang bzw. Harninkontinenz, enthaltend eine erfindungsgemäße Wirkstoffkombination sowie gegebenenfalls geeignete Zusatz- und/oder Hilfsstoffe.

Geeignete Zusatz- und/oder Hilfsstoffe im Sinne dieser Erfindung sind alle dem Fachmann aus dem Stand der Technik bekannten Stoffe zur Erreichung galenischer Formulierungen. Die Auswahl dieser Hilfsstoffe sowie die einzusetzenden Mengen derselben hängen davon ab, ob das Arzneimittel oral, intravenös, intraperitoneal, intradermal, intramuskulär, intranasal, buccal oder lokal appliziert werden soll. Für die orale Applikation eignen sich Zubereitungen in Form von Tabletten, Kautabletten, Dragees, Kapseln, Granulaten, Tropfen, Säften oder Sirupen, für die parenterale, topische und inhalative Applikation Lösungen, Suspensionen, leicht rekonstituierbare Trockenzubereitungen sowie Sprays. Eine weitere Möglichkeit sind Suppositorien für die Anwendung im Rektum. Die Anwendung in einem Depot in gelöster Form, einer Trägerfolie oder einem Pflaster, gegebenenfalls unter Zusatz von die Hautpenetration fördernden Mitteln, sind Beispiele für geeignete perkutane Applikationsformen. Beispiele für Hilfs- und Zusatzmitteln für die oralen Applikationsformen sind Sprengmittel, Gleitmittel, Binder, Füllmittel, Formtrennmittel, gegebenenfalls Lösungsmittel, Geschmacksstoffe, Zucker, insbesondere Trägermittel, Verdünnungsmittel, Farbstoffe, Antioxidantien etc. Für Suppositorien können u.a. Wachse bzw. Fettsäureester und für parenterale Applikationsmittel Trägerstoffe, Konservierungsmittel, Suspensionshilfsmittel etc. verwendet werden. Die an Patienten zu verabreichenden Wirkstoffmengen variieren in Abhängigkeit vom Gewicht des Patienten, von der Applikationsart und dem Schweregrad der Erkrankung. Aus oral, rektal oder perkutan anwendbaren Zubereitungsformen können die erfindungsgemäßen Verbindungen verzögert freigesetzt werden. Bei der erfindungsgemäßen Indikation sind entsprechende Retard-Formulierungen, insbesondere in Form eines "Once-daily"-Präparats, das nur einmal am Tag eingenommen werden muß, besonders bevorzugt.

Weiter bevorzugt sind Arzneimittel, die wenigstens 0,05 bis 90,0 % des Wirkstoffes enthalten, insbesondere niedrige wirksame Dosierungen, um Neben- oder analgetische Wirkungen zu vermeiden. Üblicherweise werden 0,1 bis 5000 mg/kg, insbesondere 1 bis 500 mg/kg, vorzugsweise 2 bis 250 mg/kg Körpergewicht wenigstens einer Verbindung der Formel I appliziert. Ebenso bevorzugt und üblich ist aber auch die Applikation von 0,01 - 5 mg/kg, vorzugsweise 0,03 bis 2 mg/kg, insbesondere 0,05 bis 1 mg/kg Körpergewicht.

Hilfsstoffe können beispielsweise sein: Wasser, Ethanol, 2-Propanol, Glycerin, Ethylenglycol, Propylenglycol, Polyethylenglycol, Polypropylenglycol, Glucose, Fructose, Lactose, Saccharose, Dextrose, Melasse, Stärke, modifizierte Stärke, Gelatine, Sorbitol, Inositol, Mannitol, mikrokristalline Cellulose, Methylcellulose, Carboxymethylcellulose, Celluloseacetat, Schellack, Cetylalkohol, Polyvinylpyrrolidon, Paraffine, Wachse, natürliche und synthetische Gummis, Akaziengummi, Alginate, Dextran, gesättigte und ungesättigte Fettsäuren, Stearinsäure, Magnesiumstearat, Zinkstearat, Glycerylstearat, Natriumlaurylsulfat, genießbare Öle, Sesamöl, Kokusnußöl, Erdnußöl, Sojabohnenöl, Lecithin, Natriumlactat, Polyoxyethylen- und -propylen-fettsäureester, Sorbitanfettsäureester, Sorbinsäure, Benzoesäure, Citronensäure, Ascorbinsäure, Tanninsäure, Natriumchlorid, Kaliumchlorid, Magnesiumchlorid, Calciumchlorid, Magnesiumoxid, Zinkoxid, Siliciumdioxid, Titanoxid, Titandioxid, Magnesiumsulfat, Zinksulfat, Calciumsulfat, Pottasche, Calciumphosphat, Dicalciumphosphat, Kaliumbromid, Kaliumiodid, Talkum, Kaolin, Pectin, Crospovidon, Agar und Bentonit.

Die Herstellung der erfindungsgemäßen Arzneimittel und pharmazeutischen Zusammensetzungen erfolgt mit Hilfe von im Stand der Technik der pharmazeutischen Formulierung wohlbekannten Mitteln, Vorrichtungen, Methoden und Verfahren, wie sie beispielsweise in "Remington's Pharmaceutical Sciences", Hrsg. A.R. Gennaro, 17. Ed., Mack Publishing Company, Easton, Pa. (1985), insbesondere in Teil 8, Kapitel 76 bis 93, beschrieben sind.

So kann z.B. für eine feste Formulierung, wie eine Tablette, der Wirkstoff des Arzneimittels mit einem pharmazeutischen Träger, z.B. herkömmlichen Tabletteninhaltsstoffen, wie Maisstärke, Lactose, Saccharose, Sorbitol, Talkum, Magnesiumstearat, Dicalciumphosphat oder pharmazeutisch akzeptable Gummis, und pharmazeutischen Verdünnungsmitteln, wie z.B. Wasser, granuliert werden, um eine feste Zusammensetzungzu bilden, die Wirkstoff in homogener Verteilung enthält. Unter einer homogenen Verteilung wird hier verstanden, daß der Wirkstoff gleichmäßig über die gesamte Zusammensetzung verteilt ist, so daß diese ohne weiteres in gleich wirksame Einheitsdosis-Formen, wie Tabletten, Pillen oder Kapseln, unterteilt werden kann. Die feste Zusammensetzungwird anschließend in Einheitsdosis-Formen unterteilt. Die Tabletten oder Pillen des erfindungsgemäßen Arzneimittels bzw. der erfindungsgemäßen Zusammensetzungen können auch überzogen oder auf andere Weise kompoundiert werden, um eine Dosisform mit verzögerter Freisetzung bereitzustellen. Geeignete Beschichtungsmittel sind u.a. polymere Säuren und Mischungen von polymeren Säuren mit Materialien wie z.B. Schellack, Cetylalkohol und/oder Celluloseacetat.

Auch wenn die erfindungsgemässen Arzneimittel lediglich geringe Nebenwirkungen zeigen, kann es beispielsweise zur Vermeidung von bestimmten Formen der Abhängigkeit von Vorteil sein, neben der Kombination der **Verbindungen A** und **B** auch Morphinantagonisten, insbesondere Naloxon, Naltrexon und/oder Levallorphan, zu verwenden.

Weiter betrifft die Erfindung auch ein Verfahren zur Behandlung von vermehrtem Harndrang bzw. Harninkontinenz, bei dem die Wirkstoffkombination aus **Verbindung A** und **Verbindung B**, insbesondere in einer therapeutisch (jeweils) wirksamen Dosierung, verwendet wird.

Die folgenden Beispiele sollen die Erfindung erläutern, ohne daß der Gegenstand der Erfindung darauf beschränkt wäre.

### Beispiele

### Beispiel 1. Testsystem Cystometrie an der narkotisierten naiven Ratte

Die cystometrische Untersuchung an naiven weiblichen Ratten wurde nach der Methode von Kimura et al. (Kimura et al., 1996, Int. J. Urol. 3:218-227) durchgeführt. An narkotisierten, ventilierten Ratten wird das Abdomen eröffnet und die Harnleiter abgebunden. Der Harn wird von den Nieren abgeleitet. Ein Katheter wird in die Blase eingeführt und fixiert. Über diesen wird Saline mittels Infusionspumpe in die Blase infundiert, bis diese rhythmische Spontanaktivität in Form von Kontraktionen zeigt, welche über einen angeschlossenen Druckaufnehmer aufgenommen werden können. Die Testsubstanz wird nach Erreichen stabiler Ausgangswerte i.v. appliziert. Eine Beeinflussung der Blasenfunktion äußert sich über die Unterdrückung der Spontankontraktionen. Im vorliegenden Beispiel wurde Verbindung A ((+)-(2*R*,3*R*)-1-Dimethylamino-3-(3-methoxy-phenyl)-2-methyl-pentan-3-ol; hydrochlorid) in der Dosierung 0.1 mg/kg i.v. mit Verbindung B (Oxybutynin) in der Dosierung 0.03mg/kg i.v. kombiniert und die Wirkung dieser Kombination mit der der Einzelsubstanzen verglichen. Einzelsubstanzen und Kombination zeigten eine Hemmung der Kontraktionsrate (Miktionsereignisse/min). Die Daten sind in der folgenden Tabelle zusammengefasst.

| Substanz | Verbindung A 0.1 mg/kg i.v. | Verbindung B 0.03mg/kg i.v. | Verbindung A 0.1 mg/kg i.v. + Verbindung B 0.3mg/kg i.v. | Vehikelkontrolle i.v. |
|---|---|---|---|---|
| Hemmung der Kontraktionsrate gegenüber dem Vortest [%MPE] | 21,7% | 10,7% | 42,5% | 4,0% |

Bei allen hier aufgelisteten Substanzen und Kombinationen war eine Unterdrückung der Spontankontraktionen in den Ratten meßbar.

Die untersuchte Kombination der Substanzen zeigt eine positive Wirkung auf die Blasenregulation und ist somit geeignet zur Behandlung der Harninkontinenz.

### Beispiel 2: Parenterale Applikationsform

20 g Tramadol und 1 g Tolterodin wird in 1 l Wasser für Injektionszwecke bei Raumtemperatur gelöst und anschließend durch Zugabe von NaCl auf isotone Bedingungen eingestellt.

## Patentansprüche

1. Verwendung einer Wirkstoffkombination aus wenigstens einer der **Verbindungen A** und wenigstens einer der **Verbindungen B**, mit **Verbindung A** ausgewählt aus:
**Gruppe a)** enthaltend:
Tramadol, O-Demethyltramadol, oder O-desmethyl-N-monodesmethyl-tramadol gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in Form ihrer Säuren oder ihrer Basen oder in Form ihrer Salze, insbesondere der physiologisch verträglichen Salze, oder in Form ihrer Solvate, insbesondere der Hydrate;;
**Gruppe b)** enthaltend:
• Codein
• Dextropropxyphen
• Dihydrocodein
• Diphenoxylat
• Ethylmorphin
• Meptazinol
• Nalbuphin
• Pethidin (Meperidine)
• Tilidin
• Tramadol
• Viminol
• Butorphanol
• Dextromoramid
• Dezocin
• Diacetylmorphin (Heroin)
• Hydrocodon
• Hydromorphon
• Ketobemidon
• Levomethadon
• Levomethadyl-Acetate (I-α-Acetylmethadol (LAAM))
• Levorphanol
• Morphin
• Nalorphin
• Oxycodon
• Pentazocin
• Piritramide
• Alfentanil
• Buprenorphin
• Etorphin
• Fentanyl
• Remifentanil
• Sufentanil
gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in Form ihrer Säuren oder ihrer Basen oder in Form ihrer Salze, insbesondere der physiologisch verträglichen Salze, oder in Form ihrer Solvate, insbesondere der Hydrate;
**Gruppe c)** enthaltend:
1-Phenyl-3-dimethylamino-propanverbindungen gemäß allgemeiner **Formel I** 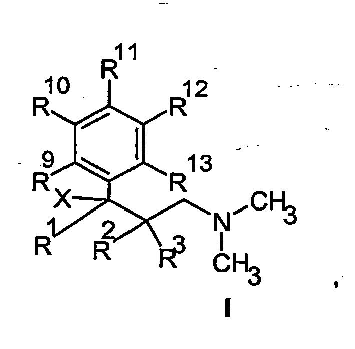
worin
X ausgewählt ist aus OH, F, Cl, H oder OC(O)R⁷ mit R⁷ ausgewählt aus C₁₋₃-Alkyl, verzweigt oder unverzweigt, gesättigt oder ungesättigt, unsubstituiert oder ein- oder mehrfach substituiert,
R¹ ausgewählt ist aus C₁₋₄-Alkyl, verzweigt oder unverzweigt, gesättigt oder ungesättigt, unsubstituiert oder ein- oder mehrfach substituiert,
R² und R³ jeweils unabhängig voneinander ausgewählt sind aus H oder C₁₋₄-Alkyl, verzweigt oder unverzweigt, gesättigt oder ungesättigt, unsubstituiert oder ein- oder mehrfach substituiert,
oder
R² und R³ zusammen einen gesättigten C₄₋₇-Cycloalkylrest bilden, unsubstituiert oder ein- oder mehrfach substituiert,
R⁹ bis R¹³ jeweils unabhängig voneinander ausgewählt sind aus H, F, Cl, Br, I, CH₂F, CHF₂, CF₃, OH, SH, OR¹⁴, OCF₃, SR¹⁴, NR¹⁷R¹⁸, SOCH₃, SOCF_{3;} SO₂CH₃, SO₂CF₃, CN, COOR¹⁴, NO₂, CONR¹⁷R¹⁸; C₁₋₆-Alkyl, verzweigt oder unverzweigt, gesättigt oder ungesättigt, unsubstituiert oder ein-oder mehrfach substituiert; Phenyl, unsubstituiert oder ein- oder mehrfach substituiert;
mit R¹⁴ ausgewählt aus C₁₋₆-Alkyl; Pyridyl, Thienyl, Thiazolyl, Phenyl, Benzyl oder Phenethyl, jeweils unsubstituiert oder ein- oder mehrfach substituiert; PO(O-C₁₋₄-Alkyl)₂, CO(OC₁₋₅-Alkyl), CONH-C₆H₄-(C₁₋₃-Alkyl), CO(C₁₋₅-Alkyl), CO-CHR¹⁷-NHR¹⁸, CO-C₆H₄-R¹⁵, mit R¹⁵ ortho-OCOC₁₋₃-Alkyl oder meta- oder para-CH₂N(R¹⁶)₂ mit R¹⁶ C₁₋₄-Alkyl oder 4-Morpholino, wobei in den Resten R¹⁴, R¹⁵ und R¹⁶ die Alkylgruppen verzweigt oder unverzweigt, gesättigt oder ungesättigt, unsubstituiert oder ein- oder mehrfach substituiert sein können;
mit R¹⁷ und R18 jeweils unabhängig voneinander ausgewählt aus H; C₁₋₆-Alkyl, verzweigt oder unverzweigt, gesättigt oder ungesättigt, unsubstituiert oder ein- oder mehrfach substituiert; Phenyl, Benzyl oder Phenethyl, jeweils unsubstituiert oder ein- oder mehrfach substituiert,
oder
R⁹ und R¹⁰ oder R¹⁰ und R¹¹ zusammen einen OCH₂O-, OCH₂CH₂O-, OCH=CH-, CH=CHO-, CH=C(CH3)O-, OC(CH3)=CH-, (CH₂)₄- oder OCH=CHO-Ring bilden,
gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form ihrer Säuren oder ihrer Basen oder in Form ihrer Salze, insbesondere der physiologisch verträglichen Salze, oder in Form ihrer Solvate, insbesondere der Hydrate;;
**Gruppe d)** enthaltend:
substituierte 6-Dimethylaminomethyl-1-phenyl-cyclohexanverbindungen gemäß allgemeiner **Formel II** 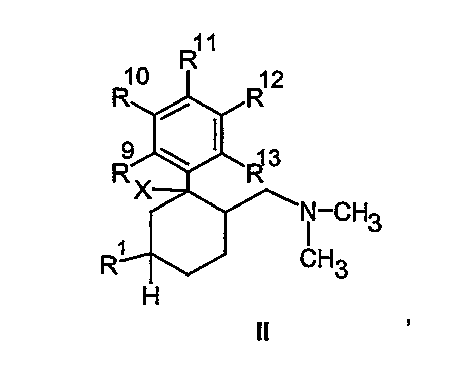
worin
X ausgewählt ist aus OH, F, Cl, H oder OC(O)R⁷ mit R⁷ ausgewählt aus C₁₋₃-Alkyl, verzweigt oder unverzweigt, gesättigt oder ungesättigt, unsubstituiert oder ein-oder mehrfach substituiert,
R¹ ausgewählt ist aus C₁₋₄-Alkyl, Benzyl, CF₃, OH, OCH₂-C₆H₅, O-C₁₋₄-Alkyl, Cl oder F und
R⁹ bis R¹³ jeweils unabhängig voneinander ausgewählt sind aus H, F, Cl, Br, I, CH₂F, CHF₂, CF₃, OH, SH, OR¹⁴, OCF₃, SR¹⁴, NR¹⁷R¹⁸, SOCH₃, SOCF₃; SO₂CH₃, SO₂CF₃, CN, COOR¹⁴, NO₂, CONR¹⁷R¹⁸; C₁₋₆-Alkyl, verzweigt oder unverzweigt, gesättigt oder ungesättigt, unsubstituiert oder ein- oder mehrfach substituiert; Phenyl, unsubstituiert oder ein- oder mehrfach substituiert;
mit R¹⁴ ausgewählt aus C₁₋₆-Alkyl; Pyridyl, Thienyl, Thiazolyl, Phenyl, Benzyl oder Phenethyl, jeweils unsubstituiert oder ein- oder mehrfach substituiert; PO(O-C₁₋₄-Alkyl)₂, CO(OC₁₋₅-Alkyl), CONH-C₆H₄-(C₁₋₃-Alkyl), CO(C₁₋₅-Alkyl), CO-CHR¹⁷-NHR¹⁸, CO-C₆H₄-R¹⁵, mit R¹⁵ ortho-OCOC₁₋₃-Alkyl oder meta- oder para-CH₂N(R¹⁶)₂ mit R¹⁶ C₁₋₄-Alkyl oder 4-Morpholino, wobei in den Resten R¹⁴, R¹⁵ und R¹⁶ die Alkylgruppen verzweigt oder unverzweigt, gesättigt oder ungesättigt, unsubstituiert oder ein- oder mehrfach substituiert sein können;
mit R¹⁷ und R18 jeweils unabhängig voneinander ausgewählt aus H; C₁₋₆-Alkyl, verzweigt oder unverzweigt, gesättigt oder ungesättigt, unsubstituiert oder ein- oder mehrfach substituiert; Phenyl, Benzyl oder Phenethyl, jeweils unsubstituiert oder ein- oder mehrfach substituiert,
oder
R⁹ und R¹⁰ oder R¹⁰ und R¹¹ zusammen einen OCH₂O-, OCH₂CH₂O-, OCH=CH-, CH=CHO-, CH=C(CH3)O-, OC(CH3)=CH-, (CH₂)₄- oder OCH=CHO-Ring bilden,
gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form ihrer Säuren oder ihrer Basen oder in Form ihrer Salze, insbesondere der physiologisch verträglichen Salze, oder in Form ihrer Solvate, insbesondere der Hydrate;
**und/oder**
**Gruppe e)** enthaltend:
6-Dimethylaminomethyl-1-phenyl-cyclohexanverbindungen gemäß allgemeiner **Formel III** 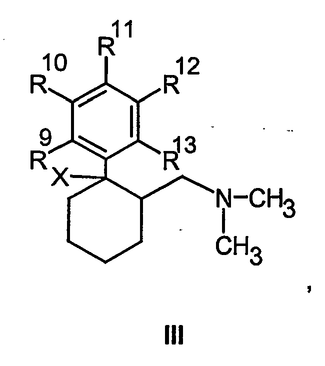
worin
X ausgewählt ist aus OH, F, Cl, H oder OC(O)R⁷ mit R⁷ ausgewählt aus C₁₋₃-Alkyl, verzweigt oder unverzweigt, gesättigt oder ungesättigt, unsubstituiert oder ein- oder mehrfach substituiert, und
R⁹ bis R¹³ jeweils unabhängig voneinander ausgewählt sind aus H, F, Cl, Br, I, CH₂F, CHF₂, CF₃, OH, SH, OR¹⁴, OCF₃, SR¹⁴, NR¹⁷R¹⁸, SOCH₃, SOCF₃; SO₂CH₃, SO₂CF₃, CN, COOR¹⁴, NO₂, CONR¹⁷R¹⁸; C₁₋₆-Alkyl, verzweigt oder unverzweigt, gesättigt oder ungesättigt, unsubstituiert oder ein-oder mehrfach substituiert; Phenyl, unsubstituiert oder ein- oder mehrfach substituiert;
mit R¹⁴ ausgewählt aus C₁₋₆-Alkyl; Pyridyl, Thienyl, Thiazolyl, Phenyl, Benzyl oder Phenethyl, jeweils unsubstituiert oder ein- oder mehrfach substituiert; PO(O-C₁₋₄-Alkyl)₂, CO(OC₁₋₅-Alkyl), CONH-C₆H₄-(C₁₋₃-Alkyl), CO(C₁₋₅-Alkyl), CO-CHR¹⁷-NHR¹⁸, CO-C₆H₄-R¹⁵, mit R¹⁵ ortho-OCOC₁₋₃-Alkyl oder meta- oder para-CH₂N(R¹⁶)₂ mit R¹⁶ C₁₋₄-Alkyl oder 4-Morpholino, wobei in den Resten R¹⁴, R¹⁵ und R¹⁶ die Alkylgruppen verzweigt oder unverzweigt, gesättigt oder ungesättigt, unsubstituiert oder ein- oder mehrfach substituiert sein können;
mit R¹⁷ und R18 jeweils unabhängig voneinander ausgewählt aus H; C₁₋₆-Alkyl, verzweigt oder unverzweigt, gesättigt oder ungesättigt, unsubstituiert oder ein- oder mehrfach substituiert; Phenyl, Benzyl oder Phenethyl, jeweils unsubstituiert oder ein- oder mehrfach substituiert,
oder
R⁹ und R¹⁰ oder R¹⁰ und R¹¹ zusammen einen OCH₂O-, OCH₂CH₂O-, OCH=CH-, CH=CHO-, CH=C(CH3)O-, OC(CH3)=CH-, (CH₂)₄- oder OCH=CHO-Ring bilden,
mit der Maßgabe, daß, wenn R⁹, R¹¹ und R¹³ H entsprechen, und einer von R¹⁰ oder R¹² H und der andere OCH₃ entspricht, X nicht OH sein darf,
gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form ihrer Säuren oder ihrer Basen oder in Form ihrer Salze, insbesondere der physiologisch verträglichen Salze, öder in Form ihrer Solvate, insbesondere der Hydrate;
**und** mit wenigstens einer der **Verbindungen B**, ausgewählt aus:
den Antimuskarinika: Atropin, Oxybutinin, Propiverin, Propanthelin, Emepronium, Trospium, Tolterodin, Darifenacin und α,α-Diphenylessigsäure-4-(N-methyl-piperidyl)-ester, sowie Duloxetin, Imipramin und Desmopressin,
gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in Form ihrer Säuren oder ihrer Basen oder in Form ihrer Salze, insbesondere der physiologisch verträglichen Salze, oder in Form ihrer Solvate, insbesondere der Hydrate;
zur Herstellung eines Arzneimittels zur Behandlung von vermehrtem Harndrang bzw. Harninkontinenz.

2. Verwendung gemäß Anspruch 1, **dadurch gekennzeichnet, daß** die **Verbindung A** in **Gruppe a)** ausgewählt ist aus:
Tramadol, (+)-Tramadol, (+)-O-Demethyltramadol oder (+)-O-desmethyl-N-mono-desmethyl-tramadol, vorzugsweise Tramadol oder (+)-Tramadol, insbesondere (+)-Tramadol.

3. Verwendung gemäß Anspruch 1, **dadurch gekennzeichnet, daß** die **Verbindung A** in **Gruppe b)** ausgewählt ist aus:
• Codein
• Dextropropxyphen
• Dihydrocodein
• Diphenoxylat
• Ethylmorphin
• Meptazinol
• Nalbuphin
• Pethidin (Meperidine)
• Tilidin
• Viminol
• Butorphanol
• Dezocin
• Nalorphin
• Pentazocin
• Buprenorphin,
vorzugsweise
• Codein
• Dextropropxyphen
• Dihydrocodein
• Meptazinol
• Nalbuphin
• Tilidin
• Buprenorphin

4. Verwendung gemäß Anspruch 1, **dadurch gekennzeichnet, daß** die **Verbindung A** in **Gruppe c)** ausgewählt ist aus Verbindungen gemäß **Formel I** für die gilt:
X ausgewählt ist aus
OH, F, Cl, OC(O)CH₃ oder H, vorzugsweise OH, F, OC(O)CH₃ oder H,
**und/oder**
R¹ ausgewählt ist aus
C₁₋₄-Alkyl, gesättigt und unsubstituiert, verzweigt oder unverzweigt; vorzugsweise CH₃, C₂H₅, C₄H₉ oder t-Butyl, insbesondere CH₃ oder C₂H₅,
**und/oder**
R² und R³ unabhängig voneinander ausgewählt sind aus
H, C₁₋₄-Alkyl, gesättigt und unsubstituiert, verzweigt oder unverzweigt; vorzugsweise H, CH₃, C₂H₅, i-Propyl oder t-Butyl, insbesondere H oder CH₃, vorzugsweise R³ = H,
oder
R² und R³ zusammen einen C₅₋₆-Cycloalkylrest bilden, gesättigt oder ungesättigt, unsubstituiert oder ein- oder mehrfach substituiert, vorzugsweise gesättigt und unsubstituiert, insbesondere Cyclohexyl.
**und/oder**
R⁹ bis R¹³, wobei 3 oder 4 der Reste R⁹ bis R¹³ H entsprechen müssen, unabhängig voneinander ausgewählt sind aus
H, Cl, F, OH, CF₂H, CF₃ oder C₁₋₄-Alkyl, gesättigt und unsubstituiert, verzweigt oder unverzweigt; OR¹⁴ oder SR¹⁴, mit R¹⁴ ausgewählt aus C₁₋₃-Alkyl, gesättigt und unsubstituiert, verzweigt oder unverzweigt;
vorzugsweise H, Cl, F, OH, CF₂H, CF₃, OCH₃ oder SCH₃
oder R¹² und R¹¹ einen 3,4-OCH=CH-Ring bilden
insbesondere
wenn R⁹, R¹¹ und R¹³ H entsprechen, einer von R¹⁰ oder R¹² auch H entspricht, während der andere ausgewählt ist aus:
Cl, F, OH, CF₂H, CF₃, OR¹⁴ oder SR¹⁴, vorzugsweise OH, CF₂H, OCH₃ oder SCH₃
oder,
wenn R⁹ und R¹³ H entsprechen und R¹¹ OH, OCH₃, Cl oder F, vorzugsweise Cl, entspricht, einer von R¹⁰ oder R¹² auch H entspricht, während der andere OH, OCH₃, Cl oder F, vorzugsweise Cl, entspricht,
oder,
wenn R⁹, R¹⁰, R¹² und R¹³ H entsprechen, R¹¹ ausgewählt ist aus CF₃, CF₂H, Cl oder F, vorzugsweise F,
oder,
wenn R¹⁰, R¹¹ und R¹² H entsprechen, einer von R⁹ oder R¹³ auch H entspricht, während der andere ausgewählt ist aus OH, OC₂H₅ oder OC₃H₇.

5. Verwendung gemäß Anspruch 4, **dadurch gekennzeichnet, daß** Verbindungen der **Formel I** mit R³ = H in Form der Diastereomeren mit der relativen Konfiguration la 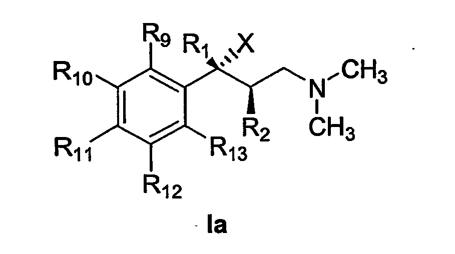 vorliegen, insbesondere in Mischungen mit höherem Anteil dieses Diastereomeren im Vergleich zum anderen Diastereomeren oder als reines Diastereomer verwendet werden
**und/oder**
daß die Verbindungen der **Formel I** in Form des (+)-Enantiomeren, insbesondere in Mischungen mit höherem Anteil des (+)-Enantiomeren im Vergleich zum (-)-Enantiomeren einer racemischen Verbindung oder als reines (+)-Enantiomer verwendet werden.

6. Verwendung gemäß einem der Ansprüche 4 oder 5, **dadurch gekennzeichnet, daß Verbindung A** ausgewählt aus folgender Gruppe verwendet wird:
■ (2RS,3RS)-1-Dimethylamino-3-(3-methoxy-phenyl)-2-methyl-pentan-3-ol,
■ (2R,3R)-1-Dimethylamino-3-(3-methoxy-phenyl)-2-methyl-pentan-3-ol,
■ (+)-(2R,3R)-1-Dimethylamino-3-(3-methoxy-phenyl)-2-methyl-pentan-3-ol,
■ (2RS,3RS)-3-(3,4-Dichlorophenyl)-1-dimethylamino-2-methyl-pentan-3-ol,
■ (2RS,3RS)-3-(3-Difluoromethyl-phenyl)-1-dimethylamino-2-methyl-pentan-3-ol,
■ (2RS,3RS)-1-Dimethylamino-2-methyl-3-(3-methylsulfanyl-phenyl)-pentan-3-ol,
*■* (3RS)-1-Dimethylamino-3-(3-methoxy-phenyl)-4,4-dimethyl-pentan-3-ol,
■ (2RS,3RS)-3-(3-Dimethylamino-1-ethyl-1-hydroxy-2-methylpropyl)-phenol,
■ (1RS,2RS)-3-(3-Dimethylamino-1-hydroxy-1,2-dimethyl-propyl)-phenol,
■ (+)-(1R,2R)-3-(3-Dimethylamino-1-hydroxy-1,2-dimethylpropyl)-phenol,
■ (+)-(1R,2R)-3-(3-Dimethylamino-1-hydroxy-1,2-dimethyl-propyl)-phenol,
■ (1R,2R)-3-(3-Dimethylamino-1-ethyl-2-methyl-propyl)-phenol,
■ (-)-(1R,2R)-3-(3-Dimethylamino-1-ethyl-2-methyl-propyl)-phenol,
■ (1S,2S)-3-(3-Dimethylamino-1-ethyl-2-methyl-propyl)-phenol,
■ (+)-(1S,2S)-3-(3-Dimethylamino-1-ethyl-2-methyl-propyl)-phenol,
■ (+)-(1R,2R)-Essigsäure-3-dimethylamino-1-ethyl-1-(3-methoxy-phenyl)-2-methyl-propylester,
■ (1RS)-1-(1-Dimethylaminomethyl-cyclohexyl)-1-(3-methoxy-phenyl)-propan-1-ol,
■ (2RS,3RS)-3-(4-Chlorophenyl)-1-dimethylamino-2-methyl-pentan-3-ol,
■ (+)-(2R,3R)-3-(3-Dimethylamino-1-ethyl-1-hydroxy-2-methylpropyl)-phenol,
■ (2RS,3RS)-4-Dimethylamino-2-(3-methoxy-phenyl)-3-methyl-butan-2-ol und
■ (+)-(2R,3R)-4-Dimethylamino-2-(3-methoxy-phenyl)-3-methyl-butan-2-ol,
vorzugsweise als Hydrochlorid.

7. Verwendung gemäß Anspruch 1, **dadurch gekennzeichnet, daß** die **Verbindung A** in **Gruppe d)** ausgewählt ist aus Verbindungen gemäß **Formel II** für die gilt, daß:
X ausgewählt ist aus
OH, F, Cl, OC(O)CH₃ oder H, vorzugsweise OH, F oder H, insbesondere OH,
**und/oder**
R¹ ausgewählt ist aus
C₁₋₄-Alkyl, CF₃, OH, O-C₁₋₄-Alkyl, Cl oder F, vorzugsweise OH, CF₃ oder CH₃,
**und/oder**
R⁹ bis R¹³, wobei 3 oder 4 der Reste R⁹ bis R¹³ H entsprechen müssen, unabhängig voneinander ausgewählt sind aus
H, Cl, F, OH, CF₂H, CF₃ oder C₁₋₄-Alkyl, gesättigt und unsubstituiert, verzweigt oder unverzweigt; OR¹⁴ oder SR¹⁴, mit R¹⁴ ausgewählt aus C₁₋₃-Alkyl, gesättigt und unsubstituiert, verzweigt oder unverzweigt;
vorzugsweise H, Cl, F, OH, CF₂H, CF₃, OCH₃ oder SCH₃
oder R¹² und R¹¹ einen 3,4-OCH=CH-Ring bilden,
insbesondere
wenn R⁹, R¹¹ und R¹³ H entsprechen, einer von R¹⁰ oder R¹² auch H entspricht, während der andere ausgewählt ist aus:
Cl, F, OH, CF₂H, CF₃, OR¹⁴ oder SR¹⁴, vorzugsweise OH, CF₂H, OR¹⁴ oder SCH₃, insbesondere OH oder OC₁₋₃-Alkyl, vorzugsweise OH oder OCH₃,
oder,
wenn R⁹ und R¹³ H entsprechen und R¹¹ OH, OCH₃, Cl oder F, vorzugsweise Cl, entspricht, einer von R¹⁰ oder R¹² auch H entspricht, während der andere OH, OCH₃, Cl oder F, vorzugsweise Cl, entspricht,
oder,
wenn R⁹, R¹⁰, R¹² und R¹³ H entsprechen, R¹¹ ausgewählt ist aus CF₃, CF₂H, Cl oder F, vorzugsweise F,
oder,
wenn R¹⁰, R¹¹ und R¹² H entsprechen, einer von R⁹ oder R¹³ auch H entspricht, während der andere ausgewählt ist aus OH, OC₂H₅ oder OC₃H₇,
ganz insbesondere bevorzugt,
wenn R⁹, R¹¹ und R¹³ H entsprechen, einer von R¹⁰ oder R¹² auch H entspricht, während der andere ausgewählt ist aus:
Cl, F, OH, SH, CF₂H, CF₃, OR¹⁴ oder SR¹⁴, vorzugsweise OH oder OR¹⁴, insbesondere OH oder OC₁₋₃-Alkyl, vorzugsweise OH oder OCH₃.

8. Verwendung gemäß Anspruch 7, **dadurch gekennzeichnet, daß** Verbindungen der **Formel II** in Form der Diastereomeren mit der relativen Konfiguration IIa 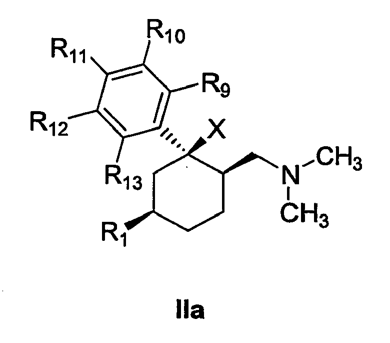 vorliegen, insbesondere in Mischungen mit höherem Anteil dieses Diastereomeren im Vergleich zum anderen Diastereomeren oder als reines Diastereomer verwendet werden,
**und/oder**
daß die Verbindungen der **Formel II** in Form des (+)-Enantiomeren, insbesondere in Mischungen mit höherem Anteil des (+)-Enantiomeren im Vergleich zum (-)-Enantiomeren einer racemischen Verbindung oder als reines (+)-Enantiomer verwendet werden.

9. Verwendung gemäß einem der Ansprüche 7 oder 8, **dadurch gekennzeichnet, daß Verbindung A** ausgewählt aus folgender Gruppe verwendet wird:
■ (1RS,3RS,6RS)-6-Dimethylaminomethyl-1-(3-methoxy-phenyl)-cyclohexan-1,3-diol,
■ (+)-(1R,3R,6R)-6-Dimethylaminomethyl-1-(3-methoxy-phenyl)-cyclohexan-1,3-diol,
■ (1RS,3RS,6RS)-6-Dimethylaminomethyl-1-(3-hydroxyphenyl)-cyclohexan-1,3-diol,
■ (1RS,3SR,6RS)-6-Dimethylaminomethyl-1-(3-methoxy-phenyl)-cyclohexan-1,3-diol,
■ (+)-(1R,2R,5S)-3-(2-Dimethylaminomethyl-1-hydroxy-5-methyl-cyclohexyl)-phenol oder
■ (1RS,2RS,5RS)-3-(2-Dimethylaminomethyl-1-hydroxy-5-trifluoromethyl-cyclohexyl)-phenol,
vorzugsweise als Hydrochlorid.

10. Verwendung gemäß Anspruch 1, **dadurch gekennzeichnet, daß** die **Verbindung A** in **Gruppe e)** ausgewählt ist aus Verbindungen gemäß **Formel III** für die gilt, daß:
X ausgewählt ist aus
OH, F, Cl, OC(O)CH₃ oder H, vorzugsweise OH, F oder H, insbesondere F oder H.
**und/oder**
R⁹ bis R¹³, wobei 3 oder 4 der Reste R⁹ bis R¹³ H entsprechen müssen, unabhängig voneinander ausgewählt sind aus
H, Cl, F, OH, CF₂H, CF₃ oder C₁₋₄-Alkyl, gesättigt und unsubstituiert, verzweigt oder unverzweigt; OR¹⁴ oder SR¹⁴, mit R¹⁴ ausgewählt aus C₁₋₃-Alkyl, gesättigt und unsubstituiert, verzweigt oder unverzweigt;
vorzugsweise H, Cl, F, OH, CF₂H, CF₃, OCH₃ oder SCH₃
oder R¹² und R¹¹ einen 3,4-OCH=CH-Ring bilden
insbesondere **dadurch gekennzeichnet, daß**,
wenn R⁹, R¹¹ und R¹³ H entsprechen, einer von R¹⁰ oder R¹² auch H entspricht, während der andere ausgewählt ist aus:
Cl, F, OH, CF₂H, CF₃, OR¹⁴ oder SR¹⁴, vorzugsweise OH, CF₂H, OR¹⁴ oder SCH₃, insbesondere OH oder OC₁₋₃-Alkyl, vorzugsweise OH oder OCH₃,
oder,
wenn R⁹ und R¹³ H entsprechen und R¹¹ OH, OCH₃, Cl oder F, vorzugsweise Cl, entspricht, einer von R¹⁰ oder R¹² auch H entspricht, während der andere OH, OCH₃, Cl oder F, vorzugsweise Cl, entspricht,
oder,
wenn R⁹, R¹⁰, R¹² und R¹³ H entsprechen, R¹¹ ausgewählt ist aus CF₃, CF₂H, Cl oder F, vorzugsweise F,
oder,
wenn R¹⁰, R¹¹ und R¹² H entsprechen, einer von R⁹ oder R¹³ auch H entspricht, während der andere ausgewählt ist aus OH, OC₂H₅ oder OC₃H₇,
ganz insbesondere bevorzugt,
wenn R⁹, R¹¹ und R¹³ H entsprechen, einer von R¹⁰ oder R¹² auch H entspricht, während der andere ausgewählt ist aus:
Cl, F, OH, SH, CF₂H, CF₃, OR¹⁴ oder SR¹⁴, vorzugsweise OH oder OR¹⁴, insbesondere OH oder OC₁₋₃-Alkyl, vorzugsweise OH oder OCH₃.

11. Verwendung gemäß Anspruch 10, **dadurch gekennzeichnet, daß** Verbindungen der **Formel lll** in Form ihrer Diastereomeren mit der relativen Konfiguration llla 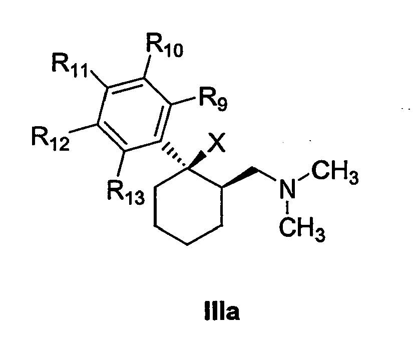 vorliegen, insbesondere in Mischungen mit höherem Anteil dieses Diastereomeren im Vergleich zum anderen Diastereomeren oder als reines Diastereomer verwendet werden
**und/oder**
, daß die Verbindungen der **Formel III** in Form des (+)-Enantiomeren, insbesondere in Mischungen mit höherem Anteil des (+)-Enantiomeren im Vergleich zum (-)-Enantiomeren einer racemischen Verbindung oder als reines (+)-Enantiomer verwendet werden.

12. Verwendung gemäß einem der Ansprüche 10 oder 11, **dadurch gekennzeichnet, daß Verbindung A** ausgewählt aus folgender Gruppe verwendet wird:
■ (+)-(1R,2R)-3-(2-Dimethylaminomethyl-1-fluoro-cyclohexyl)-phenol,
■ (+)-(1S,2S)3-(2-Dimethylaminomethyl-cyclohexyl)-phenol oder
■ (1S,2S)-3-(2-Dimethylaminomethyl-cyclohexyl)-phenol oder
■ (-)-(1R,2R)-3-(2-Dimethylaminomethyl-cyclohexyl)-phenol,
■ (1R,2R)-3-(2-Dimethylaminomethyl-cyclohexyl)-phenol,
■ (-)-(1R,2R)-[2-(3-Methoxy-phenylrcyclohexylmethyl]-dimethylamin,
■ (1R,2R)-[2-(3-Methoxy-phenyl)-cyclohexylmethyl]-dimethylamin,
vorzugsweise als Hydrochlorid.

13. Verwendung gemäß einem der Ansprüche 1 bis 12, **dadurch gekennzeichnet, daß** die **Verbindung B** ausgewählt ist aus:
Darifenacin, Duloxetin, Oxybutinin oder Tolterodin,
vorzugsweise ausgewählt ist aus
Duloxetin, Oxybutinin oder Tolterodin,
vorzugsweise ausgewählt ist aus
Oxybutinin oder Tolterodin.

14. Wirkstoffkombination aus wenigstens einer der **Verbindungen A** und wenigstens einer der **Verbindungen B**, mit **Verbindung A** ausgewählt aus:
**Gruppe a)** enthaltend:
Tramadol, O-Demethyltramadol oder O-desmethyl-N-monodesmethyl-tramadol gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in Form ihrer Säuren oder ihrer Basen oder in Form ihrer Salze, insbesondere der physiologisch verträglichen Salze, oder in Form ihrer Solvate, insbesondere der Hydrate;
**Gruppe b)** enthaltend:
• Codein
• Dextropropxyphen
• Dihydrocodein
• Meptazinol
• Nalbuphin
• Tilidin
• Buprenorphin
gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in Form ihrer Säuren oder ihrer Basen oder in Form ihrer Salze, insbesondere der physiologisch verträglichen Salze, oder in Form ihrer Solvate, insbesondere der Hydrate;
**Gruppe c)** enthaltend:
1-Phenyl-3-dimethylamino-propanverbindungen gemäß allgemeiner **Formel I** 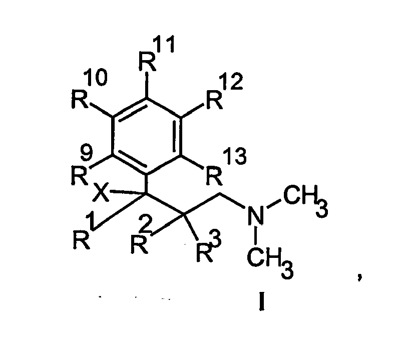
worin
X ausgewählt ist aus OH, F, Cl, H oder OC(O)R⁷ mit R⁷ ausgewählt aus C₁₋₃-Alkyl, verzweigt oder unverzweigt, gesättigt oder ungesättigt, unsubstituiert oder ein- oder mehrfach substituiert,
R¹ ausgewählt ist aus C₁₋₄-Alkyl, verzweigt oder unverzweigt, gesättigt oder ungesättigt, unsubstituiert oder ein- oder mehrfach substituiert,
R² und R³ jeweils unabhängig voneinander ausgewählt sind aus H oder C₁₋₄-Alkyl, verzweigt oder unverzweigt, gesättigt oder ungesättigt, unsubstituiert oder ein- oder mehrfach substituiert,
oder
R² und R³ zusammen einen gesättigten C₄₋₇-Cycloalkylrest bilden, unsubstituiert oder ein- oder mehrfach substituiert,
R⁹ bis R¹³ jeweils unabhängig voneinander ausgewählt sind aus H, F, Cl, Br, l, CH₂F, CHF₂, CF₃, OH, SH, OR¹⁴, OCF₃, SR¹⁴, NR¹⁷R¹⁸, SOCH₃, SOCF₃; SO₂CH₃, SO₂CF₃, CN, COOR¹⁴, NO₂, CONR¹⁷R¹⁸; C₁₋₆-Alkyl, verzweigt oder unverzweigt, gesättigt oder ungesättigt, unsubstituiert oder ein-oder mehrfach substituiert; Phenyl, unsubstituiert oder ein- oder mehrfach substituiert;
mit R¹⁴ ausgewählt aus C₁₋₆-Alkyl; Pyridyl, Thienyl, Thiazolyl, Phenyl, Benzyl oder Phenethyl, jeweils unsubstituiert oder ein- oder mehrfach substituiert; PO(O-C₁₋₄-Alkyl)2, CO(OC₁₋₅-Alkyl), CONH-C₆H₄-(C₁₋₃-Alkyl), CO(C₁₋₅-Alkyl), CO-CHR¹⁷-NHR¹⁸, CO-C₆H₄-R¹⁵, mit R¹⁵ ortho-OCOC₁₋₃-Alkyl oder meta- oder para-CH₂N(R¹⁶)₂ mit R¹⁶ C₁₋₄-Alkyl oder 4-Morpholino, wobei in den Resten R¹⁴, R¹⁵ und R¹⁶ die Alkylgruppen verzweigt oder unverzweigt, gesättigt oder ungesättigt, unsubstituiert oder ein- oder mehrfach substituiert sein können;
mit R¹⁷ und R18 jeweils unabhängig voneinander ausgewählt aus H; C₁₋₆-Alkyl, verzweigt oder unverzweigt, gesättigt oder ungesättigt, unsubstituiert oder ein- oder mehrfach substituiert; Phenyl, Benzyl oder Phenethyl, jeweils unsubstituiert oder ein- oder mehrfach substituiert,
oder
R⁹ und R¹⁰ oder R¹⁰ und R¹¹ zusammen einen OCH₂O-, -OCH₂CH₂O-, OCH=CH-, CH=CHO-, CH=C(CH3)O-, OC(CH3)=CH-, (CH₂)₄- oder OCH=CHO-Ring bilden,
gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form ihrer Säuren oder ihrer Basen oder in Form ihrer Salze, insbesondere der physiologisch verträglichen Salze, oder in Form ihrer Solvate, insbesondere der Hydrate;
**Gruppe d)** enthaltend:
substituierte 6-Dimethylaminomethyl-1-phenyl-cyclohexanverbindungen gemäß allgemeiner **Formel II** 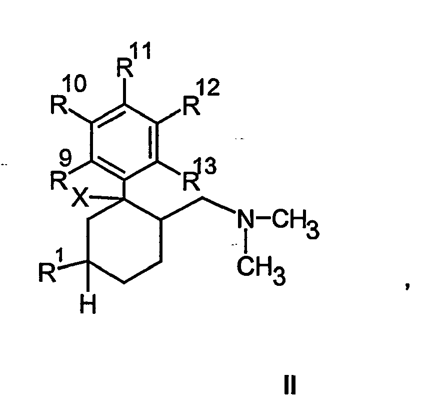
worin
X ausgewählt ist aus OH, F, Cl, H oder OC(O)R⁷ mit R⁷ ausgewählt aus C₁₋₃-Alkyl, verzweigt oder unverzweigt, gesättigt oder ungesättigt, unsubstituiert oder ein- oder mehrfach substituiert,
R¹ ausgewählt ist aus C₁₋₄-Alkyl, Benzyl, CF₃, OH, OCH₂-C₆H₅, O-C₁₋₄-Alkyl, Cl oder F und
R⁹ bis R¹³ jeweils unabhängig voneinander ausgewählt sind aus H, F, Cl, Br, I, CH₂F, CHF₂, CF₃, OH, SH, OR¹⁴, OCF₃, SR¹⁴, NR¹⁷R¹⁸, SOCH₃, SOCF₃; SO₂CH₃, SO₂CF₃, CN, COOR¹⁴, NO₂, CONR¹⁷R¹⁸; C₁₋₆-Alkyl, verzweigt oder unverzweigt, gesättigt oder ungesättigt, unsubstituiert oder ein- oder mehrfach substituiert; Phenyl, unsubstituiert oder ein- oder mehrfach substituiert;
mit R¹⁴ ausgewählt aus C₁₋₆-Alkyl; Pyridyl, Thienyl, Thiazolyl, Phenyl, Benzyl oder Phenethyl, jeweils unsubstituiert oder ein- oder mehrfach substituiert; PO(O-C₁₋₄-Alkyl)₂, CO(OC₁₋₅-Alkyl), CONH-C₆H₄-(C₁₋₃-Alkyl), CO(C₁₋₅-Alkyl), CO-CHR¹⁷-NHR¹⁸, CO-C₆H₄-R¹⁵, mit R¹⁵ ortho-OCOC₁₋₃-Alkyl oder meta- oder para-CH₂N(R¹⁶)₂ mit R¹⁶ C₁₋₄-Alkyl oder 4-Morpholino, wobei in den Resten R¹⁴, R¹⁵ und R¹⁶ die Alkylgruppen verzweigt oder unverzweigt, gesättigt oder ungesättigt, unsubstituiert oder ein- oder mehrfach substituiert sein können;
mit R¹⁷ und R18 jeweils unabhängig voneinander ausgewählt aus H; C₁₋₆-Alkyl, verzweigt oder unverzweigt, gesättigt oder ungesättigt, unsubstituiert oder ein- oder mehrfach substituiert; Phenyl, Benzyl oder Phenethyl, jeweils unsubstituiert oder ein- oder mehrfach substituiert,
oder
R⁹ und R¹⁰ oder R¹⁰ und R¹¹ zusammen einen OCH₂O-, OCH2CH₂O-, OCH=CH-, CH=CHO-, CH=C(CH3)O-, OC(CH3)=CH-, (CH₂)₄- oder OCH=CHO-Ring bilden,
gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form ihrer Säuren oder ihrer Basen oder in Form ihrer Salze, insbesondere der physiologisch verträglichen Salze, oder in Form ihrer Solvate, insbesondere der Hydrate;
**und/oder**
**Gruppe e)** enthaltend:
6-Dimethylaminomethyl-1-phenyl-cyclohexanverbindungen gemäß allgemeiner **Formel III** 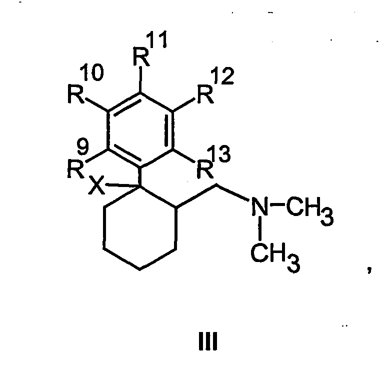
worin
X ausgewählt ist aus OH, F, Cl, H oder OC(O)R⁷ mit R⁷ ausgewählt aus C₁₋₃-Alkyl, verzweigt oder unverzweigt, gesättigt oder ungesättigt, unsubstituiert oder ein- oder mehrfach substituiert, und
R⁹ bis R¹³ jeweils unabhängig voneinander ausgewählt sind aus H, F, Cl, Br, I, CH₂F, CHF₂, CF₃, OH, SH, OR¹⁴, OCF₃, SR¹⁴, NR¹⁷R¹⁸, SOCH₃, SOCF₃; SO₂CH₃, SO₂CF₃, CN, COOR¹⁴, NO₂, CONR¹⁷R¹⁸; C₁₋₆-Alkyl, verzweigt oder unverzweigt, gesättigt oder ungesättigt, unsubstituiert oder ein-oder mehrfach substituiert; Phenyl, unsubstituiert oder ein- oder mehrfach substituiert;
mit R¹⁴ ausgewählt aus C₁₋₆-Alkyl; Pyridyl, Thienyl, Thiazolyl,. Phenyl, Benzyl oder Phenethyl, jeweils unsubstituiert oder ein- oder mehrfach substituiert; PO(O-C₁₋₄-Alkyl)₂, CO(OC₁₋₅-Alkyl), CONH-C₆H₄-(C₁₋₃-Alkyl), CO(C₁₋₅-Alkyl), CO-CHR¹⁷-NHR¹⁸, CO-C₆H₄-R¹⁵, mit R¹⁵ ortho-OCOC₁₋₃-Alkyl oder meta- oder para-CH₂N(R¹⁶)₂ mit R¹⁶ C₁₋₄-Alkyl oder 4-Morpholino, wobei in den Resten R¹⁴, R¹⁵ und R¹⁶ die Alkylgruppen verzweigt oder unverzweigt, gesättigt oder ungesättigt, unsubstituiert oder ein- oder mehrfach substituiert sein können;
mit R¹⁷ und R18 jeweils unabhängig voneinander ausgewählt aus H; C₁₋₆-Alkyl, verzweigt oder unverzweigt, gesättigt oder ungesättigt, unsubstituiert oder ein- oder mehrfach substituiert; Phenyl, Benzyl oder Phenethyl, jeweils unsubstituiert oder ein- oder mehrfach substituiert,
oder
R⁹ und R¹⁰ oder R¹⁰ und R¹¹ zusammen einen OCH₂O-, OCH₂CH₂O-, OCH=CH-, CH=CHO-, CH=C(CH3)O-, OC(CH3)=CH-, (CH₂)₄- oder OCH=CHO-Ring bilden,
mit der Maßgabe, daß, wenn R⁹, R¹¹ und R¹³ H entsprechen, und einer von R¹⁰ oder R¹² H und der andere OCH₃ entspricht, X nicht OH sein darf,
gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form ihrer Säuren oder ihrer Basen oder in Form ihrer Salze, insbesondere der physiologisch verträglichen Salze, oder in Form ihrer Solvate, insbesondere der Hydrate;
**und** mit wenigstens einer der **Verbindungen B,** ausgewählt aus:
den Antimuskarinika: Atropin, Oxybutinin, Propiverin, Propanthelin, Emepronium, Trospium, Tolterodin, Darifenacin und α,α-Diphenylessigsäure-4-(N-methyl-piperidyl)-ester, sowie Duloxetin, lmipramin und Desmopressin,
gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in Form ihrer Säuren oder ihrer Basen oder in Form ihrer Salze, insbesondere der physiologisch verträglichen Salze, oder in Form ihrer Solvate, insbesondere der Hydrate.

15. Wirkstoffkombination gemäß Anspruch 14, **dadurch gekennzeichnet, daß** die **Verbindung A** in **Gruppe a)** ausgewählt ist aus:
Tramadol, (+)-Tramadol, (+)-O-Demethyltramadol oder (+)-O-desmethyl-N-mono-desmethyl-tramadol, vorzugsweise Tramadol oder (+)-Tramadol, insbesondere (+)-Tramadol.

16. Wirkstoffkombination gemäß Anspruch 14, **dadurch gekennzeichnet, daß** die **Verbindung A** in **Gruppe c)** ausgewählt ist aus Verbindungen gemäß **Formel I** für die gilt, daß:
X ausgewählt ist aus
OH, F, Cl, OC(O)CH₃ oder H, vorzugsweise OH, F, OC(O)CH₃ oder H,
**und/oder**
R¹ ausgewählt ist aus
C₁₋₄-Alkyl, gesättigt und unsubstituiert, verzweigt oder unverzweigt; vorzugsweise CH₃, C₂H₅, C₄H₉ oder t-Butyl, insbesondere CH₃ oder C₂H₅,
**und/oder**
R² und R³ unabhängig voneinander ausgewählt sind aus
H, C₁₋₄-Alkyl, gesättigt und unsubstituiert, verzweigt oder unverzweigt; vorzugsweise H, CH₃, C₂H₅, i-Propyl oder t-Butyl, insbesondere H oder CH₃, vorzugsweise R³ = H,
oder
R² und R³ zusammen einen C₅₋₆-Cycloalkylrest bilden, gesättigt oder ungesättigt, unsubstituiert oder ein- oder mehrfach substituiert, vorzugsweise gesättigt und unsubstituiert, insbesondere Cyclohexyl.
**und/oder**
R⁹ bis R¹³, wobei 3 oder 4 der Reste R⁹ bis R¹³ H entsprechen müssen, unabhängig voneinander ausgewählt sind aus
H, Cl, F, OH, CF₂H, CF₃ oder C₁₋₄-Alkyl, gesättigt und unsubstituiert, verzweigt oder unverzweigt; OR¹⁴ oder SR¹⁴, mit R¹⁴ ausgewählt aus C₁₋₃-Alkyl, gesättigt und unsubstituiert, verzweigt oder unverzweigt;
vorzugsweise H, Cl, F, OH, CF₂H, CF₃, OCH₃ oder SCH₃
oder R¹² und R¹¹ einen 3,4-OCH=CH-Ring bilden
insbesondere
wenn R⁹, R¹¹ und R¹³ H entsprechen, einer von R¹⁰ oder R¹² auch H entspricht, während der andere ausgewählt ist aus:
Cl, F, OH, CF₂H, CF₃, OR¹⁴ oder SR¹⁴, vorzugsweise OH, CF₂H, OCH₃ oder SCH₃
oder,
wenn R⁹ und R¹³ H entsprechen und R¹¹ OH, OCH₃, Cl oder F, vorzugsweise Cl, entspricht, einer von R¹⁰ oder R¹² auch H entspricht, während der andere OH, OCH₃, Cl oder F, vorzugsweise Cl, entspricht,
oder,
wenn R⁹, R¹⁰, R¹² und R¹³ H entsprechen, R¹¹ ausgewählt ist aus CF₃, CF₂H, Cl oder F, vorzugsweise F,
oder,
wenn R¹⁰, R¹¹ und R¹² H entsprechen, einer von R⁹ oder R¹³ auch H entspricht, während der andere ausgewählt ist aus OH, OC₂H₅ oder OC₃H₇.

17. Wirkstoffkombination gemäß Anspruch 16, **dadurch gekennzeichnet, daß** die Verbindungen der **Formel I** mit R³ = H in Form der Diastereomeren mit der relativen Konfiguration la 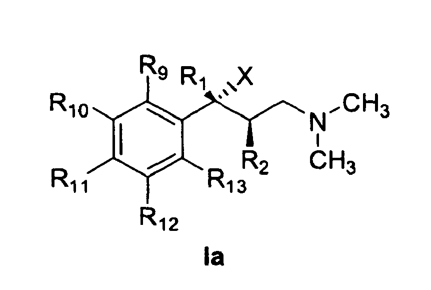 vorliegen, insbesondere in Mischungen mit höherem Anteil dieses Diastereomeren im Vergleich zum anderen Diastereomeren oder als reines Diastereomer
**und/oder**
daß die Verbindungen der **Formel I** in Form des (+)-Enantiomeren, insbesondere in Mischungen mit höherem Anteil des (+)-Enantiomeren im Vergleich zum (-)-Enantiomeren einer racemischen Verbindung oder als reines (+)-Enantiomer vorliegen.

18. Wirkstoffkombination gemäß einem der Ansprüche 16 oder 17, **dadurch gekennzeichnet, daß** die **Verbindung A** ausgewählt ist aus folgender Gruppe:
■ (2RS,3RS)-1-Dimethylamino-3-(3-methoxy-phenyl)-2-methyl-pentan-3-ol,
■ (2R,3R)-1-Dimethylamino-3-(3-methoxy-phenyl)-2-methyl-pentan-3-ol,
■ (+)-(2R,3R)-1-Dimethylamino-3-(3-methoxy-phenyl)-2-methyl-pentan-3-ol,
■ (2RS,3RS)-3-(3,4-Dichlorophenyl)-1-dimethylamino-2-methyl-pentan-3-ol,
■ (2RS,3RS)-3-(3-Difluoromethyl-phenyl)-1-dimethylamino-2-methyl-pentan-3-ol,
■ (2RS,3RS)-1-Dimethylamino-2-methyl-3-(3-methylsulfanyl-phenyl)-pentan-3-ol,
■ (3RS)-1-Dimethylamino-3-(3-methoxy-phenyl)-4,4-dimethyl-pentan-3-ol,
■ (2RS,3RS)-3-(3-Dimethylamino-1-ethyl-1-hydroxy-2-methylpropyl)-phenol,
■ (1RS,2RS)-3-(3-Dimethylamino-1-hydroxy-1,2-dimethyl-propyl)-phenol,
■ (+)-(1R,2R)-3-(3-Dimethylamino-1-hydroxy-1,2-dimethylpropyl)-phenol,
■ (+)-(1R,2R)-3-(3-Dimethylamino-1-hydroxy-1,2-dimethyl-propyl)-phenol,
■ (1R,2R)-3-(3-Dimethylamino-1-ethyl-2-methyl-propyl)-phenol,
■ (-)-(1R,2R)-3-(3-Dimethylamino-1-ethyl-2-methyl-propyl)-phenol,
■ (1S,2S)-3-(3-Dimethylamino-1-ethyl-2-methyl-propyl)-phenol,
■ (+)-(1S,2S)-3-(3-Dimethylamino-1-ethyl-2-methyl-propyl)-phenol,
■ (+)-(1R,2R)-Essigsäure-3-dimethylamino-1-ethyl-1-(3-methoxy-phenyl)-2-methyl-propylester,
■ (1RS)-1-(1-Dimethylaminomethyl-cyclohexyl)-1-(3-methoxy-phenyl)-propan-1-ol,
■ (2RS, 3RS)-3-(4-Chlorophenyl)-1-dimethylamino-2-methyl-pentan-3-ol,
■ (+)-(2R,3R)-3-(3-Dimethylamino-1-ethyl-1-hydroxy-2-methylpropyl)-phenol,
■ (2RS,3RS)-4-Dimethylamino-2-(3-methoxy-phenyl)-3-methyl-butan-2-ol und
■ (+)-(2R,3R)-4-Dimethylamino-2-(3-methoxy-phenyl)-3-methyl-butan-2-ol,
vorzugsweise als Hydrochlorid.

19. Wirkstoffkombination gemäß Anspruch 14, **dadurch gekennzeichnet, daß** die Verbindung A in Gruppe d) ausgewählt ist aus Verbindungen gemäß **Formel II** für die gilt, daß:
X ausgewählt ist aus
OH, F, Cl, OC(O)CH₃ oder H, vorzugsweise OH, F oder H, insbesondere OH,
**und/oder**
R¹ ausgewählt ist aus
C₁₋₄-Alkyl, CF₃, OH, O-C₁₋₄-Alkyl, Cl oder F, vorzugsweise OH, CF₃ oder CH₃,
**und/oder**
R⁹ bis R¹³, wobei 3 oder 4 der Reste R⁹ bis R¹³ H entsprechen müssen, unabhängig voneinander ausgewählt sind aus
H, Cl, F, OH, CF₂H, CF₃ oder C₁₋₄-Alkyl, gesättigt und unsubstituiert, verzweigt oder unverzweigt; OR¹⁴ oder SR¹⁴, mit R¹⁴ ausgewählt aus C₁₋₃-Alkyl, gesättigt und unsubstituiert, verzweigt oder unverzweigt;
vorzugsweise H, Cl, F, OH, CF₂H, CF₃, OCH₃ oder SCH₃
oder R¹² und R¹¹ einen 3,4-OCH=CH-Ring bilden,
insbesondere
wenn R⁹, R¹¹ und R¹³ H entsprechen, einer von R¹⁰ oder R¹² auch H entspricht, während der andere ausgewählt ist aus:
Cl, F, OH, CF₂H, CF₃, OR¹⁴ oder SR¹⁴, vorzugsweise OH, CF₂H, OR¹⁴ oder SCH₃, insbesondere OH oder OC₁₋₃-Alkyl, vorzugsweise OH oder OCH₃,
oder,
wenn R⁹ und R¹³ H entsprechen und R¹¹ OH, OCH₃, Cl oder F, vorzugsweise Cl, entspricht, einer von R¹⁰ oder R¹² auch H entspricht, während der andere OH, OCH₃, Cl oder F, vorzugsweise Cl, entspricht,
oder,
wenn R⁹, R¹⁰, R¹² und R¹³ H entsprechen, R¹¹ ausgewählt ist aus CF₃, CF₂H, Cl oder F, vorzugsweise F,
oder,
wenn R¹⁰, R¹¹ und R¹² H entsprechen, einer von R⁹ oder R¹³ auch H entspricht, während der andere ausgewählt ist aus OH, OC₂H₅ oder OC₃H₇,
ganz insbesondere bevorzugt,
wenn R⁹, R¹¹ und R¹³ H entsprechen, einer von R¹⁰ oder R¹² auch H entspricht, während der andere ausgewählt ist aus:
Cl, F, OH, SH, CF₂H, CF₃, OR¹⁴ oder SR¹⁴, vorzugsweise OH oder OR¹⁴, insbesondere OH oder OC₁₋₃-Alkyl, vorzugsweise OH oder OCH₃.

20. Wirkstoffkombination gemäß Anspruch 19, **dadurch gekennzeichnet, daß** die Verbindungen der **Formel II** in Form der Diastereomeren mit der relativen Konfiguration lla 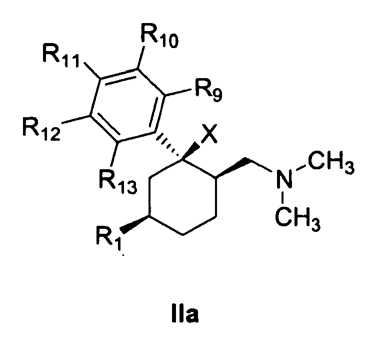 vorliegen, insbesondere in Mischungen mit höherem Anteil dieses Diastereomeren im Vergleich zum anderen Diastereomeren oder als reines Diastereomer,
**und/oder**
daß die Verbindungen der **Formel II** in Form des (+)-Enantiomeren, insbesondere in Mischungen mit höherem Anteil des (+)-Enantiomeren im Vergleich zum (-)-Enantiomeren einer racemischen Verbindung oder als reines (+)-Enantiomer vorliegen.

21. Wirkstoffkombination gemäß einem der Ansprüche 19 oder 20, **dadurch gekennzeichnet, daß Verbindung A** ausgewählt ist aus folgender Gruppe:
■ (1 RS,3RS,6RS)-6-Dimethylaminomethyl-1-(3-methoxy-phenyl)-cyclohexan-1,3-diol,
■ (+)-(1R,3R,6R)-6-Dimethylaminomethyl-1-(3-methoxy-phenyl)-cyclohexan-1,3-diol,
■ (1RS,3RS,6RS)-6-Dimethylaminomethyl-1-(3-hydroxyphenyl)-cyclohexan-1,3-diol,
■ (1RS,3SR,6RS)-6-Dimethylaminomethyl-1-(3-methoxy-phenyl)-cyclohexan-1,3-diol,
■ (+)-(1R,2R,5S)-3-(2-Dimethylaminomethyl-1-hydroxy-5-methyl-cyclohexyl)-phenol oder
■ (1RS,2RS,5RS)-3-(2-Dimethylaminomethyl-1-hydroxy-5-trifluoromethyl-cyclohexyl)-phenol,
vorzugsweise als Hydrochlorid.

22. Wirkstoffkombination gemäß Anspruch 14, **dadurch gekennzeichnet, daß** die **Verbindung A** in **Gruppe e)** ausgewählt ist aus Verbindungen gemäß **Formel III** für die gilt, daß:
X ausgewählt ist aus
OH, F, Cl, OC(O)CH₃ oder H, vorzugsweise OH, F oder H, insbesondere F oder H.
**und/oder**
R⁹ bis R¹³, wobei 3 oder 4 der Reste R⁹ bis R¹³ H entsprechen müssen, unabhängig voneinander ausgewählt sind aus
H, Cl, F, OH, CF₂H, CF₃ oder C₁₋₄-Alkyl, gesättigt und unsubstituiert, verzweigt oder unverzweigt; OR¹⁴ oder SR¹⁴, mit R¹⁴ ausgewählt aus C₁₋₃-Alkyl, gesättigt und unsubstituiert, verzweigt oder unverzweigt;
vorzugsweise H, Cl, F, OH, CF₂H, CF₃, OCH₃ oder SCH₃
oder R¹² und R¹¹ einen 3,4-OCH=CH-Ring bilden
insbesondere **dadurch gekennzeichnet, daß**,
wenn R⁹, R¹¹ und R¹³ H entsprechen, einer von R¹⁰ oder R¹² auch H entspricht, während der andere ausgewählt ist aus:
Cl, F, OH, CF₂H, CF₃, OR¹⁴ oder SR¹⁴, vorzugsweise OH, CF₂H, OR¹⁴ oder SCH₃, insbesondere OH oder OC₁₋₃-Alkyl, vorzugsweise OH oder OCH₃,
oder,
wenn R⁹ und R¹³ H entsprechen und R¹¹ OH, OCH₃, Cl oder F, vorzugsweise Cl, entspricht, einer von R¹⁰ oder R¹² auch H entspricht, während der andere OH, OCH₃, Cl oder F, vorzugsweise Cl, entspricht,
oder,
wenn R⁹, R¹⁰, R¹² und R¹³ H entsprechen, R¹¹ ausgewählt ist aus CF₃, CF₂H, Cl oder F, vorzugsweise F,
oder,
wenn R¹⁰, R¹¹ und R¹² H entsprechen, einer von R⁹ oder R¹³ auch H entspricht, während der andere ausgewählt ist aus OH, OC₂H₅ oder OC₃H₇,
ganz insbesondere bevorzugt,
wenn R⁹, R¹¹ und R¹³ H entsprechen, einer von R¹⁰ oder R¹² auch H entspricht, während der andere ausgewählt ist aus:
Cl, F, OH, SH, CF₂H, CF₃, OR¹⁴ oder SR¹⁴, vorzugsweise OH oder OR¹⁴, insbesondere OH oder OC₁₋₃-Alkyl, vorzugsweise OH oder OCH₃.

23. Wirkstoffkombination gemäß Anspruch 22, **dadurch gekennzeichnet, daß** die Verbindungen der **Formel III** in Form ihrer Diastereomeren mit der relativen Konfiguration IIIa 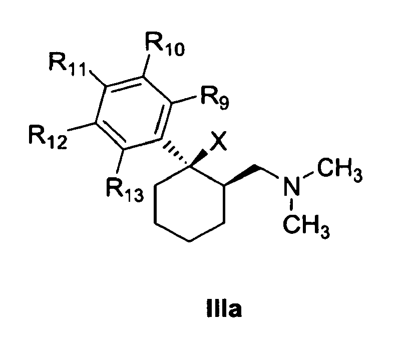 vorliegen, insbesondere in Mischungen mit höherem Anteil dieses Diastereomeren im Vergleich zum anderen Diastereomeren oder als reines Diastereomer
**und/oder**
, daß die Verbindungen der **Formel III** in Form des (+)-Enantiomeren, insbesondere in Mischungen mit höherem Anteil des (+)-Enantiomeren im Vergleich zum (-)-Enantiomeren einer racemischen Verbindung oder als reines (+)-Enantiomer vorliegen.

24. Wirkstoffkombination gemäß einem der Ansprüche 22 oder 23, **dadurch gekennzeichnet, daß** die **Verbindung A** ausgewählt ist aus folgender Gruppe:
■ (+)-(1R,2R)-3-(2-Dimethylaminomethyl-1-fluoro-cyclohexyl)-phenol,
■ (+)-(1S,2S)-3-(2-Dimethylaminomethyl-cyclohexyl)-phenol oder
■ (1S,2S)-3-(2-Dimethylaminomethyl-cyclohexyl)-phenol oder
■ (-)-(1R,2R)-3-(2-Dimethylaminomethyl-cyclohexyl)-phenol,
■ (1R,2R)-3-(2-Dimethylaminomethyl-cyclohexyl)-phenol,
■ (-)-(1R,2R)-[2-(3-Methoxy-phenyl)-cyclohexylmethyl]-dimethylamin,
■ (1R,2R)-[2-(3-Methoxy-phenyl)-cyclohexylmethyl]-dimethylamin,
vorzugsweise als Hydrochlorid.

25. Wirkstoffkombination gemäß einem der Ansprüche 14 bis 24, **dadurch gekennzeichnet, daß** die **Verbindung B** ausgewählt ist aus:
Darifenacin, Duloxetin, Oxybutinin oder Tolterodin,
vorzugsweise ausgewählt ist aus
Duloxetin, Oxybutinin oder Tolterodin,
vorzugsweise ausgewählt ist aus
Oxybutinin oder Tolterodin.

26. Arzneimittel, vorzugsweise zur Behandlung von vermehrtem Harndrang bzw. Harninkontinenz, enthaltend eine Wirkstoffkombination gemäß einem der Ansprüche 14 bis 25 sowie gegebenenfalls geeignete Zusatz- und/oder Hilfsstoffe.

## Claims

1. Use of an active compound combination of at least one of the **compounds A** and at least one of the **compounds B**, with **compound A** chosen from:
**Group a)** comprising:
tramadol, O-demethyltramadol, or O-demethyl-N-mono-demethyl-tramadol, optionally in the form of their racemates, their pure stereoisomers, in particular enantiomers or diastereomers, or in the form of mixtures of the stereoisomers, in particular the enantiomers or diastereomers, in any desired mixing ratio; in the form of their acids or their bases or in the form of their salts, in particular the physiologically acceptable salts, or in the form of their solvates, in particular the hydrates;
**Group b)** comprising:
• codeine
• dextropropoxyphene
• dihydrocodeine
• diphenoxylate
• ethylmorphine
• meptazinol
• nalbuphine
• pethidine (meperidine)
• tilidine
• tramadol
• viminol
• butorphanol
• dextromoramide
• dezocine
• diacetylmorphine (heroin)
• hydrocodone
• hydromorphone
• ketobemidone
• levomethadone
• levomethadyl acetate (1-α-acetylmethadol (LAAM))
• levorphanol
• morphine
• nalorphine
• oxycodone
• pentazocine
• piritramide
• alfentanil
• buprenorphine
• etorphine
• fentanyl
• remifentanil
• sufentanil
optionally in the form of their racemates, their pure stereoisomers, in particular enantiomers or diastereomers, or in the form of mixtures of the stereoisomers, in particular the enantiomers or diastereomers, in any desired mixing ratio; in the form of their acids or their bases or in the form of their salts, in particular the physiologically acceptable salts, or in the form of their solvates, in particular the hydrates;
**Group c)** comprising:
1-phenyl-3-dimethylamino-propane compounds according to the general **formula I** 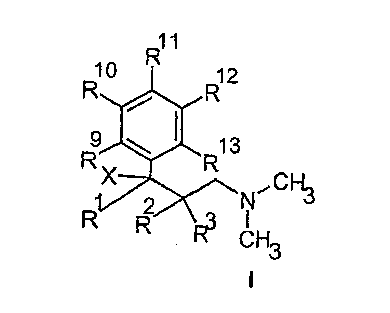
wherein
X is chosen from OH, F, Cl, H or OC(O)R⁷, where R⁷ is chosen from C₁₋₃-alkyl, branched or unbranched, saturated or unsaturated, unsubstituted or mono- or polysubstituted,
R¹ is chosen from C₁₋₄-alkyl, branched or unbranched, saturated or unsaturated, unsubstituted or mono- or polysubstituted,
R² and R³ in each case independently of one another are chosen from H or C₁₋₄-alkyl, branched or unbranched, saturated or unsaturated, unsubstituted or mono- or polysubstituted,
or
R² and R³ together form a saturated C₄₋₇-cycloalkyl radical, unsubstituted or mono- or polysubstituted,
R⁹ to R¹³ in each case independently of one another are chosen from H, F, Cl, Br, I, CH₂F, CHF₂, CF₃, OH, SH, OR¹⁴, OCF₃, SR¹⁴, NR¹⁷R¹⁸, SOCH₃, SOCF₃; SO₂CH₃, SO₂CF₃ CN, COOR¹⁴, NO₂, CONR¹⁷R¹⁸; C₁₋₆-alkyl, branched or unbranched, saturated or unsaturated, unsubstituted or mono- or polysubstituted; phenyl, unsubstituted or mono- or polysubstituted;
where R¹⁴ is chosen from C₁₋₆-alkyl; pyridyl, thienyl, thiazolyl, phenyl, benzyl or phenethyl, in each case unsubstituted or mono- or polysubstituted; PO(O-C₁₋₄-alkyl)₂, CO(OC₁₋₅-alkyl), CONH-C₆H₄- (C₁₋₃-alkyl), CO(C₁₋₅-alkyl), CO-CHR¹⁷-NHR¹⁸, CO-C₆H₄-R¹⁵, where R¹⁵ is ortho-OCOC₁₋₃-alkyl or meta- or para-CH₂N(R¹⁶)₂ where R¹⁶ is C₁₋₄-alkyl or 4-morpholino, wherein in the radicals R¹⁴, R¹⁵ and R¹⁶ the alkyl groups can be branched or unbranched, saturated or unsaturated, unsubstituted or mono- or polysubstituted;
where R¹⁷ and R¹⁸ in each case independently of one another are chosen from H; C₁₋₆-alkyl, branched or unbranched, saturated or unsaturated, unsubstituted or mono- or polysubstituted; phenyl, benzyl or phenethyl, in each case unsubstituted or mono- or polysubstituted,
or
R⁹ and R¹⁰ or R¹⁰ and R¹¹ together form an OCH₂O, OCH₂CH₂O, OCH=CH, CH=CHO, CH=C(CH₃)O, OC(CH₃)=CH, (CH₂)₄ or OCH=CHO ring,
optionally in the form of their racemates, their pure stereoisomers, in particular enantiomers or diastereomers, or in the form of mixtures of the stereoisomers, in particular the enantiomers or diastereomers, in any desired mixing ratio; in the form shown or in the form of their acids or their bases or in the form of their salts, in particular the physiologically acceptable salts, or in the form of their solvates, in particular the hydrates;
**Group d)** comprising:
substituted 6-dimethylaminomethyl-1-phenylcyclohexane compounds according to the general **formula II** 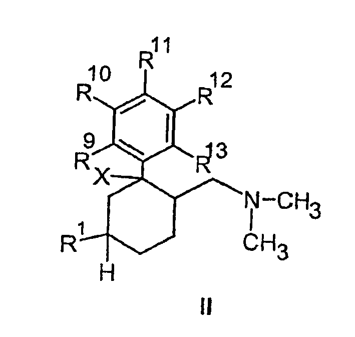
wherein
X is chosen from OH, F, Cl, H or OC(O)R⁷, where R⁷ is chosen from C₁₋₃-alkyl, branched or unbranched, saturated or unsaturated, unsubstituted or mono- or polysubstituted,
R¹ is chosen from C₁₋₄-alkyl, benzyl, CF₃, OH, OCH₂-C₆H₅, O-C₁₋₄-alkyl, Cl or F and
R⁹ to R¹³ in each case independently of one another are chosen from H, F, Cl, Br, I, CH₂F, CHF₂, CF₃, OH, SH, OR¹⁴, OCF₃, SR¹⁴, NR¹⁷R¹⁸, SOCH₃, SOCF₃; SO₂CH₃, SO₂CF₃, CN, COOR¹⁴, NO₂, CONR¹⁷R¹⁸; C₁₋₆-alkyl, branched or unbranched, saturated or unsaturated, unsubstituted or mono- or polysubstituted; phenyl, unsubstituted or mono- or polysubstituted;
where R¹⁴ is chosen from C₁₋₆-alkyl; pyridyl, thienyl, thiazolyl, phenyl, benzyl or phenethyl, in each case unsubstituted or mono- or polysubstituted; PO(O-C₁₋₄-alkyl)₂, CO(OC₁₋₅-alkyl), CONH-C₆H₄- (C₁₋₃-alkyl), CO(C₁₋₅-alkyl), CO-CHR¹⁷-NHR¹⁸, CO-C₆H₄-R¹⁵, where R¹⁵ is ortho-OCOC₁₋₃-alkyl or meta- or para-CH₂N(R¹⁶)₂ where R¹⁶ is C₁₋₄-alkyl or 4-morpholino, wherein in the radicals R¹⁴, R¹⁵ and R¹⁶ the alkyl groups can be branched or unbranched, saturated or unsaturated, unsubstituted or mono- or polysubstituted;
where R¹⁷ and R¹⁸ in each case independently of one another are chosen from H; C₁₋₆-alkyl, branched or unbranched, saturated or unsaturated, unsubstituted or mono- or polysubstituted; phenyl, benzyl or phenethyl, in each case unsubstituted or mono- or polysubstituted,
or
R⁹ and R¹⁰ or R¹⁰ and R¹¹ together form an OCH₂O, OCH₂CH₂O, OCH=CH, CH=CHO, CH=C(CH₃)O, OC(CH₃)=CH, (CH₂)₄ or OCH=CHO ring,
optionally in the form of their racemates, their pure stereoisomers, in particular enantiomers or diastereomers, or in the form of mixtures of the stereoisomers, in particular the enantiomers or diastereomers, in any desired mixing ratio; in the form shown or in the form of their acids or their bases or in the form of their salts, in particular the physiologically acceptable salts, or in the form of their solvates, in particular the hydrates;
**and/or**
**Group e)** comprising:
6-dimethylaminomethyl-1-phenyl-cyclohexane compounds according to the general **formula III** 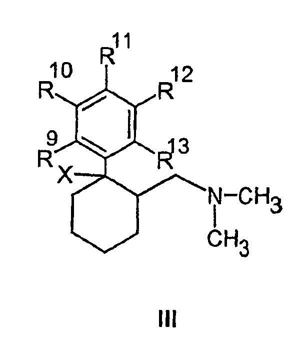
wherein
X is chosen from OH, F, Cl, H or OC(O)R⁷, where R⁷ is chosen from C₁₋₃-alkyl, branched or unbranched, saturated or unsaturated, unsubstituted or mono- or polysubstituted, and
R⁹ to R¹³ in each case independently of one another are chosen from H, F, Cl, Br, I, CH₂F, CHF₂, CF₃, OH, SH, OR¹⁴, OCF₃, SR¹⁴, NR¹⁷R¹⁸, SOCH₃, SOCF₃ ; SO₂CH₃, SO₂CF₃, CN, COOR¹⁴, NO₂, CONR¹⁷R¹⁸; C₁₋₆-alkyl, branched or unbranched, saturated or unsaturated, unsubstituted or mono- or polysubstituted; phenyl, unsubstituted or mono- or polysubstituted;
where R¹⁴ is chosen from C₁₋₆-alkyl; pyridyl, thienyl, thiazolyl, phenyl, benzyl or phenethyl, in each case unsubstituted or mono- or polysubstituted; PO(O-C₁₋₄-alkyl)₂, CO(OC₁₋₅-alkyl), CONH-C₆H₄-(C₁₋₃-alkyl), CO(C₁₋₅-alkyl), CO-CHR¹⁷-NHR¹⁸, CO-C₆H₄-R¹⁵, where R¹⁵ is ortho-OCOC₁₋₃-alkyl or meta- or para-CH₂N(R¹⁶)₂ where R¹⁶ is C₁₋₄-alkyl or 4-morpholino, wherein in the radicals R¹⁴, R¹⁵ and R¹⁶ the alkyl groups can be branched or unbranched, saturated or unsaturated, unsubstituted or mono- or polysubstituted;
where R¹⁷ and R¹⁸ in each case independently of one another are chosen from H; C₁₋₆-alkyl, branched or unbranched, saturated or unsaturated, unsubstituted or mono- or polysubstituted; phenyl, benzyl or phenethyl, in each case unsubstituted or mono- or polysubstituted,
or
R⁹ and R¹⁰ or R¹⁰ and R¹¹ together form an OCH₂O, OCH₂CH₂O, OCH=CH, CH=CHO, CH=C(CH₃)O, OC(CH₃)=CH, (CH₂)₄ or OCH=CHO ring,
with the proviso that if R⁹, R¹¹ and R¹³ correspond to H and one of R¹⁰ or R¹² corresponds to H and the other corresponds to OCH₃, X may not be OH,
optionally in the form of their racemates, their pure stereoisomers, in particular enantiomers or diastereomers, or in the form of mixtures of the stereoisomers, in particular the enantiomers or diastereomers, in any desired mixing ratio; in the form shown or in the form of their acids or their bases or in the form of their salts, in particular the physiologically acceptable salts, or in the form of their solvates, in particular the hydrates;
**and** with at least one of the **compounds B** chosen from:
the anti-muscarine agents: atropine, oxybutinin, propiverine, propantheline, emepronium, trospium, tolterodine, darifenacin and α,α-diphenylacetic acid 4-(N-methylpiperidyl) ester, as well as duloxetine, imipramine and desmopressin,
optionally in the form of their racemates, their pure stereoisomers, in particular enantiomers or diastereomers, or in the form of mixtures of the stereoisomers, in particular the enantiomers or diastereomers, in any desired mixing ratio; in the form of their acids or their bases or in the form of their salts, in particular the physiologically acceptable salts, or in the form of their solvates, in particular the hydrates;
for the preparation of medicament for treatment of an increased urge to urinate or urinary incontinence.

2. Use according to claim 1, **characterized in that** the **compound A** in **group a)** is chosen from:
tramadol, (+)-tramadol, (+)-O-demethyltramadol or (+)-O-demethyl-N-mono-demethyl-tramadol, preferably tramadol or (+)-tramadol, in particular (+)-tramadol.

3. Use according to claim 1, **characterized in that** the **compound A** in **group b)** is chosen from:
• codeine
• dextropropoxyphene
• dihydrocodeine
• diphenoxylate
• ethylmorphine
• meptazinol
• nalbuphine
• pethidine (meperidine)
• tilidine
• viminol
• butorphanol
• dezocine
• nalorphine
• pentazocine
• buprenorphine
preferably
• codeine
• dextropropoxyphene
• dihydrocodeine
• meptazinol
• nalbuphine
• tilidine
• buprenorphine

4. Use according to claim 1, **characterized in that** the **compound A** in **group c)** is chosen from compounds according to **formula I** for which:
X is chosen from
OH, F, Cl, OC(O)CH₃ or H, preferably OH, F, OC(O)CH₃ or H,
**and/or**
R¹ is chosen from
C₁₋₄-alkyl, saturated and unsubstituted, branched or unbranched; preferably CH₃, C₂H₅, C₄H₉ or t-butyl, in particular CH₃ or C₂H₅,
**and/or**
R² and R³ independently of one another are chosen from
H, C₁₋₄-alkyl, saturated and unsubstituted, branched or unbranched; preferably H, CH₃, C₂H₅, i-propyl or t-butyl, in particular H or CH₃, preferably R³ = H,
or
R² and R³ together form a C₅₋₆-cycloalkyl radical, saturated or unsaturated, unsubstituted or mono- or polysubstituted, preferably saturated and unsubstituted, in particular cyclohexyl.
**and/or**
R⁹ to R¹³, where 3 or 4 of the radicals R⁹ to R¹³ must correspond to H, independently of one another are chosen from
H, Cl, F, OH, CF₂H, CF₃ or C₁₋₄-alkyl, saturated and unsubstituted, branched or unbranched; OR¹⁴ or SR¹⁴, where R¹⁴ is chosen from C₁₋₃-alkyl, saturated and unsubstituted, branched or unbranched;
preferably H, Cl, F, OH, CF₂H, CF₃, OCH₃ or SCH₃
or R¹² and R¹¹ form a 3, 4 -OCH=CH ring
in particular
if R^{9,} R¹¹ and R¹³ correspond to H, one of R¹⁰ or R¹² also corresponds to H while the other is chosen from:
Cl, F, OH, CF₂H, CF₃, OR¹⁴ or SR¹⁴, preferably OH, CF₂H, OCH₃ or SCH₃
or
if R⁹ and R¹³ correspond to H and R¹¹ corresponds to OH, OCH₃, Cl or F, preferably Cl, one of R¹⁰ or R¹² also corresponds to H while the other corresponds to OH, OCH₃, Cl or F, preferably Cl,
or
if R⁹, R¹⁰, R¹² and R¹³ correspond to H, R¹¹ is chosen from CF₃, CF₂H, Cl or F, preferably F,
or
if R¹⁰, R¹¹ and R¹² correspond to H, one of R⁹ or R¹³ also corresponds to H while the other is chosen from OH, OC₂H₅ or OC₃H₇.

5. Use according to claim 4, **characterized in that** compounds of the **formula I** where R³ = H are in the form of the diastereomers with the relative configuration la 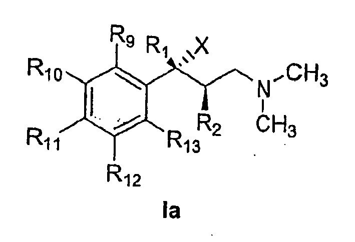 in particular are used in mixtures with a higher content of this diastereomer compared with the other diastereomer or as the pure diastereomer
**and/or**
**in that** the compounds of the **formula I** are used in the form of the (+)-enantiomer, in particular in mixtures with a higher content of the (+)-enantiomer compared with the (-)-enantiomer of a racemic compound or as the pure (+)-enantiomer.

6. Use according to one of claims 4 or 5, **characterized in that compound A** chosen from the following group is used:
■ (2RS,3RS)-1-dimethylamino-3-(3-methoxy-phenyl)-2-methyl-pentan-3-ol
■ (2R,3R)-1-dimethylamino-3-(3-methoxy-phenyl)-2-methyl-pentan-3-ol,
■ (+)-(2R,3R)-1-dimethylamino-3-(3-methoxy-phenyl)-2-methyl-pentan-3-ol,
■ (2RS,3RS)-3-(3,4-dichlorophenyl)-1-dimethylamino-2-methyl-pentan-3-ol,
■ (2RS,3RS)-3-(3-difluoromethyl-phenyl)-1-dimethylamino-2-methyl-pentan-3-ol,
■ (2RS,3RS)-1-dimethylamino-2-methyl-3-(3-methylsulfanyl-phenyl)-pentan-3-ol,
■ (3RS)-1-dimethylamino-3-(3-methoxy-phenyl)-4,4-dimethyl-pentan-3-ol,
■ (2RS,3RS)-3-(3-dimethylamino-1-ethyl-1-hydroxy-2-methyl-propyl)-phenol,
■ (1RS,2RS)-3-(3-dimethylamino-1-hydroxy-1,2-dimethylpropyl)-phenol,
■ (+)-(1R,2R)-3-(3-dimethylamino-1-hydroxy-1,2-dimethyl-propyl)-phenol,
■ (+)-(1R,2R)-3-(3-dimethylamino-1-hydroxy-1,2-dimethyl-propyl)-phenol,
■ (1R,2R)-3-(3-dimethylamino-1-ethyl-2-methyl-propyl)-phenol,
■ (-)-(1R,2R)-3-(3-dimethylamino-1-ethyl-2-methylpropyl)-phenol,
■ (1S, 2S)-3-(3-dimethylamino-1-ethyl-2-methyl-propyl)-phenol,
■ (+)-(1S,2S)-3-(3-dimethylamino-1-ethyl-2-methylpropyl)-phenol,
■ (+)-(1R,2R)-acetic acid 3-dimethylamino-1-ethyl-1-(3-methoxy-phenyl)-2-methyl-propyl ester,
■ (1RS)-1-(1-dimethylaminomethyl-cyclohexyl)-1-(3-methoxy-phenyl)-propan-1-ol,
■ (2RS,3RS)-3-(4-chlorophenyl)-1-dimethylamino-2-methyl-pentan-3-ol,
■ (+)-(2R,3R)-3-(3-dimethylamino-1-ethyl-1-hydroxy-2-methyl-propyl)-phenol,
■ (2RS,3RS)-4-dimethylamino-2-(3-methoxy-phenyl)-3-methyl-butan-2-ol and
■ (+)-(2R,3R)-4-dimethylamino-2-(3-methoxy-phenyl)-3-methyl-butan-2-ol,
preferably as the hydrochloride.

7. Use according to claim 1, **characterized in that** the **compound A** in **group d)** is chosen from compounds according to **formula II** for which:
X is chosen from
OH, F, Cl, OC(O)CH₃ or H, preferably OH, F or H, in particular OH,
**and/or**
R¹ is chosen from
C₁₋₄-alkyl, CF₃, OH, O-C₁₋₄-alkyl, Cl or F, preferably OH, CF₃ or CH₃,
**and/or**
R⁹ to R¹³, where 3 or 4 of the radicals R⁹ to R¹³ must correspond to H, independently of one another are chosen from
H, Cl, F, OH, CF₂H, CF₃ or C₁₋₄-alkyl, saturated and unsubstituted, branched or unbranched; OR¹⁴ or SR¹⁴, where R¹⁴ is chosen from C₁₋₃-alkyl, saturated and unsubstituted, branched or unbranched;
preferably H, Cl, F, OH, CF₂H, CF₃, OCH₃ or SCH₃
or R¹² and R¹¹ form a 3,4-OCH=CH ring
in particular
if R⁹, R¹¹ and R¹³ correspond to H, one of R¹⁰ or R¹² also corresponds to H while the other is chosen from:
Cl, F, OH, CF₂H, CF₃, OR¹⁴ or SR¹⁴, preferably OH, CF₂H, OR¹⁴ or SCH₃, in particular OH or OC₁₋₃-alkyl, preferably OH or OCH₃,
or
if R⁹ and R¹³ correspond to H and R¹¹ corresponds to OH, OCH₃, Cl or F, preferably Cl, one of R¹⁰ or R¹² also corresponds to H while the other corresponds to OH, OCH₃, Cl or F, preferably Cl,
or
if R⁹, R¹⁰ , R¹² and R¹³ correspond to H, R¹¹ is chosen from CF₃, CF₂H, Cl or F, preferably F,
or
if R¹⁰, R¹¹ and R¹² correspond to H, one of R⁹ or R¹³ also corresponds to H while the other is chosen from OH, OC₂H₅ or OC₃H₇.
very particularly preferably
if R⁹, R¹¹ and R¹³ correspond to H, one of R¹⁰ or R¹² also corresponds to H while the other is chosen from:
Cl, F, OH, SH, CF₂H, CF₃, OR¹⁴ or SR¹⁴, preferably OH or OR¹⁴, in particular OH or OC₁₋₃-alkyl, preferably OH or OCH₃.

8. Use according to claim 7, **characterized in that** compounds of the formula II are in the form of the diastereomers with the relative configuration IIa 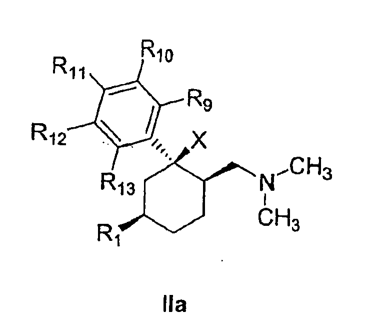 in particular are used in mixtures with a higher content of this diastereomer compared with the other diastereomer or as the pure diastereomer,
**and/or**
**in that** the compounds of the **formula II** are used in the form of the (+)-enantiomer, in particular in mixtures with a higher content of the (+)-enantiomer compared with the (-)-enantiomer of a racemic compound or as the pure (+)-enantiomer.

9. Use according to one of claims 7 or 8, **characterized in that compound A** chosen from the following group is used:
■ (1RS,3RS,6RS)-6-dimethylaminomethyl-1-(3-methoxy-phenyl)-cyclohexane-1,3-diol,
■ (+)-(1R,3R,6R)-6-dimethylaminomethyl-1-(3-methoxy-phenyl)-cyclohexane-1,3-diol,
■ (1RS,3RS,6RS)-6-dimethylaminomethyl-1-(3-hydroxyphenyl)-cyclohexane-1,3-diol,
■ (1RS,3SR,6RS)-6-dimethylaminomethyl-1-(3-methoxyphenyl)-cyclohexane-1,3-diol,
■ (+)-(1R,2R,5S)-3-(2-dimethylaminomethyl-1-hydroxy-5-methyl-cyclohexyl)-phenol or
■ (1RS,2RS,5RS)-3-(2-dimethylaminomethyl-1-hydroxy-5-trifluoromethyl-cyclohexyl)-phenol,
preferably as the hydrochloride.

10. Use according to claim 1, **characterized in that** the **compound A** in **group e)** is chosen from compounds according to **formula III** for which:
X is chosen from
OH, F, Cl, OC(O)CH₃ or H, preferably OH, F or H, in particular F or H,
**and/or**
R⁹ to R¹³, where 3 or 4 of the radicals R⁹ to R¹³ must correspond to H, independently of one another are chosen from
H, Cl, F, OH, CF₂H, CF₃ or C₁₋₄-alkyl, saturated and unsubstituted, branched or unbranched; OR¹⁴ or SR¹⁴, where R¹⁴ is chosen from C₁₋₃-alkyl, saturated and unsubstituted, branched or unbranched;
preferably H, Cl, F, OH, CF₂H, CF₃, OCH₃ or SCH₃
or R¹² and R¹¹ form a 3,4-OCH=CH ring
in particular **characterized in that**
if R⁹, R¹¹ and R¹³ correspond to H, one of R¹⁰ or R¹² also corresponds to H while the other is chosen from:
Cl, F, OH, CF₂H, CF₃, OR¹⁴ or SR¹⁴, preferably OH, CF₂H, OR¹⁴ or SCH₃, in particular OH or OC₁₋₃-alkyl, preferably OH or OCH₃,
or
if R⁹ and R¹³ correspond to H and R¹¹ corresponds to OH, OCH₃, Cl or F, preferably Cl, one of R¹⁰ or R¹² also corresponds to H while the other corresponds to OH, OCH₃, Cl or F, preferably Cl,
or
if R⁹, R¹⁰, R¹² and R¹³ correspond to H, R¹¹ is chosen from CF₃, CF₂H, Cl or F, preferably F,
or
if R¹⁰, R¹¹ and R¹² correspond to H, one of R⁹ or R¹³ also corresponds to H while the other is chosen from OH, OC₂H₅ or OC₃H₇,
very particularly preferably
if R⁹, R¹¹ and R¹³ correspond to H, one of R¹⁰ or R¹² also corresponds to H while the other is chosen from:
Cl, F, OH, SH, CF₂H, CF₃, OR¹⁴, or SR¹⁴, preferably OH or OR¹⁴, in particular OH or OC₁₋₃-alkyl, preferably OH or OCH₃.

11. Use according to claim 10, **characterized in that** compounds of the formula III are in the form of their diastereomers with the relative configuration IIIa 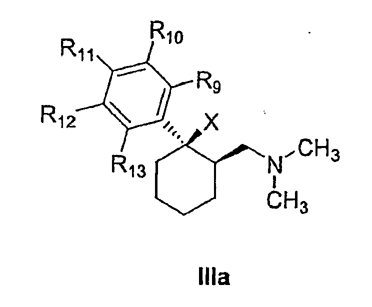 in particular are used in mixtures with a higher content of this diastereomer compared with the other diastereomer or as the pure diastereomer
**and/or**
**in that** the compounds of the **formula III** are used in the form of the (+)-enantiomer, in particular in mixtures with a higher content of the (+)-enantiomer compared with the (-)-enantiomer of a racemic compound or as the pure (+)-enantiomer.

12. Use according to one of claims 10 or 11, **characterized in that compound A** chosen from the following group is used:
■ (+)-(1R,2R)-3-(2-dimethylaminomethyl-1-fluoro-cyclohexyl)-phenol,
■ (+)-(1S,2S)-3-(2-dimethylaminomethyl-cyclohexyl)-phenol or
■ (1S,2S)-3-(2-dimethylaminomethyl-cyclohexyl)-phenol or
■ (-)-(1R,2R)-3-(2-dimethylaminomethyl-cyclohexyl)-phenol,
■ (1R,2R)-3-(2-dimethylaminomethyl-cyclohexyl)-phenol,
■ (-)-(1R,2R)-[2-(3-methoxy-phenyl)-cyclohexylmethyl]-dimethylamine,
■ (1R,2R)-[2-(3-methoxy-phenyl)-cyclohexylmethyl]-dimethylamine,
preferably as the hydrochloride.

13. Use according to one of claims 1 to 12, **characterized in that** the **compound B** is chosen from:
darifenacin, duloxetine, oxybutinin or tolterodine,
preferably is chosen from
duloxetine, oxybutinin or tolterodine,
preferably is chosen from
oxybutinin or tolterodine.

14. Active compound combination of at least one of the **compounds A** and at least one of the **compounds B**, with **compound A** chosen from:
**Group a)** comprising:
tramadol, O-demethyltramadol or O-demethyl-N-mono-demethyl-tramadol, optionally in the form of their racemates, their pure stereoisomers, in particular enantiomers or diastereomers, or in the form of mixtures of the stereoisomers, in particular the enantiomers or diastereomers, in any desired mixing ratio; in the form of their acids or their bases or in the form of their salts, in particular the physiologically acceptable salts, or in the form of their solvates, in particular the hydrates;
**Group b)** comprising:
• codeine
• dextropropoxyphene
• dihydrocodeine
• meptazinol
• nalbuphine
• tilidine
• buprenorphine
optionally in the form of their racemates, their pure stereoisomers, in particular enantiomers or diastereomers, or in the form of mixtures of the stereoisomers, in particular the enantiomers or diastereomers, in any desired mixing ratio; in the form of their acids or their bases or in the form of their salts, in particular the physiologically acceptable salts, or in the form of their solvates, in particular the hydrates;
**Group c)** comprising:
1-phenyl-3-dimethylamino-propane compounds according to the general **formula I** 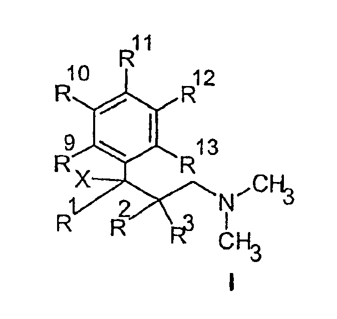
wherein
X is chosen from OH, F, Cl, H or OC (O) R⁷, where R⁷ is chosen from C₁₋₃-alkyl, branched or unbranched, saturated or unsaturated, unsubstituted or mono- or polysubstituted,
R¹ is chosen from C₁₋₄-alkyl, branched or unbranched, saturated or unsaturated, unsubstituted or mono- or polysubstituted,
R² and R³ in each case independently of one another are chosen from H or C₁₋₄-alkyl, branched or unbranched, saturated or unsaturated, unsubstituted or mono- or polysubstituted,
or
R² and R³ together form a saturated C₄₋₇-cycloalkyl radical, unsubstituted or mono- or polysubstituted,
R⁹ to R¹³ in each case independently of one another are chosen from H, F, Cl, Br, I, CH₂F, CHF₂, CF₃, OH, SH, OR¹⁴, OCF₃, SR¹⁴, NR¹⁷R¹⁸, SOCH₃, SOCF₃; SO₂CH₃, SO₂CF₃, CN, COOR¹⁴, NO₂, CONR¹⁷R¹⁸; C₁₋₆-alkyl, branched or unbranched, saturated or unsaturated, unsubstituted or mono- or polysubstituted; phenyl, unsubstituted or mono- or polysubstituted;
where R¹⁴ is chosen from C₁₋₆-alkyl; pyridyl, thienyl, thiazolyl, phenyl, benzyl or phenethyl, in each case unsubstituted or mono- or polysubstituted; PO(O-C₁₋₄-alkyl)₂, CO(OC₁₋₅-alkyl), CONH-C₆H₄-(C₁₋₃-alkyl), CO(C₁₋₅-alkyl), CO-CHR¹⁷-NHR¹⁸, CO-C₆H₄-R¹⁵, where R¹⁵ is ortho-OCOC₁₋₃-alkyl or meta- or para-CH₂N(R¹⁶)₂ where R¹⁶ is C₁₋₄-alkyl or 4-morpholino, wherein in the radicals R¹⁴, R¹⁵ and R¹⁶ the alkyl groups can be branched or unbranched, saturated or unsaturated, unsubstituted or mono- or polysubstituted;
where R¹⁷ and R¹⁸ in each case independently of one another are chosen from H; C₁₋₆-alkyl, branched or unbranched, saturated or unsaturated, unsubstituted or mono- or polysubstituted; phenyl, benzyl or phenethyl, in each case unsubstituted or mono- or polysubstituted,
or
R⁹ and R¹⁰ or R¹⁰ and R¹¹ together form an OCH₂O, OCH₂CH₂O, OCH=CH, CH=CHO, CH=C(CH₃)O, OC(CH₃)=CH, (CH₂)₄ or OCH=CHO ring,
optionally in the form of their racemates, their pure stereoisomers, in particular enantiomers or diastereomers, or in the form of mixtures of the stereoisomers, in particular the enantiomers or diastereomers, in any desired mixing ratio; in the form shown or in the form of their acids or their bases or in the form of their salts, in particular the physiologically acceptable salts, or in the form of their solvates, in particular the hydrates;
**Group d)** comprising:
substituted 6-dimethylaminomethyl-1-phenylcyclohexane compounds according to the general **formula II** 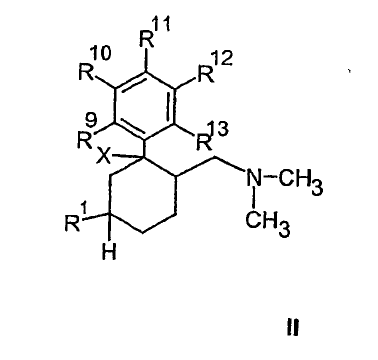
wherein
X is chosen from OH, F, Cl, H or OC(O)R⁷, where R⁷ is chosen from C₁₋₃-alkyl, branched or unbranched, saturated or unsaturated, unsubstituted or mono- or polysubstituted,
R¹ is chosen from C₁₋₄-alkyl, benzyl, CF₃, OH, OCH₂-C₆H₅, O-C₁₋₄-alkyl, Cl or F and
R⁹ to R¹³ in each case independently of one another are chosen from H, F, Cl, Br, I, CH₂F, CHF₂, CF₃, OH, SH, OR¹⁴ , OCF₃, SR¹⁴ , NR¹⁷R¹⁸, SOCH₃, SOCF₃; SO₂CH₃, SO₂CF₃, CN, COOR¹⁴, NO₂, CONR¹⁷R¹⁸; C₁₋₆-alkyl, branched or unbranched, saturated or unsaturated, unsubstituted or mono- or polysubstituted; phenyl, unsubstituted or mono- or polysubstituted;
where R¹⁴ is chosen from C₁₋₆-alkyl; pyridyl, thienyl, thiazolyl, phenyl, benzyl or phenethyl, in each case unsubstituted or mono- or polysubstituted; PO(O-C₁₋₄-alkyl)₂, CO(OC₁₋₅-alkyl), CONH-C₆H₄-(C₁₋₃-alkyl), CO(C₁₋₅-alkyl), CO-CHR¹⁷-NHR¹⁸, CO-C₆H₄-R¹⁵, where R¹⁵ is ortho-OCOC₁₋₃-alkyl or meta- or para-CH₂N(R¹⁶)₂ where R¹⁶ is C₁₋₄-alkyl or 4-morpholino, wherein in the radicals R¹⁴, R¹⁵ and R¹⁶ the alkyl groups can be branched or unbranched, saturated or unsaturated, unsubstituted or mono- or polysubstituted;
where R¹⁷ and R¹⁸ in each case independently of one another are chosen from H; C₁₋₆-alkyl, branched or unbranched, saturated or unsaturated, unsubstituted or mono- or polysubstituted; phenyl, benzyl or phenethyl, in each case unsubstituted or mono- or polysubstituted,
or
R⁹ and R¹⁰ or R¹⁰ and R¹¹ together form an OCH₂O, OCH₂CH₂O, OCH=CH, CH=CHO, CH=C(CH₃)O, OC(CH₃)=CH, (CH₂)₄ or OCH=CHO ring,
optionally in the form of their racemates, their pure stereoisomers, in particular enantiomers or diastereomers, or in the form of mixtures of the stereoisomers, in particular the enantiomers or diastereomers, in any desired mixing ratio; in the form shown or in the form of their acids or their bases or in the form of their salts, in particular the physiologically acceptable salts, or in the form of their solvates, in particular the hydrates;
**and/or**
**Group e)** comprising:
6-dimethylaminomethyl-1-phenyl-cyclohexane compounds according to the general **formula III** 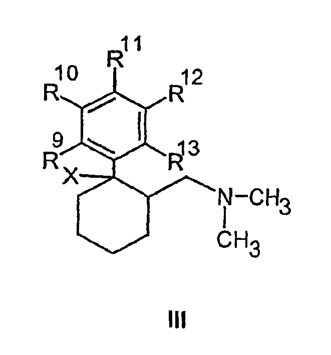
wherein
X is chosen from OH, F, Cl, H or OC(O)R⁷, where R⁷ is chosen from C₁₋₃-alkyl, branched or unbranched, saturated or unsaturated, unsubstituted or mono- or polysubstituted, and
R⁹ to R¹³ in each case independently of one another are chosen from H, F, Cl, Br, I, CH₂F, CHF₂, CF₃, OH, SH, OR¹⁴ , OCF₃, SR¹⁴ , NR¹⁷R¹⁸, SOCH₃, SOCF₃; SO₂CH₃, SO₂CF₃, CN, COOR¹⁴ , NO₂, CONR¹⁷R¹⁸; C₁₋₆-alkyl, branched or unbranched, saturated or unsaturated, unsubstituted or mono- or polysubstituted; phenyl, unsubstituted or mono- or polysubstituted;
where R¹⁴ is chosen from C₁₋₆-alkyl; pyridyl, thienyl, thiazolyl, phenyl, benzyl or phenethyl, in each case unsubstituted or mono- or polysubstituted; PO(O-C₁₋₄-alkyl)₂, CO(OC₁₋₅-alkyl), CONH-C₆H₄-(C₁₋₃-alkyl), CO(C₁₋₅-alkyl), CO-CHR¹⁷-NHR¹⁸, CO-C₆H₄-R¹⁵, where R¹⁵ is ortho-OCOC₁₋₃-alkyl or meta- or para-CH₂N(R¹⁶)₂ where R¹⁶ is C₁₋₄-alkyl or 4-morpholino, wherein in the radicals R¹⁴, R¹⁵ and R¹⁶ the alkyl groups can be branched or unbranched, saturated or unsaturated, unsubstituted or mono- or polysubstituted;
where R¹⁷ and R¹⁸ in each case independently of one another are chosen from H; C₁₋₆-alkyl, branched or unbranched, saturated or unsaturated, unsubstituted or mono- or polysubstituted; phenyl, benzyl or phenethyl, in each case unsubstituted or mono- or polysubstituted,
or
R⁹ and R¹⁰ or R¹⁰ and R¹¹ together form an OCH₂O, OCH₂CH₂O, OCH=CH, CH=CHO, CH=C(CH₃)O, OC(CH₃)=CH, (CH₂)₄ or OCH=CHO ring,
with the proviso that if R⁹, R¹¹ and R¹³ correspond to H and one of R¹⁰ or R¹² corresponds to H and the other corresponds to OCH₃, X may not be OH,
optionally in the form of their racemates, their pure stereoisomers, in particular enantiomers or diastereomers, or in the form of mixtures of the stereoisomers, in particular the enantiomers or diastereomers, in any desired mixing ratio; in the form shown or in the form of their acids or their bases or in the form of their salts, in particular the physiologically acceptable salts, or in the form of their solvates, in particular the hydrates;
and with at least one of the **compounds B** chosen from:
the anti-muscarine agents: atropine, oxybutinin, propiverine, propantheline, emepronium, trospium, tolterodine, darifenacin and α,α-diphenylacetic acid 4-(N-methylpiperidyl) ester, as well as duloxetine, imipramine and desmopressin,
optionally in the form of their racemates, their pure stereoisomers, in particular enantiomers or diastereomers, or in the form of mixtures of the stereoisomers, in particular the enantiomers or diastereomers, in any desired mixing ratio; in the form of their acids or their bases or in the form of their salts, in particular the physiologically acceptable salts, or in the form of their solvates, in particular the hydrates.

15. Active compound combination according to claim 14, **characterized in that** the compound A in **group a)** is chosen from:
tramadol, (+)-tramadol, (+)-O-demethyltramadol or (+)-O-demethyl-N-mono-demethyl-tramadol, preferably tramadol or (+)-tramadol, in particular (+)-tramadol.

16. Active compound combination according to claim 14, **characterized in that** the **compound A** in **group c)** is chosen from compounds according to **formula I** for which:
X is chosen from
OH, F, Cl, OC(O)CH₃ or H, preferably OH, F, OC (O) CH₃ or H,
**and/or**
R¹ is chosen from
C₁₋₄-alkyl, saturated and unsubstituted, branched or unbranched; preferably CH₃, C₂H₅, C₄H₉ or t-butyl, in particular CH₃ or C₂H₅,
**and/or**
R² and R³ independently of one another are chosen from
H, C₁₋₄-alkyl, saturated and unsubstituted, branched or unbranched; preferably H, CH₃, C₂H₅, i-propyl or t-butyl, in particular H or CH₃, preferably R³ = H,
or
R² and R³ together form a C₅₋₆-cycloalkyl radical, saturated or unsaturated, unsubstituted or mono- or polysubstituted, preferably saturated and unsubstituted, in particular cyclohexyl.
**and/or**
R⁹ to R¹³, where 3 or 4 of the radicals R⁹ to R¹³ must correspond to H, independently of one another are chosen from
H, Cl, F, OH, CF₂H, CF₃ or C₁₋₄-alkyl, saturated and unsubstituted, branched or unbranched; OR¹⁴ or SR¹⁴, where R¹⁴ is chosen from C₁₋₃-alkyl, saturated and unsubstituted, branched or unbranched;
preferably H, Cl, F, OH, CF₂H, CF₃, OCH₃ or SCH₃
or R¹² and R¹¹ form a 3,4-OCH=CH ring
in particular
if R⁹, R¹¹ and R¹³ correspond to H, one of R¹⁰ or R¹² also corresponds to H while the other is chosen from:
Cl, F, OH, CF₂H, CF₃, OR¹⁴ or SR¹⁴, preferably OH, CF₂H, OCH₃ or SCH₃
or
if R⁹ and R¹³ correspond to H and R¹¹ corresponds to OH, OCH₃, Cl or F, preferably Cl, one of R¹⁰ or R¹² also corresponds to H while the other corresponds to OH, OCH₃, Cl or F, preferably Cl,
or
if R⁹, R¹⁰, R¹² and R¹³ correspond to H, R¹¹ is chosen from CF₃, CF₂H, Cl or F, preferably F,
or
if R¹⁰, R¹¹ and R¹² correspond to H, one of R⁹ or R¹³ also corresponds to H while the other is chosen from OH, OC₂H₅ or OC₃H₇.

17. Active compound combination according to claim 16, **characterized in that** the compounds of the **formula I** where R³ = H are in the form of the diastereomers with the relative configuration 1a 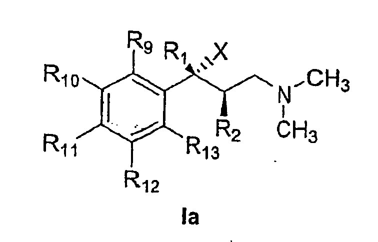 in particular in mixtures with a higher content of this diastereomer compared with the other diastereomer or as the pure diastereomer
**and/or**
**in that** the compounds of the **formula I** are in the form of the (+)-enantiomer, in particular in mixtures with a higher content of the (+)-enantiomer compared with the (-)-enantiomer of a racemic compound or as the pure (+)-enantiomer.

18. Active compound combination according to one of claims 16 or 17, **characterized in that** the **compound A** is chosen from the following group:
■ (2RS,3RS)-1-dimethylamino-3-(3-methoxy-phenyl)-2-methyl-pentan-3-ol
■ (2R,3R)-1-dimethylamino-3-(3-methoxy-phenyl)-2-methyl-pentan-3-ol,
■ (+)-(2R,3R)-1-dimethylamino-3-(3-methoxy-phenyl)-2-methyl-pentan-3-ol,
■ (2RS, 3RS)-3-(3,4-dichlorophenyl)-1-dimethylamino-2-methyl-pentan-3-ol,
■ (2RS, 3RS)-3-(3-difluoromethyl-phenyl)-1-dimethylamino-2-methyl-pentan-3-ol,
■ (2RS,3RS)-1-dimethylamino-2-methyl-3-(3-methylsulfanyl-phenyl)-pentan-3-ol,
■ (3RS)-1-dimethylamino-3-(3-methoxy-phenyl)-4,4-dimethyl-pentan-3-ol,
■ (2RS,3RS)-3-(3-dimethylamino-1-ethyl-1-hydroxy-2-methyl-propyl)-phenol,
■ (1RS,2RS)-3-(3-dimethylamino-1-hydroxy-1,2-dimethylpropyl)-phenol,
■ (+)-(1R,2R)-3-(3-dimethylamino-1-hydroxy-1,2-dimethyl-propyl)-phenol,
■ (+)-(1R,2R)-3-(3-dimethylamino-1-hydroxy-1,2-dimethyl-propyl)-phenol,
■ (1R,2R)-3-(3-dimethylamino-1-ethyl-2-methyl-propyl)-phenol,
■ (-)-(1R,2R)-3-(3-dimethylamino-1-ethyl-2-methylpropyl)-phenol,
■ (1S,2S)-3-(3-dimethylamino-1-ethyl-2-methyl-propyl)-phenol,
■ (+)-(1S,2S)-3-(3-dimethylamino-1-ethyl-2-methylpropyl)-phenol,
■ (+)-(1R,2R)-acetic acid 3-dimethylamino-1-ethyl-1-(3-methoxy-phenyl)-2-methyl-propyl ester,
■ (1RS)-1-(1-dimethylaminomethyl-cyclohexyl)-1-(3-methoxy-phenyl)-propan-1-ol,
■ (2RS,3RS)-3-(4-chlorophenyl)-1-dimethylamino-2-methyl-pentan-3-ol,
■ (+)-(2R,3R)-3-(3-dimethylamino-1-ethyl-1-hydroxy-2-methyl-propyl)-phenol,
■ (2RS,3RS)-4-dimethylamino-2-(3-methoxy-phenyl)-3-methyl-butan-2-ol and
■ (+)-(2R,3R)-4-dimethylamino-2-(3-methoxy-phenyl)-3-methyl-butan-2-ol,
preferably as the hydrochloride.

19. Active compound combination according to claim 14, **characterized in that** the **compound A** in **group d)** is chosen from compounds according to **formula II** for which:
X is chosen from
OH, F, Cl, OC(O)CH₃ or H, preferably OH, F or H, in particular OH,
**and/or**
R¹ is chosen from
C₁₋₄-alkyl, CF₃, OH, O-C₁₋₄-alkyl, Cl or F, preferably OH, CF₃ or CH₃,
**and/or**
R⁹ to R¹³, where 3 or 4 of the radicals R⁹ to R¹³ must correspond to H, independently of one another are chosen from
H, Cl, F, OH, CF₂H, CF₃ or C₁₋₄-alkyl, saturated and unsubstituted, branched or unbranched; OR¹⁴ or SR¹⁴, where R¹⁴ is chosen from C₁₋₃-alkyl, saturated and unsubstituted, branched or unbranched;
preferably H, Cl, F, OH, CF₂H, CF₃, OCH₃ or SCH₃
or R¹² and R¹¹ form a 3,4-OCH=CH ring
in particular
if R⁹, R¹¹ and R¹³ correspond to H, one of R¹⁰ or R¹² also corresponds to H while the other is chosen from:
Cl, F, OH, CF₂H, CF₃, OR¹⁴ or SR¹⁴, preferably OH, CF₂H, OR¹⁴ or SCH₃, in particular OH or OC₁₋₃-alkyl, preferably OH or OCH₃,
or
if R⁹ and R¹³ correspond to H and R¹¹ corresponds to OH, OCH₃, Cl or F, preferably Cl, one of R¹⁰ or R¹² also corresponds to H while the other corresponds to OH, OCH₃, Cl or F, preferably Cl,
or
if R⁹, R¹⁰, R¹² and R¹³ correspond to H, R¹¹ is chosen from CF₃, CF₂H, Cl or F, preferably F,
or
if R¹⁰, R¹¹ and R¹² correspond to H, one of R⁹ or R¹³ also corresponds to H while the other is chosen from OH, OC₂H₅ or OC₃H₇.
very particularly preferably
if R^{9,} R¹¹ and R¹³ correspond to H, one of R¹⁰ or R¹² also corresponds to H while the other is chosen from:
Cl, F, OH, SH, CF₂H, CF₃, OR¹⁴ or SR¹⁴, preferably OH or OR¹⁴, in particular OH or OC₁₋₃-alkyl, preferably OH or OCH₃.

20. Active compound combination according to claim 19, **characterized in that** the compounds of the **formula II** are in the form of the diastereomers with the relative configuration IIa 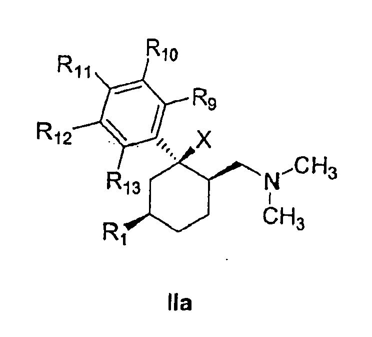 in particular in mixtures with a higher content of this diastereomer compared with the other diastereomer or as the pure diastereomer,
**and/or**
**in that** the compounds of the **formula II** are in the form of the (+)-enantiomer, in particular in mixtures with a higher content of the (+)-enantiomer compared with the (-)-enantiomer of a racemic compound or as the pure (+)-enantiomer.

21. Active compound combination according to one of claims 19 or 20, **characterized in that compound A** is chosen from the following group:
■ (1RS,3RS,6RS)-6-dimethylaminomethyl-1-(3-methoxy-phenyl)-cyclohexane-1,3-diol,
■ (+)-(1R,3R,6R)-6-dimethylaminomethyl-1-(3-methoxy-phenyl)-cyclohexane-1,3-diol,
■ (1RS,3RS,6RS)-6-dimethylaminomethyl-1-(3-hydroxyphenyl)-cyclohexane-1,3-diol,
■ (1RS,3SR,6RS)-6-dimethylaminomethyl-1-(3-methoxy-phenyl)-cyclohexane-1,3-diol,
■ (+)-(1R,2R,5S)-3-(2-dimethylaminomethyl-1-hydroxy-5-methyl-cyclohexyl)-phenol or
■ (1RS,2RS,5RS)-3-(2-dimethylaminomethyl-1-hydroxy-5-trifluoromethyl-cyclohexyl)-phenol,
preferably as the hydrochloride.

22. Active compound combination according to claim 14, **characterized in that** the **compound A** in **group e)** is chosen from compounds according to **formula III** for which:
X is chosen from
OH, F, Cl, OC(O)CH₃ or H, preferably OH, F or H, in particular F or H,
**and/or**
R⁹ to R¹³, where 3 or 4 of the radicals R⁹ to R¹³ must correspond to H, independently of one another are chosen from
H, Cl, F, OH, CF₂H, CF₃ or C₁₋₄-alkyl, saturated and unsubstituted, branched or unbranched; OR¹⁴ or SR¹⁴, where R¹⁴ is chosen from C₁₋₃-alkyl, saturated and unsubstituted, branched or unbranched;
preferably H, Cl, F, OH, CF₂H, CF₃, OCH₃ or SCH₃
or R¹² and R¹¹ form a 3,4-OCH=CH ring
in particular **characterized in that**
if R⁹, R¹¹ and R¹³ correspond to H, one of R¹⁰ or R¹² also corresponds to H while the other is chosen from:
Cl, F, OH, CF₂H, CF₃, OR¹⁴ or SR¹⁴, preferably OH, CF₂H, OR¹⁴ or SCH₃, in particular OH or OC₁₋₃-alkyl, preferably OH or OCH₃,
or
if R⁹ and R¹³ correspond to H and R¹¹ corresponds to OH, OCH₃, Cl or F, preferably Cl, one of R¹⁰ or R¹² also corresponds to H while the other corresponds to OH, OCH₃, Cl or F, preferably Cl,
or
if R⁹, R¹⁰, R¹² and R¹³ correspond to H, R¹¹ is chosen from CF₃, CF₂H, Cl or F, preferably F,
or
if R¹⁰, R¹¹ and R¹² correspond to H, one of R⁹ or R¹³ also corresponds to H while the other is chosen from OH, OC₂H₅ or OC₃H₇,
very particularly preferably
if R^{9,} R¹¹ and R¹³ correspond to H, one of R¹⁰ or R¹² also corresponds to H while the other is chosen from:
Cl, F, OH, SH, CF₂H, CF₃, OR¹⁴, or SR¹⁴, preferably OH or OR¹⁴, in particular OH or OC₁₋₃-alkyl, preferably OH or OCH₃.

23. Active compound combination according to claim 22, **characterized in that** the compounds of the **formula III** are in the form of their diastereomers with the relative configuration IIIa 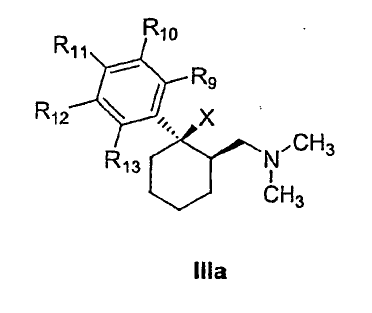 in particular in mixtures with a higher content of this diastereomer compared with the other diastereomer or as the pure diastereomer
**and/or**
**in that** the compounds of the **formula III** are in the form of the (+)-enantiomer, in particular in mixtures with a higher content of the (+)-enantiomer compared with the (-)-enantiomer of a racemic compound or as the pure (+)-enantiomer.

24. Active compound combination according to one of claims 22 or 23, **characterized in that** the **compound A** is chosen from the following group:
■ (+)-(1R,2R)-3-(2-dimethylaminomethyl-1-fluoro-cyclohexyl)-phenol,
■ (+)-(1S,2S)-3-(2-dimethylaminomethyl-cyclohexyl)-phenol or
■ (1S,2S)-3-(2-dimethylaminomethyl-cyclohexyl)-phenol or
■ (-)-(1R,2R)-3-(2-dimethylaminomethyl-cyclohexyl)-phenol,
■ (1R,2R)-3-(2-dimethylaminomethyl-cyclohexyl)-phenol,
■ (-)-(1R,2R)-[2-(3-methoxy-phenyl)-cyclohexylmethyl]-dimethylamine,
■ (1R,2R)-[2-(3-methoxy-phenyl)-cyclohexylmethyl]-dimethylamine,
preferably as the hydrochloride.

25. Active compound combination according to one of claims 14 to 24, **characterized in that** the compound B is chosen from:
darifenacin, duloxetine, oxybutinin or tolterodine, preferably is chosen from
duloxetine, oxybutinin or tolterodine,
preferably is chosen from
oxybutinin or tolterodine.

26. Medicament, preferably for treatment of an increased urge to urinate or urinary incontinence, comprising an active compound combination according to one of claims 14 to 25 and optionally suitable additives and/or auxiliary substances.

## Revendications

1. Utilisation d'une combinaison de substances actives formée d'au moins l'un des composés A et d'au moins l'un des composés B, le composé A étant choisi dans :
le groupe a) contenant : le tramadol, le O-déméthyltramadol ou le O-desméthyl-N-mono-desméthyl-tramadol, éventuellement sous forme de leurs racémates, de leurs stéréoisomères purs, en particulier des énantiomères ou des diastéréoisomères, ou sous forme de mélanges des stéréoisomères, en particulier des énantiomères ou des diastéréoisomères, dans un rapport de mélange quelconque ; sous forme de leurs acides ou de leurs bases ou sous forme de leurs sels, en particulier des sels physiologiquement acceptables ou sous forme de leurs produits de solvatation, en particulier des hydrates ;
le groupe b) contenant les substances suivantes :
• la codéine,
• le dextropropoxyphène,
• la dihydrocodéine,
• le diphénoxylate,
• l'éthylmorphine,
• le meptazinol,
• la nalbuphine,
• la péthidine (mépéridine),
• la tilidine,
• le tramadol,
• le viminol,
• le butorphanol,
• le dextromoramide,
• la dézocine,
• la diacétylmorphine (héroïne),
• l'hydrocodoné,
• l'hydromorphone,
• la kétobémidone,
• la lévométhadone,
• le lévométhadylacétate (1-α-acétylméthadol) (LAAM),
• le lévorphanol,
• la morphine,
• la nalorphine,
• l'oxycodone,
• la pentazocine,
• le piritramide,
• l'alfentanil,
• la buprénorphine,
• l'étorphine,
• le fentanyl,
• le rémifentanil,
• le sufentanil,
éventuellement sous forme de leurs racémates, de leurs stéréoisomères purs, en particulier des énantiomères ou des diastéréoisomères, ou sous forme de mélanges des stéréoisomères, en particulier des énantiomères ou des diastéréoisomères, dans un rapport de mélange quelconque ; sous forme de leurs acides ou de leurs bases ou sous forme de leurs sels, en particulier des sels physiologiquement acceptables, ou sous forme de leurs produits de solvatation, en particulier des hydrates ;
le groupe c) contenant :
des composés de 1-phényl-3-diméthylamino-propane répondant à la formule générale I : 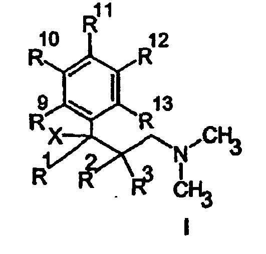
dans laquelle :
X est choisi entre OH, F, Cl, H ou un groupe OC(O)R⁷ dans lequel R⁷ est choisi entre des restes alkyle en C₁ à C₃ ramifiés ou non ramifiés, saturés ou non saturés, non substitués ou substitués une ou plusieurs fois,
R¹ est choisi entre des restes alkyle en C₁ à C₄, ramifiés ou non ramifiés, saturés ou non saturés, non substitués ou substitués une ou plusieurs fois,
R² et R³ sont choisis tous deux, indépendamment l'un de l'autre, entre H ou des restes alkyle en C₁ à C₄ ramifiés ou non ramifiés, saturés ou non saturés, non substitués ou substitués une ou plusieurs fois,
ou bien
R² et R³ forment ensemble un reste cycloalkyle en C₄ à C₇ non substitué ou substitué une ou plusieurs fois,
R⁹ à R¹³ sont chacun choisis, indépendamment les uns des autres, entre H, F, Cl, Br, I, CH₂F, CHF₂, CF₃, OH, SH, OR¹⁴, OCF₃, SR¹⁴, NR¹⁷R¹⁸, SOCH₃, SOCF₃ ; SO₂CH₃, SO₂CF₃, CN, COOR¹⁴, NO₂, CONR¹⁷R¹⁸ ; un reste alkyle en C₁ à C₆, ramifié ou non ramifié, saturé ou non saturé, non substitué ou substitué une ou plusieurs fois ; un reste phényle, non substitué ou substitué une ou plusieurs fois ;
R¹⁴ étant choisi entre un reste alkyle en C₁ à C₆ ; pyridyle, thiényle, thiazolyle, phényle, benzyle ou phénéthyle, chacun non substitué ou substitué une ou plusieurs fois ;
un groupe PO(O-alkyle en C₁ à C₄)₂, CO(O-alkyle en C₁ à C₅), CONH-C₆H₄-(alkyle en C₁ à C₃), CO(alkyle en C₁ à C₅), CO-CHR¹⁷-NHR¹⁸, CO-C₆H₄-R¹⁵, R¹⁵ désignant un groupe ortho-OCO(alkyle en C₁ à C₃) ou méta- ou para-CH₂N(R¹⁶)₂ où R¹⁶ désigne un reste alkyle en C₁ à C₄ ou 4-morpholino, les groupes alkyle dans les restes R¹⁴, R¹⁵ et R¹⁶ pouvant être ramifiés ou non ramifiés, saturés ou non saturés, non substitués ou substitués une ou plusieurs fois ;
R¹⁷ et R¹⁸ étant tous deux choisis, indépendamment l'un de l'autre, entre H ; des restes alkyle en C₁ à C₆, ramifiés ou non ramifiés, saturés ou non saturés, non substitués ou substitués une ou plusieurs fois ; phényle, benzyle ou phénéthyle, chacun non substitué ou substitué une ou plusieurs fois,
ou bien
R⁹ et R¹⁰, ou R¹⁰ et R¹¹, forment conjointement un noyau OCH₂O-, OCH₂CH₂O-, OCH=CH-, CH=CHO-, CH=C(CH₃)O-, OC(CH₃) =CH-, (CH₂)₄- ou OCH=CHO-,
le cas échéant sous forme de leurs racémates, de leurs stéréoisomères purs, en particulier des énantiomères ou des diastéréoisomères, ou sous forme de mélanges des stéréoisomères, en particulier des énantiomères ou des diastéréoisomères, dans un rapport de mélange quelconque ; sous la forme représentée ou sous forme de leurs acides ou de leurs bases ou sous forme de leurs sels, en particulier des sels physiologiquement acceptables, ou sous forme de leurs produits de solvatation, en particulier des hydrates ;
le groupe d) comprenant :
des composés substitués de 6-diméthylaminométhyl-1-phényl-cyclohexane de formule générale II : 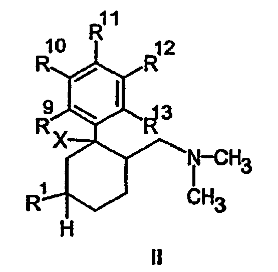
dans laquelle :
X est choisi entre OH, F, Cl, H ou un groupe OC(O)R⁷ dans lequel R⁷ est choisi entre des restes alkyle en C₁ à C₃, ramifiés ou non ramifiés, saturés ou non saturés, non substitués ou substitués une ou plusieurs fois,
R¹ est choisi entre un reste alkyle en C₁ à C₄, benzyle, CF₃, OH, OCH₂-C₆H₅, O-alkyle en C₁ à C₄, C1 ou F, et
R⁹ à R¹³ sont chacun choisis, indépendamment les uns des autres, entre H, F, Cl, Br, I, CH₂F, CHF₂, CF₃, OH, SH, OR¹⁴, OCF₃, SR¹⁴, NR¹⁷R¹⁸, SOCH₃, SOCF₃ ; SO₂CH₃, SO₂CF₃, CN, COOR¹⁴ NO₂, CONR¹⁷R¹⁸ ; des restes alkyle en C₁ à C₆, ramifiés ou non ramifiés, saturés ou non saturés, non substitués ou substitués une ou plusieurs fois ; un reste phényle, non substitué ou substitué une ou plusieurs fois ;
R¹⁴ étant choisi entre des restes alkyle en C₁ à C₆; pyridyle, thiényle, thiazolyle, phényle, benzyle ou phénéthyle, chacun non substitué ou substitué une ou plusieurs fois ;
un groupe PO (O-alkyle en C₁ à C₄)₂, CO(O-alkyle en C₁ à C₅), CONH-C₆H₄-(alkyle en C₁ à C₃), CO(alkyle en C₁ à C₅), CO-CHR¹⁷-NHR¹⁸, CO-C₆H₄-R¹⁵, R¹⁵ désignant un groupe ortho-OCO(alkyle en C₁ à C₃) ou méta- ou para-CH₂N(R¹⁶)₂ où R¹⁶ désigne un reste alkyle en C₁ à C₄ ou 4-morpholino, les groupes alkyle dans les restes R¹⁴, R¹⁵ et R¹⁶ pouvant être ramifiés ou non ramifiés, saturés ou non saturés, non substitués ou substitués une ou plusieurs fois ;
R¹⁷ et R¹⁸ étant tous deux choisis, indépendamment l'un de l'autre, entre H ; des restes alkyle en C₁ à C₆, ramifiés ou non ramifiés, saturés ou non saturés, non substitués ou substitués une ou plusieurs fois ; phényle, benzyle ou phénéthyle, chacun non substitué ou substitué une ou plusieurs fois,
ou bien
R⁹ et R¹⁰, ou R¹⁰ et R¹¹, forment conjointement un noyau OCH₂O-, OCH₂CH₂O-, OCH=CH-, CH=CHO-, CH=C(CH₃)O-, OC (CH₃) =CH-, (CH₂)₄- ou OCH=CHO-,
le cas échéant sous forme de leurs racémates, de leurs stéréoisomères purs, en particulier des énantiomères ou des diastéréoisomères, ou sous forme de mélanges des stéréoisomères, en particulier des énantiomères ou des diastéréoisomères, dans un rapport de mélange quelconque ; sous la forme représentée ou sous forme de leurs acides ou de leurs bases ou sous forme de leurs sels, en particulier des sels physiologiquement acceptables, ou sous forme de leurs produits de solvatation, en particulier des hydrates ; et/ou
le groupe e) comprenant :
des composés de 6-diméthylaminométhyl-1-phényl-cyclohexane de formule générale III : 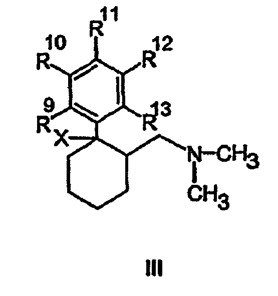
dans laquelle :
X est choisi entre OH, F, Cl, H ou un groupe OC(O)R⁷ dans lequel R⁷ est choisi entre des restes alkyle en
C₁ à C₃, ramifiés ou non ramifiés, saturés ou non saturés, non substitués ou substitués une ou plusieurs fois, et
R⁹ à R¹³ sont chacun choisis, indépendamment les uns des autres, entre H, F, Cl, Br, I, CH₂F, CHF₂, CF₃, OH, SH, OR¹⁴, OCF₃, SR¹⁴, NR¹⁷R¹⁸, SOCH₃, SOCF₃ ; SO₂CH₃, SO₂CF₃, CN, COOR¹⁴, NO₂, CONR¹⁷R¹⁸ ; des restes alkyle en C₁ à C₆, ramifiés ou non ramifiés, saturés ou non saturés, non substitués ou substitués une ou plusieurs fois ; un reste phényle, non substitué ou substitué une ou plusieurs fois ;
R¹⁴ étant choisi entre des restes alkyle en C₁ à C₆ ; pyridyle, thiényle, thiazolyle, phényle, benzyle ou phénéthyle, chacun non substitué ou substitué une ou plusieurs fois ;
un groupe PO(O-alkyle en C₁ à C₄)₂, CO(O-alkyle en C₁ à C₅), CONH-C₆H₄-(alkyle en C₁ à C₃), CO(alkyle en C₁ à C₅), CO-CHR¹⁷-NHR¹⁸, CO-C₆H₄-R¹⁵, R¹⁵ désignant un groupe ortho-OCO(alkyle en C₁ à C₃)
ou méta- ou para-CH₂N(R¹⁶)₂ où R¹⁶ désigne un reste alkyle en C₁ à C₄ ou 4-morpholino, les groupes alkyle dans les restes R¹⁴, R¹⁵ et R¹⁶ pouvant être ramifiés ou non ramifiés, saturés ou non saturés, non substitués ou substitués une ou plusieurs fois ;
R¹⁷ et R¹⁸ étant tous deux choisis, indépendamment l'un de l'autre, entre H ; des restes alkyle en C₁ à C₆, ramifiés ou non ramifiés, saturés ou non saturés, non substitués ou substitués une ou plusieurs fois ; un reste phényle, benzyle ou phénéthyle, chacun non substitué ou substitué une ou plusieurs fois,
ou bien
R⁹ et R¹⁰, ou R¹⁰ et R¹¹, forment conjointement un noyau OCH₂O-, OCH₂CH₂O-, OCH=CH-, CH=CHO-, CH=C(CH₃)O-, OC(CH₃)=CH-, (CH₂)₄- ou OCH=CHO-,
sous réserve que lorsque R⁹, R¹¹ et R¹³ correspondent à H et que l'un de R¹⁰ ou R¹² représente H et l'autre représente un groupe OCH₃, X ne puisse pas représenter OH,
le cas échéant sous forme de leurs racémates, de leurs stéréoisomères purs, en particulier des énantiomères ou des diastéréoisomères, ou sous forme de mélanges des stéréoisomères, en particulier des énantiomères ou des diastéréoisomères, dans un rapport de mélange quelconque ; sous la forme représentée ou sous forme de leurs acides ou de leurs bases ou sous forme de leurs sels, en particulier des sels physiologiquement acceptables, ou sous forme de leurs produits de solvatation, en particulier des hydrates ;
et avec au moins l'un des composés B, choisis parmi :
les antimuscariniques : atropine, oxybutynine, propivérine, propanthéline, émépronium, trospium, toltérodine, darifénacine et l'ester de 4-(N-méthyl-pipéridyle) de l'acide α,α-diphénylacétique, ainsi que la duloxétine, l'imipramine et la desmopressine,
le cas échéant sous forme de leurs racémates, de leurs stéréoisomères purs, en particulier des énantiomères ou des diastéréoisomères, ou sous forme de mélanges des stéréoisomères, en particulier des énantiomères ou des diastéréoisomères, dans un rapport de mélange quelconque ; sous forme de leurs acides ou de leurs bases ou sous forme de leurs sels, en particulier des sels physiologiquement acceptables, ou sous forme de leurs produits de solvatation, en particulier des hydrates ;
pour la préparation d'un médicament destiné au traitement de la répétition du besoin impérieux d'uriner, ou de l'incontinence d'urine.

2. Utilisation suivant la revendication 1, **caractérisée en ce que** le composé A du groupe a) est choisi entre le tramadol, le (+)-tramadol, le (+)-O-déméthyltramadol ou le (+)-O-desméthyl-N-mono-desméthyl-tramadol, avantageusement le tramadol ou le (+)-tramadol, en particulier le (+)-tramadol.

3. Utilisation suivant la revendication 1, **caractérisée en ce que** le composé A du groupe b) est choisi entre:
• la codéine,
• le dextropropoxyphène,
• la dihydrocodéine,
• le diphénoxylate,
• l'éthylmorphine,
• le meptazinol,
• la nalbuphine,
• la péthidine (mépéridine),
• la tilidine,
• le viminol,
• le butorphanol,
• la dézocine,
• la nalorphine,
• la pentazocine,
• la buprénorphine,
avantageusement
• la codéine,
• le dextropropoxyphène,
• la dihydrocodéine,
• le meptazinol,
• la nalbuphine,
• la tilidine,
• la buprénorphine.

4. Utilisation suivant la revendication 1, **caractérisée en ce que** le composé A du groupe c) est choisi parmi des composés de formule I dans laquelle :
X est choisi entre OH, F, Cl, OC(O)CH₃ ou H, avantageusement OH, F, OC(O)CH₃ ou H,
et/ou
R¹ est choisi entre :
des restes alkyle en C₁ à C₄, saturés et non substitués, ramifiés ou non ramifiés ; avantageusement CH₃, C₂H₅, C₄H₉ ou tertiobutyle, en particulier CH₃ ou C₂H₅,
et/ou
R² et R³ sont choisis indépendamment l'un de l'autre entre :
H, des restes alkyle en C₁ à C₄, saturés et non substitués, ramifiés ou non ramifiés ; avantageusement H, CH₃, C₂H₅, isopropyle ou tertiobutyle, en particulier H ou CH₃, R³ représentant préférentiellement H,
ou bien
R² et R³ forment ensemble un reste cycloalkyle en C₅ ou C₆, saturé ou non saturé, non substitué ou substitué une ou plusieurs fois, avantageusement saturé et non substitué, en particulier cyclohexyle,
et/ou
R⁹ à R¹³, dont 3 ou 4 des restes R⁹ à R¹³ doivent représenter H, sont choisis indépendamment les uns des autres entre :
H, Cl, F, OH, CF₂H, CF₃ ou des restes alkyle en C₁ à C₄ saturés et non substitués, ramifiés ou non ramifiés ; OR¹⁴ ou SR¹⁴, R¹⁴ étant choisi entre des restes alkyle en C₁ à C₃ saturés et non substitués, ramifiés ou non ramifiés ;
avantageusement H, Cl, F, OH, CF₂H, CF₃, OCH₃ ou SCH₃,
ou bien R¹² et R¹¹ forment un noyau 3,4-OCH=CH,
en particulier
lorsque R⁹, R¹¹ et R¹³ représentent H, l'un de R¹⁰ ou R¹² représente aussi H, tandis que l'autre est choisi entre :
Cl, F, OH, CF₂H, CF₃, OR¹⁴ ou SR¹⁴, avantageusement OH, CF₂H, OCH₃ ou SCH₃,
ou bien
lorsque R⁹ et R¹³ représentent H et R¹¹ représente OH, OCH₃, Cl ou F, avantageusement Cl, l'un de R¹⁰ ou R¹² représente aussi H, tandis que l'autre représente OH, OCH₃, Cl ou F, avantageusement Cl,
ou bien
lorsque R⁹ R¹⁰, R¹² et R¹³ représentent H, R¹¹ est choisi entre CF₃, CF₂H, Cl ou F, avantageusement F,
ou bien
lorsque R¹⁰, R¹¹ et R¹² représentent H, l'un de R⁹ ou R¹³ représente aussi H, tandis que l'autre est choisi entre OH, OCH₂H₅ ou OC₃H₇.

5. Utilisation suivant la revendication 4, **caractérisée en ce que** des composés de formule I dans laquelle R³ représente H se présentant sous forme des diastéréoisomères ayant la configuration relative la : 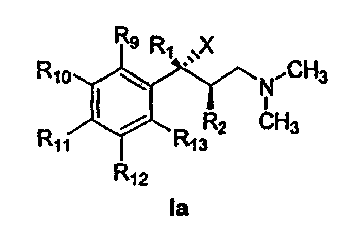 sont utilisés, en particulier dans des mélanges contenant une plus forte proportion de ce diastéréoisomère comparativement à l'autre diastéréoisomère, ou sous forme de diastéréoisomère pur,
et/ou **en ce que** :
les composés de formule I sont utilisés sous forme du (+)-énantiomère, en particulier dans des mélanges contenant une plus forte proportion du (+)-énantiomère comparativement au (-)-énantiomère d'un composé racémique, ou sous forme du (+)-énantiomère pur.

6. Utilisation suivant l'une des revendications 4 ou 5, **caractérisée en ce que** le composé A est choisi dans le groupe suivant :
■ (2RS,3RS)-1-diméthylamino-3-(3-méthoxy-phényl)-2-méthyl-pentane-3-ol,
■ (2R,3R)-1-diméthylamino-3-(3-méthoxy-phényl)-2-méthyl-pentane-3-ol,
■ (+)-(2R,3R)-1-diméthylamino-3-(3-méthoxy-phényl)-2-méthyl-pentane-3-ol,
■ (2RS,3RS)-3-(3,4-dichlorophényl)-1-diméthylamino-2-méthyl-pentane-3-ol,
■ (2RS,3RS)-3-(3-difluorométhyl-phényl)-1-diméthylamino-2-méthyl-pentane-3-ol,
■ (2RS,3RS)-1-diméthylamino-2-méthyl-3-(3-méthylsulfanyl-phényl)-pentane-3-ol,
■ (3RS)-1-diméthylamino-3-(3-méthoxy-phényl)-4,4-diméthyl-pentane-3-ol,
■ (2RS,3RS)-3-(3-diméthylamino-1-éthyl-1-hydroxy-2-méthyl-propyl)-phénol,
■ (1RS,2RS)-3-(3-diméthylamino-1-hydroxy-1,2-diméthyl-propyl)-phénol,
■ (+)-(1R,2R)-3-(3-diméthylamino-1-hydroxy-1,2-diméthyl-propyl)-phénol,
■ (+)-(1R,2R)-3- (3-diméthylamino-1-hydroxy-1,2-diméthyl-propyl)-phénol,
■ (1R,2R)-3-(3-diméthylamino-1-éthyl-2-méthyl-propyl)-phénol,
■ (-)-(1R,2R)-3-(3-diméthylamino-1-éthyl-2-méthyl-propyl)-phénol,
■ (1S,2S)-3-(3-diméthylamino-1-éthyl-2-méthyl-propyl)-phénol,
■ (+)-(1S,2S)-3-(3-diméthylamino-1-éthyl-2-méthyl-propyl)-phénol,
■ ester de (+)-(1R,2R)-3-diméthylamino-1-éthyl-(3-méthoxy-phényl)-2-méthyl-propyle d'acide acétique
■ (1RS)-1-(1-diméthylaminométhyl-cyclohexyl)-1-(3-méthoxy-phényl)-propane-1-ol,
■ (2RS,3RS)-3-(4-chlorophényl)-1-diméthylamino-2-méthyl-pentane-3-ol,
■ (+)-(2R,3R)-3-(3-diméthylamino-1-éthyl-1-hydroxy-2-méthyl-propyl)-phénol,
■ (2RS,3RS)-4-diméthylamino-2-(3-méthoxy-phényl)-3-méthyl-butane-2-ol, et
■ (+)-(2R,3R)-4-diméthylamino-2-(3-méthoxy-phényl)-3-méthyl-butane-2-ol,
sous forme du chlorhydrate.

7. Utilisation suivant la revendication 1, **caractérisée en ce que** le composé A du groupe d) est choisi parmi des composés de formule II dans laquelle :
X est choisi entre :
OH, F, Cl, OC(O)CH₃ ou H, avantageusement OH, F ou H, en particulier OH,
et/ou
R¹ est choisi entre :
alkyle en C₁ à C₄, CF₃, OH, O-alkyle en C₁ à C₄, Cl ou F, avantageusement OH, CF₃ ou CH₃,
et/ou
R⁹ à R¹³, dont 3 ou 4 de ces restes R⁹ à R¹³ doivent représenter H, sont choisis indépendamment les uns des autres entre :
H, Cl, F, OH, CF₂H, CF₃ ou des restes alkyle en C₁ à C₄ saturés et non substitués, ramifiés ou non ramifiés ; OR¹⁴ ou SR¹⁴, R¹⁴ étant choisi entre des restes alkyle en C₁ à C₃ saturés et non substitués, ramifiés ou non ramifiés ;
avantageusement H, Cl, F, OH, CF₂H, CF₃, OCH₃ ou SCH₃,
ou bien R¹² et R¹¹ forment un noyau 3, 4-OCH=CH,
en particulier
lorsque R^{9,} R¹¹ et R¹³ représentent H, l'un de R¹⁰ ou R¹² représente aussi H, tandis que l'autre est choisi entre :
Cl, F, OH, CF₂H, CF₃, OR¹⁴ ou SR¹⁴, avantageusement OH, CF₂H, OR¹⁴ ou SCH₃, en particulier OH ou O-alkyle en C₁ à C₃, avantageusement OH ou OCH₃,
ou bien
lorsque R⁹ et R¹³ représentent H et R¹¹ représente OH, OCH₃, Cl ou F, avantageusement Cl, l'un de R¹⁰ ou R¹² représente aussi H, tandis que l'autre représente OH, OCH₃, Cl ou F, avantageusement Cl,
ou bien
lorsque R⁹, R¹⁰, R¹² et R¹³ représentent H, R¹¹ est choisi entre CF₃, CF₂H, Cl ou F, avantageusement F,
ou bien
lorsque R¹⁰, R¹¹ et R¹² représentent H, l'un de R⁹ ou R¹³ représente aussi H, tandis que l'autre est choisi entre OH, OC₂H₅ ou OC₃H₇,
de façon tout particulièrement appréciée,
lorsque R⁹, R¹¹ et R¹³ représentent H, l'un de R¹⁰ ou R¹² représente aussi H, tandis que l'autre est choisi entre:
Cl, F, OH, SH, CF₂H, CF₃, OR¹⁴ ou SR¹⁴, avantageusement OH ou OR¹⁴, en particulier OH ou O-alkyle en C₁ à C₃, avantageusement OH ou OCH₃.

8. Utilisation suivant la revendication 7, **caractérisée en ce que** des composés de formule II se présentant sous forme des diastéréoisomères ayant la configuration relative IIa : 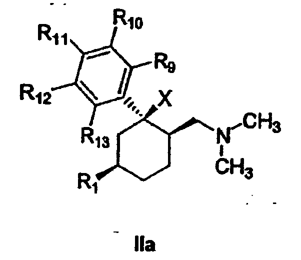 sont utilisés en particulier dans des mélanges contenant une plus forte proportion de ce diastéréoisomère comparativement à l'autre diastéréoisomère, ou sous forme de diastéréoisomère pur,
et/ou **en ce que**
les composés de formule II sont utilisés sous forme du (+)-énantiomère, en particulier dans des mélanges contenant une plus forte proportion du (+)-énantiomère comparativement au (-)-énantiomère d'un composé racémique, ou sous forme du (+)-énantiomère pur.

9. Utilisation suivant l'une des revendications 7 ou 8, **caractérisée en ce que** le composé A est choisi dans le groupe de composés suivant :
■ (1RS,3RS,6RS)-6-diméthylaminométhyl-1-(3-méthoxy-phényl)-cyclohexane-1,3-diol,
■ (+)-(1R,3R,6R)-6-diméthylaminométhyl-1-(3-méthoxy-phényl)-cyclohexane-1,3-diol,
■ (1RS,3RS,6RS)-6-diméthylaminométhyl-1-(3-hydroxy-phényl)-cyclohexane-1,3-diol,
■ (1RS,3SR,6RS)-6-diméthylaminométhyl-1-(3-méthoxy-phényl)-cyclohexane-1,3-diol,
■ (+)-(1R, 2R, 5S)-3-(2-diméthylaminométhyl-1-hydroxy-5-méthyl-cyclohexyl)-phénol ou
■ (1RS,2RS,5RS)-3-(2-diméthylaminométhyl-1-hydroxy-5-trifluorométhyl-cyclohexyl)-phénol,
avantageusement sous forme du chlorhydrate.

10. Utilisation suivant la revendication 1, **caractérisée en ce que** le composé A du groupe e) est choisi parmi des composés de formule III dans laquelle :
X est choisi entre :
OH, F, Cl, OC(O)CH₃ ou H, avantageusement OH, F ou H, en particulier F ou H,
et/ou
R⁹ à R¹³, dont 3 ou 4 de ces restes R⁹ à R¹³ doivent représenter H, sont choisis indépendamment les uns des autres entre :
H, Cl, F, OH, CF₂H, CF₃ ou des groupes alkyle en C₁ à C₄, saturés et non substitués, ramifiés ou non ramifiés ; OR¹⁴ ou SR¹⁴, R¹⁴ étant choisi entre des restes alkyle en C₁ à C₃ saturés et non substitués, ramifiés ou non ramifiés ;
avantageusement H, Cl, F, OH, CF₂H, CF₃, OCH₃ ou SCH₃,
ou bien R¹² et R¹¹ forment un noyau 3,4-OCH=CH,
ayant en particulier pour caractéristique que,
lorsque R⁹, R¹¹ et R¹³ représentent H, l'un de R¹⁰ ou R¹² représente aussi H, tandis que l'autre est choisi entre :
Cl, F, OH, CF₂H, CF₃, OR¹⁴ ou SR¹⁴, avantageusement OH, CF₂H, OR¹⁴ ou SCH₃, en particulier OH ou O-alkyle en C₁ à C₃, avantageusement OH ou OCH₃,
ou bien
lorsque R⁹ et R¹³ représentent H et R¹¹ représente OH, OCH₃, Cl ou F, avantageusement Cl, l'un de R¹⁰ ou R¹² représente aussi H, tandis que l'autre représente OH, OCH₃, Cl ou F, avantageusement Cl,
ou bien
lorsque R⁹, R¹⁰, R¹² et R¹³ représentent H, R¹¹ est choisi entre CF₃, CF₂H, Cl ou F, avantageusement F,
ou bien,
lorsque R¹⁰, R¹¹ et R¹² représentent H, l'un de R⁹ ou R¹³ représente aussi H, tandis que l'autre est choisi entre OH, OC₂H₅ ou OC₃H₇,
de façon tout particulièrement appréciée,
lorsque R⁹, R¹¹ et R¹³ représentent H, l'un de R¹⁰ ou R¹² représente aussi H, tandis que l'autre est choisi entre :
Cl, F, OH, SH, CF₂H, CF₃, OR¹⁴ ou SR¹⁴, avantageusement OH ou OR¹⁴, en particulier OH ou O-alkyle en C₁ à C₃, préférentiellement OH ou OCH₃.

11. Utilisation suivant la revendication 10, **caractérisée en ce que** des composés de formule III se présentant sous forme de leurs diastéréoisomères ayant la configuration relative IIIa 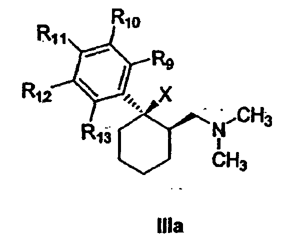 sont utilisés en particulier dans des mélanges contenant une plus forte proportion de ce diastéréoisomère comparativement à l'autre diastéréoisomère, ou sous forme de diastéréoisomère pur,
et/ou **en ce que**
les composés de formule III sont utilisés sous forme du (+)-énantiomère, en particulier dans des mélanges contenant une plus forte proportion du (+)-énantiomère comparativement au (-)-énantiomère d'un composé racémique ou sous forme de (+)-énantiomère pur.

12. Utilisation suivant l'une des revendications 10 ou 11, **caractérisée en ce que** le composé A est choisi dans le groupe de composés suivant :
■ (+)-(1R,2R)-3-(2-diméthylaminométhyl-1-fluoro-cyclohexyl)-phénol,
■ (+)-(1S,2S)-3-(2-diméthylaminométhyl-cyclohexyl)-phénol, ou
■ (1S,2S)-3-(2-diméthylaminométhyl-cyclohexyl)-phénol, ou
■ (-)-(1R,2R)-3-(2-diméthylaminométhyl-cyclohexyl)-phénol,
■ (1R,2R)-3-(2-diméthylaminométhyl-cyclohexyl)-phénol,
■ (-) - (1R,2R) - [2- (3-méthoxy-phényl) -cyclohexylméthyl]-diméthylamine,
■ (1R,2R)-[2-(3-méthoxy-phényl)-cyclohexylméthyl]-diméthylamine,
avantageusement sous forme du chlorhydrate.

13. Utilisation suivant l'une des revendications 1 à 12, **caractérisée en ce que** le composé B est choisi entre :
la darifénacine, la duloxétine, l'oxybutynine ou la toltérodine,
avantageusement choisi entre
la duloxétine, l'oxybutynine ou la toltérodine,
préférentiellement choisi entre
l'oxybutynine ou la toltérodine.

14. Combinaison de substances actives comprenant au moins l'un des composés A et au moins l'un des composés B, le composé A étant choisi dans :
le groupe a) comprenant :
le tramadol, le O-déméthyltramadol ou le O-desméthyl-N-mono-desméthyl-tramadol, éventuellement sous forme de leurs racémates, de leurs stéréoisomères purs, en particulier des énantiomères ou des diastéréoisomères, ou sous forme de mélanges des stéréoisomères, en particulier des énantiomères ou des diastéréoisomères, dans un rapport de mélange quelconque ; sous forme de leurs acides ou de leurs bases ou sous forme de leurs sels, en particulier des sels physiologiquement acceptables, ou sous forme de leurs produits de solvatation, en particulier des hydrates ;
le groupe b) comprenant :
• la codéine,
• le dextropropoxyphène,
• la dihydrocodéine,
• le meptazinol,
• la nalbuphine,
• la tilidine,
• la buprénorphine,
éventuellement sous forme de leurs racémates, de leurs stéréoisomères purs, en particulier des énantiomères ou des diastéréoisomères, ou sous forme de mélanges des stéréoisomères, en particulier des énantiomères ou des diastéréoisomères, dans un rapport de mélange quelconque ; sous forme de leurs acides ou de leurs bases, ou sous forme de leurs sels, en particulier des sels physiologiquement acceptables, ou sous forme de leurs produits de solvatation, en particulier des hydrates ;
le groupe c) comprenant :
des composés de 1-phényl-3-diméthylamino-propane répondant à la formule générale I : 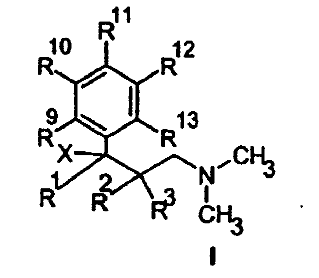
dans laquelle :
X est choisi entre OH, F, Cl, H ou un groupe OC(O)R⁷ dans lequel R⁷ est choisi entre des restes alkyle en C₁ à C₃ ramifiés ou non ramifiés, saturés ou non saturés, non substitués ou substitués une ou plusieurs fois,
R¹ est choisi entre des restes alkyle en C₁ à C₄, ramifiés ou non ramifiés, saturés ou non saturés, non substitués ou substitués une ou plusieurs fois,
R² et R³ sont choisis tous deux, indépendamment l'un de l'autre, entre H ou des restes alkyle en C₁ à C₄ ramifiés ou non ramifiés, saturés ou non saturés, non substitués ou substitués une ou plusieurs fois,
ou bien
R² et R³ forment ensemble un reste cycloalkyle en C₄ à C₇ non substitué ou substitué une ou plusieurs fois, R⁹ à R¹³ sont chacun choisis, indépendamment les uns des autres, entre H, F, Cl, Br, I, CH₂F, CHF₂, CF₃, OH, SH, OR¹⁴, OCF₃, SR¹⁴, NR¹⁷R¹⁸, SOCH₃, SOCF₃ ; SO₂CH₃, SO₂CF₃, CN, COOR¹⁴, NO₂, CONR¹⁷R¹⁸; un reste alkyle en C₁ à C₆, ramifié ou non ramifié, saturé ou non saturé, non substitué ou substitué une ou plusieurs fois ; un reste phényle, non substitué ou substitué une ou plusieurs fois ;
R¹⁴ étant choisi entre un reste alkyle en C₁ à C₆ ; pyridyle, thiényle, thiazolyle, phényle, benzyle ou phénéthyle, chacun non substitué ou substitué une ou plusieurs fois ;
un groupe PO(O-alkyle en C₁ à C₄)₂, CO(O-alkyle en C₁ à C₅), CONH-C₆H₄- (alkyle en C₁ à C₃), CO(alkyle en C₁ à C₅), CO-CHR¹⁷-NHR¹⁸, CO-C₆H₄-R¹⁵, R¹⁵ désignant un groupe ortho-OCO (alkyle en C₁ à C₃) ou méta- ou para-CH₂N(R¹⁶)₂ où R¹⁶ désigne un reste alkyle en C₁ à C₄ ou 4-morpholino, les groupes alkyle dans les restes R¹⁴, R¹⁵ et R¹⁶ pouvant être ramifiés ou non ramifiés, saturés ou non saturés, non substitués ou substitués une ou plusieurs fois ;
R¹⁷ et R¹⁸ étant tous deux choisis, indépendamment l'un de l'autre, entre H ; des restes alkyle en C₁ à C₆, ramifiés ou non ramifiés, saturés ou non saturés, non substitués ou substitués une ou plusieurs fois ; phényle, benzyle ou phénéthyle, chacun non substitué ou substitué une ou plusieurs fois,
ou bien
R⁹ et R¹⁰, ou R¹⁰ et R¹¹, forment conjointement un noyau OCH₂O-, OCH₂CH₂O-, OCH=CH-, CH=CHO-, CH=C(CH₃)O-, OC(CH₃)=CH-, (CH₂)₄- ou OCH=CHO-,
le cas échéant sous forme de leurs racémates, de leurs stéréoisomères purs, en particulier des énantiomères ou des diastéréoisomères, ou sous forme de mélanges des stéréoisomères, en particulier des énantiomères ou des diastéréoisomères, dans un rapport de mélange quelconque ; sous la forme représentée ou sous forme de leurs acides ou de leurs bases ou sous forme de leurs sels, en particulier des sels physiologiquement acceptables, ou sous forme de leurs produits de solvatation, en particulier des hydrates ;
le groupe d) comprenant :
des composés substitués de 6-diméthylaminométhyl-1-phényl-cyclohexane répondant à la formule générale II : 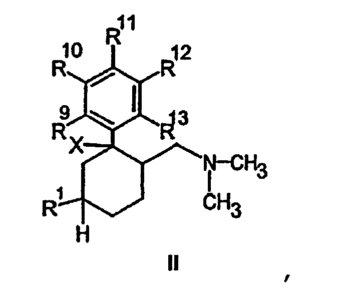 dans laquelle :
X est choisi entre OH, F, Cl, H ou un groupe OC(O)R⁷ dans lequel R⁷ est choisi entre des restes alkyle en C₁ à C₃, ramifiés ou non ramifiés, saturés ou non saturés, non substitués ou substitués une ou plusieurs fois,
R¹ est choisi entre un reste alkyle en C₁ à C₄, benzyle, CF₃, OH, OCH₂-C₆H₅, O-alkyle en C₁ à C₄, C1 ou F, et
R⁹ à R¹³ sont chacun choisis, indépendamment les uns des autres, entre H, F, Cl, Br, I, CH₂F, CHF₂, CF₃, OH, SH, OR¹⁴, OCF₃, SR¹⁴, NR¹⁷R¹⁸, SOCH₃, SOCF₃ ; SO₂CH₃; SO₂CF₃, CN, COOR¹⁴, NO₂, CONR¹⁷R¹⁸ ; des restes alkyle en C₁ à C₆, ramifiés ou non ramifiés, saturés ou non saturés, non substitués ou substitués une ou plusieurs fois ; un reste phényle, non substitué ou substitué une ou plusieurs fois ;
R¹⁴ étant choisi entre des restes alkyle en C₁ à C₆ ; pyridyle, thiényle, thiazolyle, phényle, benzyle ou phénéthyle, chacun non substitué ou substitué une ou plusieurs fois ;
un groupe PO(O-alkyle en C₁ à C₄)₂, CO(O-alkyle en C₁ à C₅), CONH-C₆H₄-(alkyle en C₁ à C₃), CO(alkyle en C₁ à C₅), CO-CHR¹⁷-NHR¹⁸, CO-C₆H₄-R¹⁵, R¹⁵ désignant un groupe ortho-OCO(alkyle en C₁ à C₃) ou méta- ou para-CH₂N(R¹⁶)₂ où R¹⁶ désigne un reste alkyle en C₁ à C₄ ou 4-morpholino, les groupes alkyle dans les restes R¹⁴ , R¹⁵ et R¹⁶ pouvant être ramifiés ou non ramifiés, saturés ou non saturés, non substitués ou substitués une ou plusieurs fois ;
R¹⁷ et R¹⁸ étant tous deux choisis, indépendamment l'un de l'autre, entre H ; des restes alkyle en C₁ à C₆, ramifiés ou non ramifiés, saturés ou non saturés, non substitués ou substitués une ou plusieurs fois ; phényle, benzyle ou phénéthyle, chacun non substitué ou substitué une ou plusieurs fois,
ou bien
R⁹ et R¹⁰, ou R¹⁰ et R¹¹, forment conjointement un noyau OCH₂O-, OCH₂CH₂O-, OCH=CH-, CH=CHO-, CH=C(CH₃)O-, OC(CH₃)=CH-, (CH₂)₄- ou OCH=CHO-,
le cas échéant sous forme de leurs racémates, de leurs stéréoisomères purs, en particulier des énantiomères ou des diastéréoisomères, ou sous forme de mélanges des stéréoisomères, en particulier des énantiomères ou des diastéréoisomères, dans un rapport de mélange quelconque ; sous la forme représentée ou sous forme de leurs acides ou de leurs bases ou sous forme de leurs sels, en particulier des sels physiologiquement acceptables, ou sous forme de leurs produits de solvatation, en particulier des hydrates ;
et/ou
le groupe e) comprenant :
des composés de 6-diméthylaminométhyl-1-phényl-cyclohexane de formule générale III : 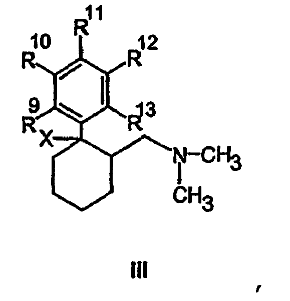
dans laquelle :
X est choisi entre OH, F, Cl, H ou un groupe OC(O)R⁷ dans lequel R⁷ est choisi entre des restes alkyle en
C₁ à C₃, ramifiés ou non ramifiés, saturés ou non saturés, non substitués ou substitués une ou plusieurs fois, et
R⁹ à R¹³ sont chacun choisis, indépendamment les uns des autres, entre H, F, Cl, Br, I, CH₂F, CHF₂, CF₃, OH, SH, OR¹⁴, OCF₃, SR¹⁴, NR¹⁷R¹⁸, SOCH₃, SOCF₃ ; SO₂CH₃, SO₂CF₃, CN, COOR¹⁴, NO₂, CONR¹⁷R¹⁸ ; des restes alkyle en C₁ à C₆, ramifiés ou non ramifiés, saturés ou non saturés, non substitués ou substitués une ou plusieurs fois ; un reste phényle, non substitué ou substitué une ou plusieurs fois ;
R¹⁴ étant choisi entre des restes alkyle en C₁ à C₆ ; pyridyle, thiényle, thiazolyle, phényle, benzyle ou phénéthyle, chacun non substitué ou substitué une ou plusieurs fois ;
un groupe PO(O-alkyle en C₁ à C₄)₂, CO(O-alkyle en C₁ à C₅), CONH-C₆H₄-(alkyle en C₁ à C₃), CO(alkyle en C₁ à C5), CO-CHR17-NHRla, CO-C₆H₄-R¹⁵, R¹⁵ désignant un groupe ortho-OCO(alkyle en C₁ à C₃) ou méta- ou para-CH₂N(R¹⁶)₂ où R¹⁶ désigne un reste alkyle en C₁ à C₄ ou 4-morpholino, les groupes alkyle dans les restes R¹⁴, R¹⁵ et R¹⁶ pouvant être ramifiés ou non ramifiés, saturés ou non saturés, non substitués ou substitués une ou plusieurs fois ;
R¹⁷ et R¹⁸ étant tous deux choisis, indépendamment l'un de l'autre, entre H ; des restes alkyle en C₁ à C₆, ramifiés ou non ramifiés, saturés ou non saturés, non substitués ou substitués une ou plusieurs fois ; un reste phényle, benzyle ou phénéthyle, chacun non substitué ou substitué une ou plusieurs fois,
ou bien
R⁹ et R¹⁰, ou R¹⁰ et R¹¹, forment conjointement un noyau OCH₂O-, OCH₂CH₂O-, OCH=CH-, CH=CHO-, CH=C(CH₃)O-, OC(CH₃)=CH-, (CH₂)₄- ou OCH=CHO-,
sous réserve que, lorsque R⁹, R¹¹ et R¹³ correspondent à H et que l'un de R¹⁰ ou R¹² représente H et l'autre représente un groupe OCH₃, X ne puisse pas représenter OH,
le cas échéant sous forme de leurs racémates, de leurs stéréoisomères purs, en particulier des énantiomères ou des diastéréoisomères, ou sous forme de mélanges des stéréoisomères, en particulier des énantiomères ou des diastéréoisomères, dans un rapport de mélange quelconque ; sous la forme représentée ou sous forme de leurs acides ou de leurs bases ou sous forme de leurs sels, en particulier des sels physiologiquement acceptables, ou sous forme de leurs produits de solvatation, en particulier des hydrates ;
et avec au moins l'un des composés B, choisis parmi :
les antimuscariniques : atropine, oxybutynine, propivérine, propanthéline, émépronium, trospium, toltérodine, darifénacine et l'ester de 4-(N-méthyl-pipéridyle) de l'acide α,α-diphénylacétique, ainsi que la duloxétine, l'imipramine et la desmopressine,
le cas échéant sous forme de leurs racémates, de leurs stéréoisomères, en particulier des énantiomères ou des diastéréoisomères, ou sous forme de mélanges des stéréoisomères, en particulier des énantiomères ou des diastéréoisomères, dans un rapport de mélange quelconque ; sous forme de leurs acides ou de leurs bases ou sous forme de leurs sels, en particulier des sels physiologiquement acceptables, ou sous forme de leurs produits de solvatation, en particulier des hydrates.

15. Combinaison de substances actives suivant la revendication 14, **caractérisée en ce que** le composé A du groupe a) est choisi entre :
le tramadol, le (+)-tramadol, le (+)-O-déméthyltramadol ou le (+)-O-desméthyl-N-mono-desméthyl-tramadol,
avantageusement le tramadol ou le (+)-tramadol, préférentiellement le (+)-tramadol.

16. Combinaison de substances actives suivant la revendication 14, **caractérisée en ce que** le composé A du groupe c) est choisi entre des composés de formule I dans laquelle :
X est choisi entre :
OH, F, Cl, OC(O)CH₃ ou H, avantageusement OH, F, OC(O)CH₃ ou H,
et/ou
R¹ est choisi entre :
des restes alkyle en C₁ à C₄, saturés et non substitués, ramifiés ou non ramifiés ; avantageusement CH₃, C₂H₅, C₄H₉ ou tertiobutyle, en particulier CH₃ ou C₂H₅,
et/ou
R² et R³ sont choisis indépendamment l'un de l'autre entre :
H, des restes alkyle en C₁ à C₄, saturés et non substitués, ramifiés ou non ramifiés ; avantageusement H, CH₃, C₂H₅, isopropyle ou tertiobutyle, en particulier H ou CH₃, R³ représentant préférentiellement H,
ou bien
R² et R³ forment ensemble un reste cycloalkyle en C₅ ou C₆, saturé ou non saturé, non substitué ou substitué une ou plusieurs fois, avantageusement saturé et non substitué, en particulier cyclohexyle,
et/ou
R⁹ à R¹³, dont 3 ou 4 des restes R⁹ à R¹³ doivent représenter H, sont choisis indépendamment les uns des autres entre :
H, Cl, F, OH, CF₂H, CF₃ ou des restes alkyle en C₁ à C₄ saturés et non substitués, ramifiés ou non ramifiés ; OR¹⁴ ou SR¹⁴, R¹⁴ étant choisi entre des restes alkyle en C₁ à C₃ saturés et non substitués, ramifiés ou non ramifiés ;
avantageusement H, Cl, F, OH, CF₂H, CF₃, OCH₃ ou SCH₃,
ou bien R¹² et R¹¹ forment un noyau 3, 4-OCH=CH,
en particulier
lorsque R⁹, R¹¹ et R¹³ représentent H, l'un de R¹⁰ ou R¹² représente aussi H, tandis que l'autre est choisi entre :
Cl, F, OH, CF₂H, CF₃, OR¹⁴ ou SR¹⁴, avantageusement OH, CF₂H, OCH₃ ou SCH₃,
ou bien
lorsque R⁹ et R¹³ représentent H et R¹¹ représente OH, OCH₃, Cl ou F, avantageusement Cl, l'un de R¹⁰ ou R¹² représente aussi H, tandis que l'autre représente OH, OCH₃, Cl ou F, avantageusement Cl,
ou bien,
lorsque R⁹, R¹⁰, R¹² et R¹³ représentent H, R¹¹ est choisi entre CF₃, CF₂H, Cl ou F, avantageusement F,
ou bien,
lorsque R¹⁰, R¹¹ et R¹² représentent H, l'un de R⁹ ou R¹³ représente aussi H, tandis que l'autre est choisi entre OH, OC₂H₅ ou OC₃H₇.

17. Combinaison de substances actives suivant la revendication 16, **caractérisée en ce que** les composés de formule I dans laquelle R³ représente H se présentent sous forme des diastéréoisomères ayant la configuration relative Ia : 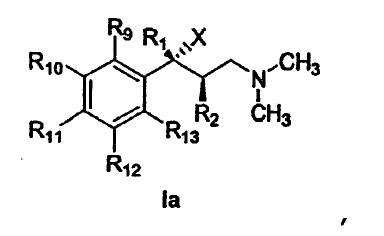 en particulier dans des mélanges contenant une plus forte proportion de ce diastéréoisomère comparativement à l'autre diastéréoisomère, ou sous forme de diastéréoisomère pur,
et/ou **en ce que**
les composés de formule I se présentent sous forme du (+)-énantiomère, en particulier dans des mélanges contenant une plus forte proportion du (+)-énantiomère comparativement au (-)-énantiomère d'un composé racémique, ou sous forme du (+)-énantiomère pur.

18. Combinaison de substances actives suivant l'une des revendications 16 ou 17, **caractérisée en ce que** le composé A est choisi dans le groupe suivant :
■ (2RS,3RS)-1-diméthylamino-3-(3-méthoxy-phényl)-2-méthyl-pentane-3-ol,
■ (2R,3R)-1-diméthylamino-3-(3-méthoxy-phényl)-2-méthyl-pentane-3-ol,
■ (+)-(2R,3R)-1-diméthylamino-3-(3-méthoxy-phényl)-2-méthyl-pentane-3-ol,
■ (2RS,3RS)-3-(3,4-dichlorophényl)-1-diméthylamino-2-méthyl-pentane-3-ol,
■ (2RS,3RS)-3- (3-difluorométhyl-phényl)-1-diméthylamino-2-méthyl-pentane-3-ol,
■ (2RS,3RS)-1-diméthylamino-2-méthyl-3-(3-méthylsulfanyl-phényl)-pentane-3-ol,
■ (3RS)-1-diméthylamino-3-(3-méthoxy-phényl)-4,4-diméthyl-pentane-3-ol,
■ (2RS,3RS)-3-(3-diméthylamino-1-éthyl-1-hydroxy-2-méthyl-propyl)-phénol,
■ (1RS,2RS)-3-(3-diméthylamino-1-hydroxy-1,2-diméthyl-propyl)-phénol,
■ (+)-(1R,2R)-3-(3-diméthylamino-1-hydroxy-1,2-diméthyl-propyl)-phénol,
■ (+)- (1R, 2R) -3- (3-diméthylamino-1-hydroxy-1,2-diméthyl-propyl)-phénol,
■ (1R, 2R) -3- (3-diméthylamino-1-éthyl-2-méthyl-propyl)-phénol,
■ (-)-(1R,2R)-3-(3-diméthylamino-1-éthyl-2-méthyl-propyl)-phénol,
■ (1S,2S)-3-(3-diméthylamino-1-éthyl-2-méthyl-propyl)-phénol,
■ (+)-(1S,2S)-3-(3-diméthylamino-1-éthyl-2-méthyl-propyl)-phénol,
■ ester de (+)-(1R,2R)-3-diméthylamino-1-éthyl-(3-méthoxy-phényl)-2-méthyl-propyle d'acide acétique
■ (IRS)-1-(1-diméthylaminométhyl-cyclohexyl)-1-(3-méthoxy-phényl)-propane-1-ol,
■ (2RS,3RS)-3-(4-chlorophényl)-1-diméthylamino-2-méthyl-pentane-3-ol,
■ (+)-(2R,3R)-3-(3-diméthylamino-1-éthyl-1-hydroxy-2-méthyl-propyl)-phénol,
■ (2RS, 3RS) -4-diméthylamino-2- (3-méthoxy-phényl)-3-méthyl-butane-2-ol, et
■ (+)-(2R,3R)-4-diméthylamino-2-(3-méthoxy-phényl)-3-méthyl-butane-2-ol,
avantageusement sous forme du chlorhydrate.

19. Combinaison de substances actives suivant la revendication 14, **caractérisée en ce que** le composé A du groupe d) est choisi parmi des composés répondant à la formule II dans laquelle :
X est choisi entre :
OH, F, Cl, OC(O)CH₃ ou H, avantageusement OH, F ou H, en particulier OH,
et/ou
R¹ est choisi entre :
alkyle en C₁ à C₄, CF₃, OH, O-alkyle en C₁ à C₄, Cl ou F, avantageusement OH, CF₃ ou CH₃,
et/ou
R⁹ à R¹³, dont 3 ou 4 de ces restes R⁹ à R¹³ doivent représenter H, sont choisis indépendamment les uns des autres entre
H, Cl, F, OH, CF₂H, CF₃ ou des restes alkyle en C₁ à C₄ saturés et non substitués, ramifiés ou non ramifiés ; OR¹⁴ ou SR¹⁴, R¹⁴ étant choisi entre des restes alkyle en C₁ à C₃ saturés et non substitués, ramifiés ou non ramifiés ;
avantageusement H, Cl, F, OH, CF₂H, CF₃, OCH₃ ou SCH₃,
ou bien R¹² et R¹¹ forment un noyau 3, 4-OCH=CH,
en particulier
lorsque R⁹, R¹¹ et R¹³ représentent H, l'un de R¹⁰ ou R¹² représente aussi H, tandis que l'autre est choisi entre :
Cl, F, OH, CF₂H, CF₃, OR¹⁴ ou SR¹⁴, avantageusement OH, CF₂H, OR¹⁴ ou SCH₃, en particulier OH ou O-alkyle en C₁ à C₃, avantageusement OH ou OCH₃,
ou bien,
lorsque R⁹ et R¹³ représentent H et R¹¹ représente OH, OCH₃, Cl ou F, avantageusement Cl, l'un de R¹⁰ ou R¹² représente aussi H, tandis que l'autre représente OH, OCH₃, Cl ou F, avantageusement Cl,
ou bien,
lorsque R⁹, R¹⁰, R¹² et R¹³ représentent H, R¹¹ est choisi entre CF₃, CF₂H, Cl ou F, avantageusement F,
ou bien,
lorsque R¹⁰, R¹¹ et R¹² représentent H, l'un de R⁹ ou R¹³ représente aussi H, tandis que l'autre est choisi entre OH, OC₂H₅ ou OC₃H₇,
de façon tout particulièrement appréciée,
lorsque R^{9,} R¹¹ et R¹³ représentent H, l' un de R¹⁰ ou R¹² représente aussi H, tandis que l'autre est choisi entre :
Cl, F, OH, SH, CF₂H, CF₃, OR¹⁴ ou SR¹⁴, avantageusement OH ou OR¹⁴, en particulier OH ou O-alkyle en C₁ à C₃, avantageusement OH ou OCH₃.

20. Combinaison de substances actives suivant la revendication 19, **caractérisée en ce que** les composés de formule II se présentent sous forme de diastéréoisomères ayant la configuration relative IIa : 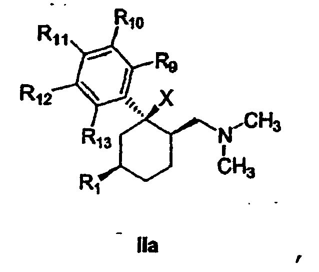 en particulier dans des mélanges contenant une plus forte proportion de ce diastéréoisomère comparativement à l'autre diastéréoisomère, ou sous forme de diastéréoisomère pur,
et/ou
les composés de formule II se présentent sous forme du (+)-énantiomère, en particulier dans des mélanges contenant une plus forte proportion du (+)-énantiomère comparativement au (-)-énantiomère d'un composé racémique, ou sous forme du (+)-énantiomère.

21. Combinaison de substances actives suivant l'une des revendications 19 ou 20, **caractérisée en ce que** le composé A est choisi dans le groupe suivant :
■ (1RS,3RS,6RS)-6-diméthylaminométhyl-1-(3-méthoxy-phényl)-cyclohexane-1,3-diol,
■ (+)-(1R,3R,6R)-6-diméthylaminométhyl-1-(3-méthoxy-phényl)-cyclohexane-1,3-diol,
■ (1RS,3RS,6RS)-6-diméthylaminométhyl-1-(3-hydroxy-phényl)-cyclohexane-1,3-diol,
■ (1RS,3SR,6RS)-6-diméthylaminométhyl-1-(3-méthoxy-phényl)-cyclohexane-1,3-diol,
■ (+)-(1R,2R,5S)-3-(2-diméthylaminométhyl-1-hydroxy-5-méthyl-cyclohexyl)-phénol ou
■ (1RS,2RS,5RS)-3-(2-diméthylaminométhyl-1-hydroxy-5-trifluorométhyl-cyclohexyl)-phénol,
avantageusement sous forme du chlorhydrate.

22. Combinaison de substances actives suivant la revendication 14, **caractérisée en ce que** le composé A du groupe e) est choisi parmi des composés de formule III dans laquelle :
X est choisi entre :
OH, F, Cl, OC(O)CH₃ ou H, avantageusement OH, F ou H, en particulier F ou H,
et/ou
R⁹ à R¹³, dont 3 ou 4 de ces restes R⁹ à R¹³ doivent représenter H, sont choisis indépendamment les uns des autres entre :
H, Cl, F, OH, CF₂H, CF₃ ou des groupes alkyle en C₁ à C₄, saturés et non substitués, ramifiés ou non ramifiés ; OR¹⁴ ou SR¹⁴, R¹⁴ étant choisi entre des restes alkyle en C₁ à C₃ saturés et non substitués, ramifiés ou non ramifiés ;
avantageusement H, Cl, F, OH, CF₂H, CF₃, OCH₃ ou SCH₃,
ou bien R¹² et R¹¹ forment un noyau 3, 4-OCH=CH,
ayant en particulier pour caractéristique que,
lorsque R⁹, R¹¹ et R¹³ représentent H, l'un de R¹⁰ ou R¹² représente aussi H, tandis que l'autre est choisi entre :
Cl, F, OH, CF₂H, CF₃, OR¹⁴ ou SR¹⁴, avantageusement OH, CF₂H, OR¹⁴ ou SCH₃, en particulier OH ou O-alkyle en C₁ à C₃, avantageusement OH ou OCH₃,
ou bien,
lorsque R⁹ et R¹³ représentent H et R¹¹ représente OH, OCH₃, Cl ou F, avantageusement Cl, l'un de R¹⁰ ou R¹² représente aussi H, tandis que l'autre représente OH, OCH₃, Cl ou F, avantageusement Cl,
ou bien,
lorsque R⁹, R¹⁰, R¹² et R¹³ représentent H, R¹¹ est choisi entre CF₃, CF₂H, Cl ou F, avantageusement F,
ou bien,
lorsque R¹⁰, R¹¹ et R¹² représentent H, l'un de R⁹ ou R¹³ représente aussi H, tandis que l'autre est choisi entre OH, OC₂H₅ ou OC₃H₇,
de façon tout particulièrement appréciée,
lorsque R^{9,} R¹¹ et R¹³ représentent H, l'un de R¹⁰ ou R¹² représente aussi H, tandis que l'autre est choisi entre :
Cl, F, OH, SH, CF₂H, CF₃, OR¹⁴ ou SR¹⁴, avantageusement OH ou OR¹⁴, en particulier OH ou O-alkyle en C₁ à C₃, préférentiellement OH ou OCH₃.

23. Combinaison de substances actives suivant la revendication 22, **caractérisée en ce que** les composés de formule III se présentent sous forme de leurs diastéréoisomères ayant la configuration relative IIIa : 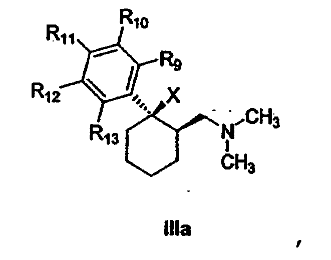 en particulier dans des mélanges contenant une plus forte proportion de ce diastéréoisomère comparativement à l'autre diastéréoisomère, ou sous forme de diastéréoisomère pur,
et/ou **en ce que**
les composés de formule III se présentent sous forme du (+)-énantiomère, en particulier dans des mélanges contenant une plus forte proportion du (+)-énantiomère comparativement au (-)-énantiomère d'un composé racémique ou sous forme du (+)-énantiomère.

24. Combinaison de substances actives suivant l'une des revendications 22 ou 23, **caractérisée en ce que** le composé A est choisi dans le groupe de composés suivant :
■ (+)-(1R,2R)-3-(2-diméthylaminométhyl-1-fluoro-cyclohexyl)-phénol,
■ (+)-(1S,2S)-3-(2-diméthylaminométhyl-cyclohexyl)-phénol, ou
■ (1S,2S)-3-(2-diméthylaminométhyl-cyclohexyl)-phénol, ou
■ (-)-(1R,2R)-3-(2-diméthylaminométhyl-cyclohexyl)-phénol,
■ (1R,2R)-3-(2-diméthylaminométhyl-cyclohexyl)-phénol,
■ (-) - (1R, 2R) - [2- (3-méthoxy-phényl) -cyclohexylméthyl]-diméthylamine,
■ (1R,2R)-[2-(3-méthoxy-phényl)-cyclohexylméthyl]-diméthylamine,
avantageusement sous forme du chlorhydrate.

25. Combinaison de substances actives suivant l'une des revendications 14 à 24, **caractérisée en ce que** le composé B est choisi entre
la darifénacine, la duloxétine, l'oxybutynine ou la toltérodine,
avantageusement choisi entre
la duloxétine, l'oxybutynine ou la toltérodine,
préférentiellement choisi entre
l'oxybutynine ou la toltérodine.

26. Médicament, destiné avantageusement au traitement de la répétition du besoin impérieux d'uriner ou de l'incontinence d'urine, contenant une combinaison de substances actives suivant l'une des revendications 14 à 25 ainsi que, le cas échéant, des additifs et/ou des substances auxiliaires convenables.
